# EUROPEAN PATENT SPECIFICATION

(11) **EP 4 340 809 B1**
(45) Date of publication and mention of the grant of the patent: **12.08.2026**
(21) Application number: 22729558.1
(22) Date of filing: 17.05.2022
(51) Int. Cl.: A61K 9/00, A61K 9/14, A61K 9/19, A61K 9/20, A61K 31/675, A61K 31/4045, A61P 25/28, A61P 25/18, A61P 25/04, C07B 59/00, C07D 209/14, A61P 1/00

(54) **FORMULATIONS OF PSILOCYBIN**
FORMULIERUNGEN VON PSILOCYBIN
FORMULATIONS DE PSILOCYBINE

(30) Priority: 17.05.2021 US 202163189449 P
(43) Date of publication of application: 27.03.2024
(60) Divisional application: 26189164.2
(73) Proprietor: Cybin IRL Limited, Dublin 1, D01X9R7 (IE)
(72) Inventor: NIVOROZHKIN, Alex, West Roxbury, Massachusetts 02132 (US); PALFREYMAN, Michael, St. Petersburg, Florida 33701 (US); AVERY, Kenneth L., Merrimack, New Hampshire 03054 (US); PATHARE, Pradip M., Lexington, Massachusetts 02421 (US); SHUKOOR, Mohammed I., Lexington, Massachusetts 02421 (US)
(74) Representative: Müller-Boré & Partner Patentanwälte PartG mbB
(86) International application number: PCT/EP2022/063269
(87) International publication number: WO 2022/243285

(56) References cited:
- WO-A1-2020/148442
- WO-A1-2020/245133
- WO-A1-2021/003467
- US-A1- 2021 015 738
- US-B1- 11 000 534

## Description

### CROSS REFERENCE TO RELATED APPLICATIONS

This application claims the benefit of U.S. Provisional Application No. 63/189,449 filed May 17, 2021.

### FIELD

The present disclosure relates generally to compositions of psilocybin and/or deuterated psilocybin and, in some embodiments, to serotonin 5-HT₂ receptor agonists and uses in the treatment of diseases associated with a 5-HT₂ receptor.

### BACKGROUND

Psilocybin (PY) and psilocin (PI) are tryptamine alkaloids and structural analogs of the neurotransmitter serotonin. Psilocybin is a prodrug of psilocin. That is, when consumed, psilocybin is rapidly metabolized into the active form, psilocin. Specifically, a chemical process called dephosphorylation removes the phosphate group on psilocybin, creating psilocin.

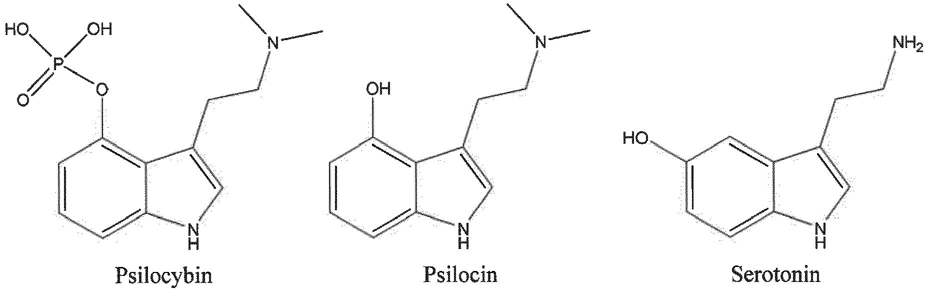

Psilocin is a short-lived and unstable molecule. Therefore, therapeutic applications involving the use of psilocin are generally accomplished by administration of the precursor, psilocybin. However, psilocybin has a slow onset of drug action (1.5-2 hours) and a long duration of drug action (2-3 hours), often requiring 7-8 hours of supervised clinical observation of a patient before discharge. Therefore, there is a need for a psilocybin formulation that has a faster/quicker therapeutic onset and a shorter duration of drug action (i.e., shorter duration of therapeutic effect) than current therapeutic applications of psilocybin.

Generally, amorphous drug forms tend to have improved solubility in water compared to their crystalline counterparts, and thus can give rise to markedly improved pharmaceutical performance such as faster onset, higher bioavailability, etc.-however, in the case of psilocybin, the amorphous form is unstable and has a tendency to crystallize. *See* Greenan et al., Preparation and Characterization of Novel Crystalline solvates and Polymorphs of Psilocybin and Identification of Solid Forms Suitable for Clinical Development, 2020 pre-publication; DOI:10.13140/RG.2.2.32357.14560. US 2021/015738 A1 discloses an oral dissolvable film that includes a psychedelic compound embedded in a polymer matrix.

### SUMMARY

In view of the forgoing, there is a need for new psilocybin compositions that allow psilocybin to stably exist primarily in amorphous form with an extended shelf life. The present disclosure is based at least in part on the identification of stable formulations of amorphous psilocybin and/or deuterated psilocybin which prevent/reduce amorphous to crystalline transitions, and which demonstrate improved pharmaceutical performance (e.g., faster/quicker therapeutic onset, a shorter duration of drug action) compared to crystalline dosage forms. More specifically, the present disclosure provides stable compositions of amorphous psilocybin and/or deuterated psilocybin that modulate serotonin 5-HT₂ receptors and methods of using the same to treat diseases associated with a serotonin 5-HT₂ receptor. The present disclosure also provides novel compositions of amorphous psilocybin and/or deuterated psilocybin that permit, for example, once-daily dosing to selectively engage 5-HT_{2A}Rs without producing psychedelic effects, and to treat neuropsychiatric and other disorders associated with inflammation.

Thus, the present disclosure provides:
(1) A pharmaceutical composition, comprising:
   a solid dispersion comprising a therapeutically effective amount of a compound of Formula (I) in amorphous form dispersed in a polymer, 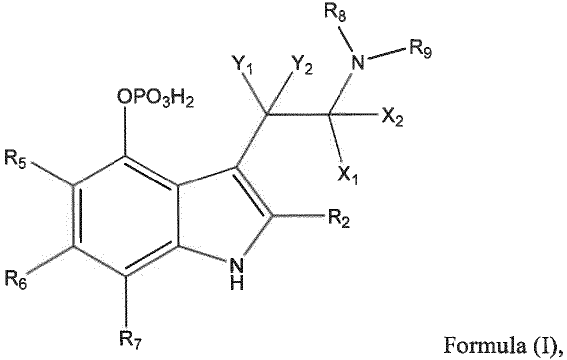 or a pharmaceutically acceptable salt, stereoisomer, a tautomer, or solvate thereof,
   wherein:
      R₂, R₅, R₆, and R₇ are independently selected from the group consisting of hydrogen and deuterium,
      R₈ and R₉ are independently selected from the group consisting of -CH₃ and -CD₃, and
      X₁, X₂, Y₁, and Y₂ are independently selected from the group consisting of hydrogen and deuterium.
(2) The pharmaceutical composition of (1), wherein R₂, R₅, R₆, and R₇ are hydrogen.
(3) The pharmaceutical composition of (1), wherein R₂, R₅, R₆, and R₇ are deuterium.
(4) The pharmaceutical composition of any one of (1) to (3), wherein R₈ and R₉ are -CH₃.
(5) The pharmaceutical composition of any one of (1) to (3), wherein R₈ and R₉ are -CD₃.
(6) The pharmaceutical composition of any one of (1) to (5), wherein X₁, X₂, Y₁, and Y₂ are deuterium.
(7) The pharmaceutical composition of any one of (1) to (6), wherein X₁ and X₂ are deuterium.
(8) The pharmaceutical composition of any one of (1) to (7), wherein Y₁ and Y₂ are deuterium.
(9) The pharmaceutical composition of any one of (1) to (5), wherein Y₁ and Y₂ are hydrogen.
(10) The pharmaceutical composition of any one of (1) to (9), wherein the compound of Formula (I) is at least one selected from the group consisting of: 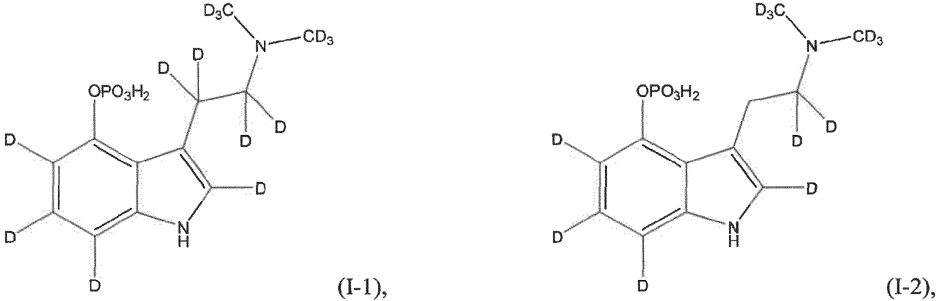 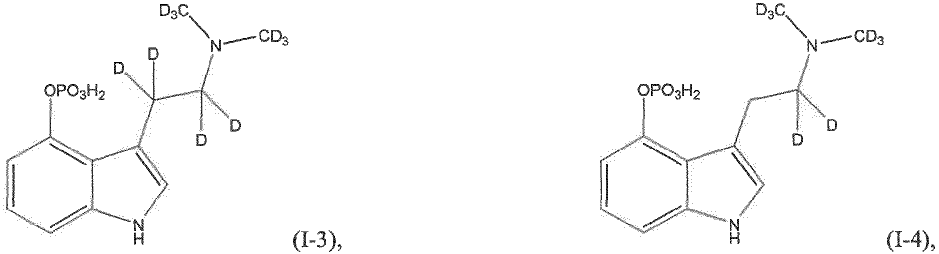 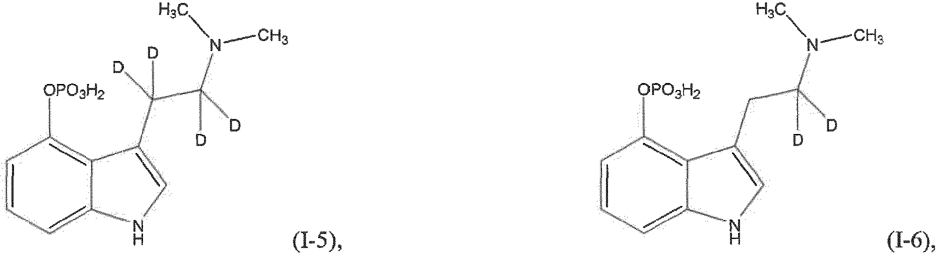 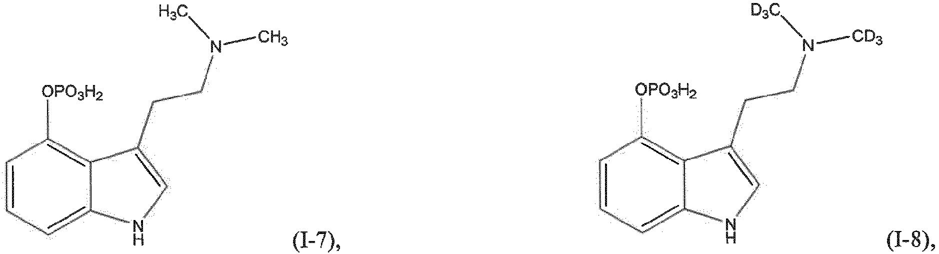 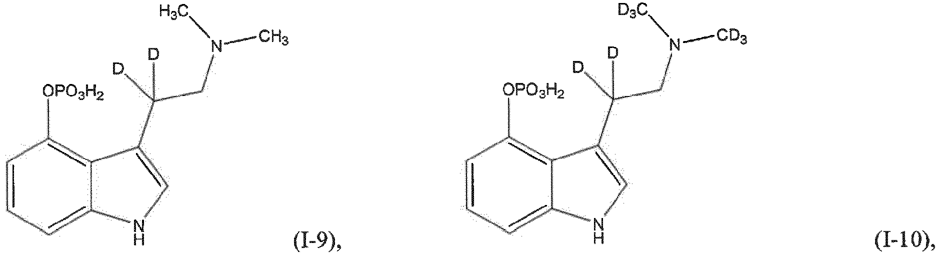 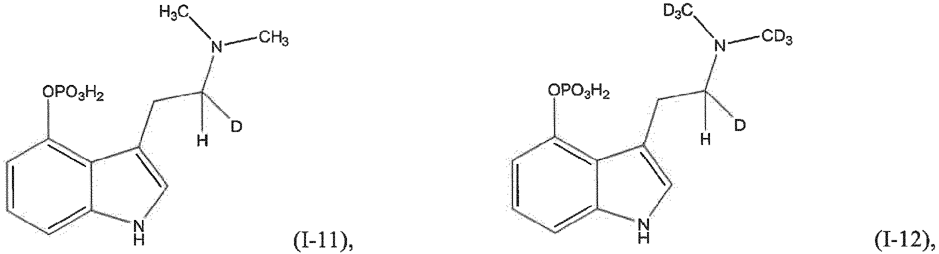 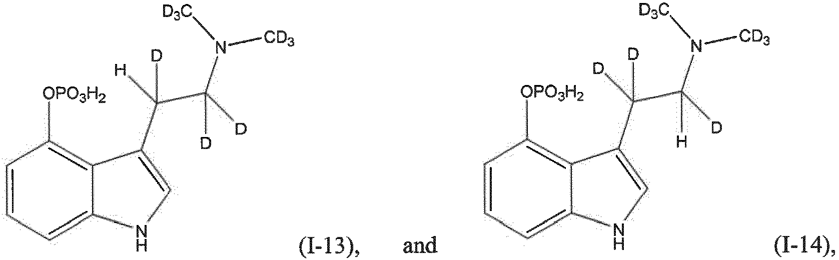 or a pharmaceutically acceptable salt, stereoisomer, a tautomer, or solvate thereof.
(11) The pharmaceutical composition of (1), wherein the compound of Formula (I) is 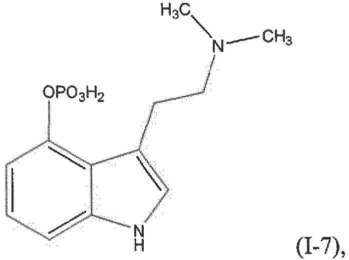 or a pharmaceutically acceptable salt, tautomer, or solvate thereof.
(12) The pharmaceutical composition of any one of (1) to (11), wherein the compound of Formula (I) is an agonist of a serotonin 5-HT₂ receptor.
(13) The pharmaceutical composition of any one of (1) to (12), wherein the compound of Formula (I) is an agonist of a serotonin 5-HT_{2A} receptor.
(14) The pharmaceutical composition of any one of (1) to (13), wherein the solid dispersion is a solid molecular complex.
(15) The pharmaceutical composition of any one of (1) to (14), wherein the compound of Formula (I) is present in the solid dispersion in an amount of 0.1 wt.% to 90 wt.%, based on a total weight of the solid dispersion.
(16) The pharmaceutical composition of any one of (1) to (15), wherein a weight ratio of the compound of Formula (I) to the polymer in the solid dispersion is from 1:9 to 9:1.
(17) The pharmaceutical composition of any one of (1) to (16), wherein the polymer is at least one selected from the group consisting of a vinyl polymer, a methacrylate, a polysaccharide, gelatin, and a cellulose polymer, or a blend or a copolymer thereof.
(18) The pharmaceutical composition of any one of (1) to (17), wherein the polymer is at least one selected from the group consisting of gelatin, polyvinyl acetate, polyvinyl alcohol, polyvinylpyrrolidone, pullulan, hydroxypropyl methyl cellulose, hydroxypropyl methyl cellulose acetate succinate, and a methacrylate copolymer, or a blend or a copolymer thereof.
(19) The pharmaceutical composition of any one of (1) to (18), wherein the polymer comprises gelatin.
(20) The pharmaceutical composition of (19), wherein the solid dispersion further comprises a pharmaceutically acceptable excipient.
(21) The pharmaceutical composition of (20), wherein the pharmaceutically acceptable excipient comprises mannitol.
(22) The pharmaceutical composition of any one of (1) to (21), wherein the polymer comprises a copolymer of vinyl pyrrolidone and vinyl acetate (PVP-VAc).
(23) The pharmaceutical composition of any one of (1) to (22), wherein the polymer comprises a methacrylate copolymer.
(24) The pharmaceutical composition of any one of (1) to (23), wherein the polymer comprises a cellulose polymer.
(25) The pharmaceutical composition of (24), wherein the cellulose polymer has a weight average molecular weight of from 150,000 g/mol to 5,000,000 g/mol.
(26) The pharmaceutical composition of (24), wherein the cellulose polymer has a weight average molecular weight of from 1,000 g/mol to 100,000 g/mol.
(27) The pharmaceutical composition of any one of (24) to (26), wherein the cellulose polymer is hydroxypropyl methyl cellulose acetate succinate.
(28) The pharmaceutical composition of any one of (24) to (26), wherein the cellulose polymer is hydroxypropyl methyl cellulose.
(29) The pharmaceutical composition of any one of (1) to (18), (24) to (26), or (28), wherein the polymer is a blend of hydroxypropyl methyl cellulose and polyvinylpyrrolidone.
(30) The pharmaceutical composition of any one of (1) to (29), further comprising a pharmaceutically acceptable excipient which is not dispersed with the solid dispersion.
(31) The pharmaceutical composition of any one of (1) to (30), further comprising sodium phosphate and/or a natural amino acid.
(32) The pharmaceutical composition of any one of (1) to (31), wherein the solid dispersion has a glass transition (Tg) onset of from 110°C to 200°C, as determined by modulated differential scanning calorimetry (mDSC).
(33) The pharmaceutical composition of any one of (1) to (32), wherein the solid dispersion has a heat capacity change (ΔCp), in J/(g·°C), of from 0.1 to 0.75, as determined by modulated differential scanning calorimetry (mDSC).
(34) The pharmaceutical composition of any one of (1) to (33), wherein the compound of Formula (I) is present in an amount of 0.1 to 1000 mg.
(35) The pharmaceutical composition of any one of (1) to (34), which is adapted for intraoral administration.
(36) The pharmaceutical composition of (35), wherein the pharmaceutical composition is in orodispersible dosage form.
(37) The pharmaceutical composition of (35) or (36), wherein the pharmaceutical composition is in a form of lyophilized fast dissolving tablets.
(38) The pharmaceutical composition of (35) or (36), wherein the pharmaceutical composition is in a form of lyophilized wafers.
(39) The pharmaceutical composition of any one of (1) to (34), which is adapted for oral administration.
(40) The pharmaceutical composition of any one of (1) to (34) or (39), which is adapted for extended-release.
(41) A pharmaceutical composition of any one of (1) to (40) for use in treating a subject with a disease or disorder.
(42) A pharmaceutical composition of any one of (1) to (40) for use in treating a subject with a disease or disorder associated with a serotonin 5-HT₂ receptor.
(43) The pharmaceutical composition for use of (42), wherein the disease or disorder is a neuropsychiatric disease or disorder or an inflammatory disease or disorder.
(44) The pharmaceutical composition for use of (42), wherein the disease or disorder is a central nervous system (CNS) disorder.
(45) The pharmaceutical composition for use of (44), wherein the central nervous system (CNS) disorder is at least one selected from the group consisting of major depressive disorder (MDD), treatment-resistant depression (TRD), post-traumatic stress disorder (PTSD), bipolar and related disorders, obsessive-compulsive disorder (OCD), generalized anxiety disorder (GAD), social anxiety disorder, a substance use disorder, an eating disorder, Alzheimer's disease, cluster headache and migraine, attention deficit hyperactivity disorder (ADHD), pain and neuropathic pain, aphantasia, childhood-onset fluency disorder, major neurocognitive disorder, mild neurocognitive disorder, suicidal ideation, suicidal behavior, major depressive disorder with suicidal ideation or suicidal behavior, melancholic depression, atypical depression, dysthymia, non-suicidal self-injury disorder (NSSID), chronic fatigue syndrome, Lyme's disease, gambling disorder, a paraphilic disorder, sexual dysfunction, peripheral neuropathy, and obesity.
(46) The pharmaceutical composition for use of (44), wherein the central nervous system (CNS) disorder is major depressive disorder (MDD).
(47) The pharmaceutical composition for use of (44), wherein the central nervous system (CNS) disorder is treatment-resistant depression (TRD).
(48) The pharmaceutical composition for use of (44), wherein the central nervous system (CNS) disorder is generalized anxiety disorder (GAD).
(49) The pharmaceutical composition for use of (44), wherein the central nervous system (CNS) disorder is social anxiety disorder.
(50) The pharmaceutical composition for use of (44), wherein the central nervous system (CNS) disorder is obsessive-compulsive disorder (OCD).
(51) The pharmaceutical composition for use of (44), wherein the central nervous system (CNS) disorder is cluster headaches or migraine.
(52) The pharmaceutical composition for use of (44), wherein the central nervous system (CNS) disorder is a substance use disorder.
(53) The pharmaceutical composition for use of (52), wherein the substance use disorder is alcohol use disorder.
(54) The pharmaceutical composition for use of (42), wherein the disease or disorder is mental distress in frontline healthcare workers.
(55) The pharmaceutical composition for use of (42), wherein the disease or disorder is an autonomic nervous system (ANS) condition.
(56) The pharmaceutical composition for use of (42), wherein the disease or disorder is a pulmonary disorder.
(57) The pharmaceutical composition for use of (42), wherein the disease or disorder is a cardiovascular disorder.
(58) The pharmaceutical composition for use of any one of (42) to (57), wherein the pharmaceutical composition is administered orally to the subject.
(59) The pharmaceutical composition for use of of (42) to (57), wherein the pharmaceutical composition is administered intraorally to the subject.
(60) The pharmaceutical composition for use of any one of (42) to (57), wherein the pharmaceutical composition is administered subcutaneously to the subject.
(61) The pharmaceutical composition for use of any one of (42) to (60), wherein the pharmaceutical composition is administered to provide the compound of Formula (I) to the subject at a psychedelic dose of about 0.083 mg/kg to about 1 mg/kg.
(62) The pharmaceutical composition for use of any one of (42) to (60), wherein the pharmaceutical composition is administered to provide the compound of Formula (I) to the subject at a sub-psychedelic dose of about 0.00001 mg/kg to less than about 0.083 mg/kg.
(63) The pharmaceutical composition for use of (42), wherein the disease or disorder is a neurological or neurodegenerative disease.
(64) The pharmaceutical composition for use of (63), wherein the neurological or neurodegenerative disease is Alzheimer's disease or other dementia subtype or Parkinson's disease.
(65) The pharmaceutical composition for use of (63) or (64), wherein the method reduces neuroinflammation in the subject compared to neuroinflammation prior to treatment commencement.
(66) The pharmaceutical composition for use of (63) to (65), wherein the pharmaceutical composition is administered to provide the compound of Formula (I) to the subject at a sub-psychedelic dose of about 0.00001 mg/kg to less than about 0.083 mg/kg.
(67) The pharmaceutical composition for use of (63) to (66), wherein the pharmaceutical composition is in an oral and/or extended-release dosage form.
(68) The pharmaceutical composition for use of (63) to (67), wherein the neurological or neurodegenerative disease is Alzheimer's disease.
(69) The pharmaceutical composition for use of (68), wherein the method treats depression, anxiety, and/or stress associated with Alzheimer's disease.
(70) A pharmaceutical composition of any one of (1) to (40) for use in decreasing time of therapeutic onset relative to a crystalline psilocybin-based drug, comprising:
   administering the pharmaceutical composition to a subject in need thereof.
(71) A pharmaceutical composition of any one of (1) to (40) for use in reducing psychedelic side effects relative to a crystalline psilocybin-based drug, comprising:
   administering the pharmaceutical composition to a subject in need thereof
(72) A pharmaceutical composition of any one of (1) to (40) for use in decreasing duration of therapeutic effect compared to a crystalline psilocybin-based drug, comprising:
   administering the pharmaceutical composition to a subject in need thereof.

### BRIEF DESCRIPTION OF THE DRAWINGS

The forgoing paragraphs have been provided by way of general introduction and are not intended to limit the scope of the following claims. The described embodiments, together with further advantages, will be best understood by reference to the following detailed description when considered in conjunction with the accompanying drawings, wherein:
Fig. 1 is a synthetic route to psilocybin-*d*₁₀ (**I-3**);
Fig. 2 shows the calculated X-ray power diffraction (XRPD) pattern of psilocybin Form A (Cambridge structural database (CSD) Reference Code HATCAK & TAVZID)
Fig. 3 shows the calculated XRPD pattern of psilocybin Form B (CSD Reference Code TAVZID01);
Fig. 4 shows the calculated XRPD pattern of psilocybin methanol solvate (CSD Reference Code PSILOC);
Fig. 5 shows the calculated XRPD pattern of psilocybin trihydrate (CSD Reference Code OKOKAD);
Fig. 6 shows the XRPD pattern of crystalline psilocybin methanol solvate with a small quantity of Form B (commercially available from Quality Chemical Labs);
Fig. 7 shows the XRPD pattern of Reference Example 1b;
Fig. 8 shows the XRPD pattern of Reference Example 2b;
Fig. 9 shows the XRPD pattern of Reference Example 3b;
Fig. 10 shows the XRPD pattern of Reference Example 4b;
Fig. 11 shows the XRPD pattern of Reference Example 5b;
Fig. 12 shows the XRPD pattern of Example 1;
Fig. 13 shows the XRPD pattern of Example 3;
Fig. 14 shows the XRPD pattern of Example 4;
Fig. 15 shows the XRPD pattern of Example 2;
Fig. 16 shows the XRPD pattern of Example 5;
Fig. 17 shows the high-resolution XRPD pattern of Reference Example 5b;
Fig. 18 shows the high-resolution XRPD pattern of Example 5;
Fig. 19 shows the XRPD pattern of Reference Example 1a;
Fig. 20 shows the XRPD pattern of Reference Example 2a;
Fig. 21 shows the XRPD pattern of Reference Example 3a;
Fig. 22 shows the XRPD pattern of Reference Example 4a;
Fig. 23 shows the XRPD pattern of Reference Example 5a;
Fig. 24 shows the modulated differential scanning calorimetry (mDSC) profile of crystalline psilocybin methanol solvate with a small quantity of Form B (commercially available from Quality Chemical Labs);
Fig. 25 shows the mDSC profile of Example 1;
Fig. 26 shows the mDSC profile of Example 3;
Fig. 27 shows the mDSC profile of Example 4;
Fig. 28 shows the mDSC profile of Example 5;
Fig. 29 shows a graph of the dissolution/release rate of psilocybin from Example 1 at 1, 5, and 10 minute timepoints in 0.1 N citric acid (CA) and 1x phosphate buffered saline (PBS);
Fig. 30 shows a graph of the dissolution/release rate of psilocybin from Example 3 at 1, 5, and 10 minute timepoints in 0.1 N citric acid (CA) and 1x phosphate buffered saline (PBS);
Fig. 31 shows a graph of the dissolution/release rate of psilocybin from Example 4 at 1, 5, and 10 minute timepoints in 0.1 N citric acid (CA) and 1x phosphate buffered saline (PBS);
Fig. 32 shows a graph of the dissolution/release rate of psilocybin from Example 5 at 1, 5, and 10 minute timepoints in 0.1 N citric acid (CA) and 1x phosphate buffered saline (PBS);
Fig. 33 shows a graph of the dissolution/release rate of psilocybin from Example 7 at 1, 5, and 10 minute timepoints in 0.1 N citric acid (CA) and 1x phosphate buffered saline (PBS);
Fig. 34 shows a graph of the dissolution/release rate of psilocybin from Example 8 at 1, 5, and 10 minute timepoints in 0.1 N citric acid (CA) and 1x phosphate buffered saline (PBS);
Fig. 35 shows a graph of the dissolution/release rate of psilocybin from Example 9 at 1, 5, and 10 minute timepoints in 0.1 N citric acid (CA) and 1x phosphate buffered saline (PBS);
Fig. 36 shows a graph of the dissolution/release rate of psilocybin from Example 10 at 1, 5, and 10 minute timepoints in 0.1 N citric acid (CA) and 1x phosphate buffered saline (PBS);
Fig. 37 shows a graph of the dissolution/release rate of psilocybin from Example 11 at 1, 5, and 10 minute timepoints in 0.1 N citric acid (CA) and 1x phosphate buffered saline (PBS);
Fig. 38 shows a graph of the dissolution/release rate of psilocybin from Example 12 at 1, 5, and 10 minute timepoints in 0.1 N citric acid (CA) and 1x phosphate buffered saline (PBS);
Fig. 39 shows the high-resolution XRPD pattern of Example 1 after storage at 40°C, 75% relative humidity (RH) for 27 days;
Fig. 40 shows the high-resolution XRPD pattern of Example 1 after storage at 40°C, 15% relative humidity (RH) for 27 days;
Fig. 41 shows the high-resolution XRPD pattern of Example 1 after storage at room temperature for 27 days;
Fig. 42 shows the high-resolution XRPD pattern of Example 3 after storage at 40°C, 75% relative humidity (RH) for 27 days;
Fig. 43 shows the high-resolution XRPD pattern of Example 3 after storage at 40°C, 15% relative humidity (RH) for 27 days;
Fig. 44 shows the high-resolution XRPD pattern of Example 3 after storage at room temperature for 27 days;
Fig. 45 shows the high-resolution XRPD pattern of Example 4 after storage at 40°C, 75% relative humidity (RH) for 27 days;
Fig. 46 shows the high-resolution XRPD pattern of Example 4 after storage at 40°C, 15% relative humidity (RH) for 27 days;
Fig. 47 shows the high-resolution XRPD pattern of Example 4 after storage at room temperature for 27 days;
Fig. 48 shows the high-resolution XRPD pattern of Example 5 after storage at 40°C, 75% relative humidity (RH) for 24 days;
Fig. 49 shows the high-resolution XRPD pattern of Example 5 after storage at 40°C, 15% relative humidity (RH) for 24 days;
Fig. 50 shows the high-resolution XRPD pattern of Example 5 after storage at room temperature for 24 days;
Fig. 51 shows the XRPD pattern of Example 16 after storage at 40°C for 6 hours (t = 6 hr), 24 hours (t = 24 hr), 1 week (t = 1 w), and 4 weeks (t = 4 w);
Fig. 52 shows the mDSC profile of Example 16 after initially being prepared (t = 0);
Fig. 53 shows the mDSC profile of Example 16 after storage at 40°C for 1 week (t = 1 w) and 4 weeks (t = 4 w);
Fig. 54 shows the thermogravimetric analysis (TGA) profile of Example 16 after initially being prepared (t = 0) and after storage at 40°C for 24 hours (t = 24 hr) and 1 week (t = 1 w);
Fig. 55 shows the TGA profile of Example 16 after storage at 40°C for 1 week (t = 1 w) and 4 weeks (t = 4 w);
Fig. 56 shows the XRPD pattern of Example 17 after storage at 40°C for 6 hours (t = 6 hr), 24 hours (t = 24 hr), 1 week (t = 1 w), and 4 weeks (t = 4 w);
Fig. 57 shows the mDSC profile of Example 17 after initially being prepared (t = 0);
Fig. 58 shows the mDSC profile of Example 17 after storage at 40°C for 1 week (t = 1 w) and 4 weeks (t = 4 w);
Fig. 59 shows the TGA profile of Example 17 after initially being prepared (t = 0) and after storage at 40°C for 24 hours (t = 24 hr) and 1 week (t = 1 w);
Fig. 60 shows the TGA profile of Example 17 after storage at 40°C for 1 week (t = 1 w) and 4 weeks (t = 4 w);
Fig. 61 shows the XRPD pattern of Example 18 after storage at 40°C for 6 hours (t = 6 hr), 24 hours (t = 24 hr), 1 week (t = 1 w), and 4 weeks (t = 4 w);
Fig. 62 shows the mDSC profile of Example 18 after initially being prepared (t = 0);
Fig. 63 shows the mDSC profile of Example 18 after storage at 40°C for 1 week (t = 1 w) and 4 weeks (t = 4 w);
Fig. 64 shows the TGA profile of Example 18 after initially being prepared (t = 0) and after storage at 40°C for 24 hours (t = 24 hr) and 1 week (t = 1 w);
Fig. 65 shows the TGA profile of Example 18 after storage at 40°C for 1 week (t = 1 w) and 4 weeks (t = 4 w);
Fig. 66 shows the XRPD pattern of Example 19 after storage at 40°C for 6 hours (t = 6 hr), 24 hours (t = 24 hr), 1 week (t = 1 w), and 4 weeks (t = 4 w);
Fig. 67 shows the mDSC profile of Example 19 after initially being prepared (t = 0) and after being stored at 40°C for 24 hours (t = 24 hr);
Fig. 68 shows the mDSC profile of Example 19 after storage at 40°C for 1 week (t = 1 w) and 4 weeks (t = 4 w);
Fig. 69 shows the TGA profile of Example 19 after initially being prepared (t = 0) and after storage at 40°C for 24 hours (t = 24 hr);
Fig. 70 shows the TGA profile of Example 19 after storage at 40°C for 24 hours (t = 24 hr) and 4 weeks (t = 4 w);
Fig. 71 shows the plasma concentration-time profiles for psilocin after psilocybin dosing with orally disintegrating tablets (ODT) and powder in capsule (PIC) dosage forms;
Fig. 72 shows the exposure comparison between psilocin after psilocybin dosing in ODT and PIC dosage forms as assessed by Cmax; and
Fig. 73 shows the exposure comparison between psilocin after psilocybin dosing in ODT and PIC dosage forms as assessed by AUCinf.

### DETAILED DESCRIPTION

In the following detailed description of the embodiments of the instant disclosure, numerous specific details are set forth in order to provide a thorough understanding of the disclosed embodiments. However, it will be obvious to one skilled in the art that the embodiments of this disclosure may be practiced without these specific details.

### Definitions

Unless defined otherwise, all technical and scientific terms used herein have the same meaning as is commonly understood by one of skill in the art to which this disclosure belongs.

When it is stated that a substituent or group "comprise(s) deuterium" or is "comprising deuterium," it is to be understood that the substituent or group may itself be deuterium, or the substituent or group may contain at least one deuterium substitution in its chemical structure. For example, when substituent "-R" is defined to comprise deuterium, it is to be understood that -R may be -D (-deuterium), or a group such as -CD₃ that is consistent with the other requirements set forth of -R.

As used herein, the term "fatty" describes a compound with a long-chain (linear) hydrophobic portion made up of hydrogen and anywhere from 4 to 26 carbon atoms, which may be fully saturated or partially unsaturated.

The phrases "pharmaceutically acceptable," "physiologically acceptable," and the like, are employed herein to refer to those compounds, materials, compositions, and/or dosage forms which are, within the scope of sound medical judgment, suitable for use in contact with the tissues of human beings without excessive toxicity, irritation, allergic response, or other problem or complication, commensurate with a reasonable benefit/risk ratio. When referencing salts, the phrases "pharmaceutically acceptable salt," "physiologically acceptable salt," and the like, means a salt which is acceptable for administration to a patient, such as a mammal (salts with counterions having acceptable mammalian safety for a given dosage regime). As is well known in the art, such salts can be derived from pharmaceutically acceptable inorganic or organic bases, by way of example, sodium, potassium, calcium, magnesium, ammonium, and tetraalkylammonium salts, and the like, and when the molecule contains a basic functionality, addition salts with inorganic acids, such as hydrochloride, hydrobromide, sulfate, sulfamate, phosphate, nitrate, perchlorate salts, and the like, and addition salts with organic acids, such as formate, tartrate, besylate, mesylate, acetate, maleate, malonate, oxalate, fumarate, benzoate, salicylate, succinate, oxalate, glycolate, hemi-oxalate, hemi-fumarate, propionate, stearate, lactate, citrate, ascorbate, pamoate, hydroxymaleate, phenylacetate, glutamate, 2-acetoxybenzoate, tosylate, ethanedisulfonate, isethionate salts, and the like. The term "salt thereof" means a compound formed when a proton of an acid is replaced by a cation, such as a metal cation or an organic cation and the like. Where applicable, the salt is a pharmaceutically acceptable salt, although this is not required for salts of intermediate compounds that are not intended for administration to a patient. By way of example, salts of the present compounds include those wherein the compound is protonated by an inorganic or organic acid to form a cation, with the conjugate base of the inorganic or organic acid as the anionic component of the salt.

"Solvate" refers to a physical association of a compound or salt of the present disclosure with one or more solvent molecules, whether organic, inorganic, or a mixture of both. This physical association includes hydrogen bonding. In certain instances, the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Some examples of solvents include, but are not limited to, methanol, ethanol, isopropanol, N,N-dimethylformamide, tetrahydrofuran, dimethylsulfoxide, and water. When the solvent is water, the solvate formed is a hydrate (e.g., monohydrate, dihydrate, etc.). Exemplary solvates thus include, but are not limited to, hydrates, methanolates, ethanolates, isopropanolates, etc. Methods of solvation are generally known in the art.

"Stereoisomer" and "stereoisomers" refer to compounds that have same atomic connectivity but different atomic arrangement in space. Stereoisomers include cis-trans isomers, E and Z isomers, enantiomers, and diastereomers.

"Tautomer" refers to alternate forms of a molecule that differ only in electronic bonding of atoms and/or in the position of a proton, such as enol-keto, imine-enamine, and neutral/zwitterionic tautomers, or the tautomeric forms of heteroaryl groups containing a -N=C(H)-NH- ring atom arrangement, such as pyrazoles, imidazoles, benzimidazoles, triazoles, and tetrazoles. Other tautomeric ring atom arrangements are also possible. For example, compounds containing an acid and a base group within the same molecule depicted in neutral form may exist also in a zwitterionic form, as is the case for amino acid/ammonium carboxylate tautomers. Thus, compounds of the present disclosure, e.g., compounds of Formula (I), which are depicted to contain both amino and dihydrogen phosphate functionality in neutral form may also exist in zwitterionic form as the ammonium monohydrogen phosphate zwitterion.

It will be appreciated that the compounds herein can exist in different salt, solvate, stereoisomer, and tautomeric forms, and the present disclosure is intended to include all permutations of salts, solvates, stereoisomers, and tautomers, such as a solvate of a pharmaceutically acceptable salt of a stereoisomer of the subject compound.

As used herein, the term "amorphous" refers to a solid material having substantially no long range order in the position of its molecules-the molecules are arranged in a random manner so that there is effectively no well-defined arrangement, e.g., molecular packing, and no long range order. Amorphous solids are generally isotropic, i.e., exhibit similar properties in all directions and do not have definite melting points. For example, an amorphous material is a solid material having substantially no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. Thus, an "amorphous" subject compound/material is one characterized as having substantially no crystallinity-less than 10% crystallinity, less than 8% crystallinity, less than 6% crystallinity, less than 4% crystallinity, less than 2% crystallinity, less than 1% crystallinity, or 0% crystallinity-i.e., is at least 90%, at least 92%, at least 94%, at least 96%, at least 98%, or 100% amorphous, as determined for example by XRPD. As used herein, the phrase "primarily in amorphous form" means that greater than 50% of the subject compound/material, e.g., a compound of Formula (I) present in a composition, is in amorphous form, for example, as determined by XRPD. It is also noted that the term "primarily in amorphous form" includes the descriptor, "amorphous," defined above, and thus includes compounds/materials which are 100% amorphous (0% crystalline). Other characterization techniques, such as modulated differential scanning calorimetry (mDSC) analysis, Fourier transform infrared spectroscopy (FTIR), and other quantitative methods, may also be employed to determine the percent a subject compound/material is amorphous or crystalline.

The term "stable," "stability," and the like, as used herein includes chemical stability and solid state (physical) stability. The term "chemical stability" means that the compound can be stored in an isolated form, or in the form of a formulation in which it is provided in admixture with for example, pharmaceutically acceptable carriers, diluents or adjuvants as described herein, under normal storage conditions, with little or no chemical degradation or decomposition. "Solid-state stability" means the compound can be stored in an isolated solid form, or the form of a solid formulation in which it is provided, for example, within a polymer(s) and any optional pharmaceutically acceptable excipient (vehicles, carriers, diluents, or adjuvants) as described herein, under normal storage conditions, with little or no solid-state transformation (e.g., hydration, dehydration, solvatization, desolvatization, crystallization, recrystallization or solid-state phase transition). For example, a compound in an amorphous form is deemed stable if at least 50% of the compound remains in the amorphous form at the end of the specified period, e.g., as determined by XRPD, including an amorphous compound which does not form any detectable crystalline peaks by XRPD analysis during the indicated period. In the context of a pharmaceutically or biologically active ingredient (for example, the compound of Formula (1)), the stability may also be measured by the ability of the compound to retain at least 50% of its activity with reference to the beginning of the specified period, or to retain certain physical or chemical properties under certain specified conditions.

A "crystalline psilocybin-based drug" is any solid dosage form formulated with a prodrug of a psilocin-type compound, the prodrug of a psilocin-type compound being primarily in crystalline form (>50% crystalline, e.g., as determined by XRPD). Prodrugs include an alkyl/aryl ester, an α-amino ester (e.g., an amino acid ester), a hemi-ester, a bis-ester, a phosphate ester, a sulfate ester, etc. of a psilocin-type compound, that when administered releases psilocin or a deuterated analog thereof (e.g., a dephosphorylated form of a compound of Formula (I)) as the active component. A crystalline psilocybin-based drug encompasses crystal forms of psilocybin itself, including, but not limited to the following polymorphs (Cambridge structural database (CSD) Reference Codes identified in parentheses): Form A (HATCAK & TAVZID), Form B (TAVZID01), methanol solvate (PSILOC), trihydrate (OKOKAD), and ethanol solvate (KOWHOT); *see* Sherwood et al., "Psilocybin: crystal structure solutions enable phase analysis of prior art and recently patented examples", Acta Cryst. (2022), C78 (1), 36-55; as well as the polymorph described as polymorph A in US 10,519,175.

As used herein, the term "composition" is equivalent to the term "formulation."

The term "treating" or "treatment" as used herein means the treating or treatment of a disease or medical condition in a patient, such as a mammal (particularly a human) that includes: ameliorating the disease or medical condition, such as, eliminating or causing regression of the disease or medical condition in a patient; suppressing the disease or medical condition, for example by, slowing or arresting the development of the disease or medical condition in a patient; or alleviating one or more symptoms of the disease or medical condition in a patient. In some embodiments, prophylactic treatment can result in preventing the disease or medical condition from occurring, in a subject. Any references to methods of treatment by therapy or surgery or *in vivo* diagnosis methods of this description is to be interpreted as a reference to compounds, pharmaceutical compositions and medicaments of the present invention for use in those methods.

A "patient" or "subject," used interchangeably herein, refers to human and non-human subjects, especially mammalian subjects. A patient or subject can have a condition to be treated or can be susceptible to a condition to be treated.

As used herein, and unless otherwise specified, the terms "prevent," "preventing" and "prevention" refer to the prevention of the onset, recurrence or spread of a disease, disorder, or condition, or of one or more symptoms thereof. The terms encompass the inhibition or reduction of a symptom of the particular disease, disorder, or condition. Subjects with familial history of a disease, disorder, or condition, in particular, are candidates for preventive regimens in some embodiments. In addition, subjects who have a history of recurring symptoms are also potential candidates for the prevention. In this regard, the term "prevention" may be interchangeably used with the term "prophylactic treatment."

"Therapeutically effective amount" refers to an amount of a compound sufficient to treat a specified disorder or disease or one or more of its symptoms and/or to prevent the occurrence of the disease or disorder (prophylactically effective amount).

As used herein, and unless otherwise specified, a "prophylactically effective amount" of an active ingredient, is an amount sufficient to prevent a disease, disorder, or condition, or prevent its recurrence. The term "prophylactically effective amount" can encompass an amount that improves overall prophylaxis or enhances the prophylactic efficacy of another prophylactic agent.

The term "administration schedule" is a plan in which the type, amount, period, procedure, etc. of the drug in the drug treatment are shown in time series, and the dosage, administration method, administration order, administration date, and the like of each drug are indicated. The date specified to be administered is determined before the start of the drug administration. The administration is continued by repeating the course with the set of administration schedules as "courses". A "continuous" administration schedule means administration every day without interruption during the treatment course. If the administration schedule follows an "intermittent" administration schedule, then days of administration may be followed by "rest days" or days of non-administration of drug within the course. A "drug holiday" indicates that the drug is not administered in a predetermined administration schedule. For example, after undergoing several courses of treatment, a subject may be prescribed a regulated drug holiday as part of the administration schedule, e.g., prior to re-recommencing active treatment.

As used herein, and unless otherwise specified, a "neuropsychiatric disease or disorder" is a behavioral or psychological problem associated with a known neurological condition, and typically defined as a cluster of symptoms that co-exist. Examples of neuropsychiatric disorders include, but are not limited to, attention deficit disorder, attention deficit hyperactivity disorder, bipolar and manic disorders, depression, or any combinations thereof.

"Inflammatory conditions" or "inflammatory disease," as used herein, refers broadly to chronic or acute inflammatory diseases, including, but not limited to, rheumatic diseases (e.g., rheumatoid arthritis, osteoarthritis, psoriatic arthritis) spondyloarthropathies (e.g., ankylosing spondylitis, reactive arthritis, Reiter's syndrome), crystal arthropathies (e.g., gout, pseudogout, calcium pyrophosphate deposition disease), multiple sclerosis, Lyme disease, polymyalgia rheumatica; connective tissue diseases (e.g., systemic lupus erythematosus, systemic sclerosis, polymyositis, dermatomyositis, Sjogren's syndrome); vasculitides (e.g., polyarteritis nodosa, Wegener's granulomatosis, Churg-Strauss syndrome); inflammatory conditions including consequences of trauma or ischaemia, sarcoidosis; vascular diseases including atherosclerotic vascular disease, atherosclerosis, and vascular occlusive disease (e.g., atherosclerosis, ischaemic heart disease, myocardial infarction, stroke, peripheral vascular disease), and vascular stent restenosis; ocular diseases including uveitis, corneal disease, iritis, iridocyclitis, glaucoma, and cataracts.

As used herein, the term "dispersion" refers to a disperse system in which a first substance, the dispersed phase, is distributed, in discrete units, throughout a second substance (the continuous phase or vehicle). The size of the dispersed phase can vary considerably (e.g., single molecules, colloidal particles of nanometer dimension, to multiple microns in size). In general, the dispersed phases can be solids, liquids, or gases. In the case of a "solid dispersion," the dispersed and continuous phases are both solids, and thus includes any solid composition having at least two components. In the present disclosure, a solid dispersion can include an amorphous solid dispersion, i.e., an amorphous drug in an amorphous polymer, an amorphous drug in crystalline polymer, or an amorphous drug in a mixture of an amorphous polymer and a crystalline polymer. In any case, the polymer may constitute the dispersed phase while the drug constitutes the continuous phase, or, the drug may constitute the dispersed phase while the polymer constitutes the continuous phase. The solid dispersion as disclosed herein may include an active ingredient (for example a compound of Formula (I)) dispersed among at least one other component, for example a polymer. In some embodiments, a solid dispersion includes the compound of Formula (I) molecularly dispersed with a polymer. The solid dispersion can exist, for example, as a one phase/homogenous system, a two phase system, etc.

The term "molecularly dispersed", as used herein, refers to the random distribution of a compound (e.g., compound of Formula (I)) with a polymer. In some embodiments the compound is present in the polymer in a final state of subdivision, *see,* e.g., M. G. Vachon et al., J. Microencapsulation, 14:281-301 (1997) and Vandelli et al., J. Microencapsulation, 10: 55-65 (1993). In some embodiments, a compound (for example, a compound of Formula (I)) may be dispersed within a matrix formed by the polymer in its solid state such that the compound is immobilized in its amorphous form. Whether a compound is molecularly dispersed in a polymer may be evidenced in a variety of ways, e.g., by the resulting solid molecular complex having a single glass transition temperature.

The term "solid molecular complex" as used herein means a solid dispersion that includes compound of Formula (I) molecularly dispersed within a polymer matrix.

The term "immobilize," as used herein with reference to the immobilization of the active ingredient in the polymer matrix, means that molecules of the active ingredient interact with molecules of the polymer in such a way that the molecules of the active ingredient are held in the aforementioned matrix and prevented from crystal nucleation due to lack of mobility. In some embodiments, the polymer may prevent intermolecular hydrogen bonding or weak dispersion forces between two or more active ingredient molecules (e.g., molecules of compound of Formula (I)). *See,* for example, Matsumoro and Zografi, Pharmaceutical Research, Vo. 16, No. 11, p 1722-1728, 1999.

As used herein, the term "and/or" includes any and all combinations of one or more of the associated listed items. As used in the description herein and throughout the claims that follow, the meaning of "a", "an", and "the" includes plural reference as well as the singular reference unless the context clearly dictates otherwise. The term "about" in association with a numerical value means that the value varies up or down by 5%. For example, for a value of about 100, means 95 to 105 (or any value between 95 and 105).

### Pharmaceutical compositions

Disclosed herein is a pharmaceutical composition comprising (i) a therapeutically effective amount of a compound of Formula (I), or a pharmaceutically acceptable salt, a polymorph, stereoisomer, a tautomer, or solvate thereof, and (ii) a polymer. The therapeutically effective amount of a compound of Formula (I) (or a pharmaceutically acceptable salt, a polymorph, stereoisomer, a tautomer, or solvate thereof) and the polymer may be provided in the form of a solid dispersion (e.g., solid molecular complex), for example a solid dispersion in which the compound of Formula (I) (or a pharmaceutically acceptable salt, stereoisomer, a tautomer, or solvate thereof) is stably dispersed in amorphous form in the polymer, sometimes referred to herein as an amorphous solid dispersion (ASD). In addition to the compound of Formula (I) (or a pharmaceutically acceptable salt, a polymorph, stereoisomer, a tautomer, or solvate thereof) and the polymer, the solid dispersion may optionally contain one or more pharmaceutically acceptable excipients, i.e., pharmaceutically acceptable excipients dispersed with the compound of Formula (I) (or a pharmaceutically acceptable salt, a polymorph, stereoisomer, a tautomer, or solvate thereof) and the polymer, as part of the solid dispersion. The pharmaceutically acceptable excipient may be any one or more as set forth hereinafter. The solid dispersion may be used per se as a pharmaceutical composition. Alternatively, a pharmaceutical composition may optionally be formulated with one or more pharmaceutically acceptable excipients which are separate from/not dispersed within the solid dispersion containing the compound of Formula (I) (or a pharmaceutically acceptable salt, a polymorph, stereoisomer, a tautomer, or solvate thereof) and the polymer, i.e., where the pharmaceutically acceptable excipient(s) is considered to be a separate component of the dosage form, and not dispersed with the solid dispersion.

The compound of Formula (I) is provided as follows: 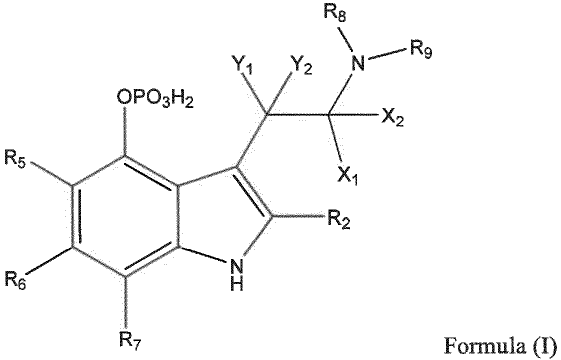 wherein:
R₂, R₅, R₆, and R₇ are independently selected from the group consisting of hydrogen and deuterium,
R₈ and R₉ are independently selected from the group consisting of -CH₃, -CH₂D, -CHD₂, and -CD₃, and
X₁, X₂, Y₁, and Y₂ are independently selected from the group consisting of hydrogen and deuterium.

In some embodiments, R₂, R₅, R₆, and R₇ are independently selected from the group consisting of hydrogen and deuterium. In some embodiments, R₂ is deuterium. In some embodiments, R₂ is hydrogen. In some embodiments, R₅ is deuterium. In some embodiments, R₅ is hydrogen. In some embodiments, R₆ is deuterium. In some embodiments, R₆ is hydrogen. In some embodiments, R₇ is deuterium. In some embodiments, R₇ is hydrogen. R₂, R₅, R₆, and R₇ may be the same, for example, R₂, R₅, R₆, and R₇ may each be hydrogen, or alternatively, R₂, R₅, R₆, and R₇ may each be deuterium. In some embodiments, at least one of R₂, R₅, R₆, and R₇ is deuterium. In some embodiments, at least two of R₂, R₅, R₆, and R₇ are deuterium. In some embodiments, at least three of R₂, R₅, R₆, and R₇ are deuterium. In some embodiments, R₂, R₅, R₆, and R₇ are deuterium. In some embodiments, R₂, R₅, R₆, and R₇ are hydrogen.

In some embodiments, R₈ and R₉ are independently selected from the group consisting of -CH₃, -CH₂D, -CHD₂, and -CD₃. R₈ and R₉ may be the same, or different. In some embodiments, R₈ and R₉ are the same. In some embodiments, R₈ and R₉ are different. In some embodiments, R₈ and R₉ are independently selected from the group consisting of -CH₃ and -CD₃. In some embodiments, R₈ and R₉ are methyl (-CH₃). In some embodiments, R₈ and R₉ are a partially deuterated methyl group, i.e., -CDH₂ or -CD₂H. In some embodiments, R₈ and R₉ are a fully deuterated methyl group (-CD₃). In some embodiments, at least one of R₈ and R₉ is -CD₃.

In some embodiments, X₁, X₂, Y₁, and Y₂ are independently selected from the group consisting of hydrogen and deuterium. X₁ and X₂ may be the same, or different. In some embodiments, X₁ and X₂ are the same. In some embodiments, X₁ and X₂ are hydrogen. In some embodiments, X₁ and X₂ are deuterium. In some embodiments, X₁ is deuterium and X₂ is hydrogen.

Y₁ and Y₂ may be the same, or different. In some embodiments, Y₁ and Y₂ are the same. In some embodiments, Y₁ and Y₂ are hydrogen. In some embodiments, Y₁ and Y₂ are deuterium. In some embodiments, Y₁ is deuterium and Y₂ is hydrogen. In some embodiments, X₁, X₂, Y₁, and Y₂ are hydrogen. In some embodiments, X₁, X₂, Y₁, and Y₂ are deuterium.

In some embodiments, X₁, X₂, Y₁, Y₂, R₂, R₅, R₆, and R₇ are each hydrogen, and R₈ and R₉ are each -CH₃. In some embodiments, X₁, X₂, Y₁, Y₂, R₂, R₅, R₆, and R₇ are each hydrogen, and R₈ and R₉ comprise deuterium (e.g., are -CD₃ groups or a partially deuterated methyl group). In some embodiments, at least one of X₁, X₂, Y₁, Y₂, R₂, R₅, R₆, R₇, R₈, and R₉ comprises deuterium. In some embodiments, at least X₁, X₂, R₈, and R₉ comprise deuterium. In some embodiments, at least X₁, X₂, Y₁, Y₂, R₈, and R₉ comprise deuterium. In some embodiments, X₁, X₂, Y₁, and Y₂ are deuterium, and R₈ and R₉ are a fully deuterated methyl group (-CD₃).

The compounds of Formula (I) may contain a stereogenic center. In such cases, the compounds may exist as different stereoisomeric forms, even though Formula (I) is drawn without reference to stereochemistry. Accordingly, the present disclosure includes all possible stereoisomers and includes not only racemic compounds but the individual enantiomers (enantiomerically pure compounds), individual diastereomers (diastereomerically pure compounds), and their non-racemic mixtures as well. When a compound is desired as a single enantiomer, such may be obtained by, e.g., stereospecific synthesis, as is known in the art.

In some embodiments, the compounds described herein, e.g., compounds of Formula (I), are non-stereogenic. In some embodiments, the compounds described herein, e.g., compounds of Formula (I), are racemic. In some embodiments, the compounds described herein, e.g., compounds of Formula (I), are enantiomerically enriched (one enantiomer is present in a higher percentage), including enantiomerically pure. In some embodiments, the compounds described herein, e.g., compounds of Formula (I), are provided as a single diastereomer. In some embodiments, the compounds described herein, e.g., compounds of Formula (I), are provided as a mixture of diastereomers. When provided as a mixture of diastereomers, the mixtures may include equal mixtures, or mixtures which are enriched with a particular diastereomer (one diastereomer is present in a higher percentage than another).

In some embodiments, the compound of Formula (I) is an agonist of a serotonin 5-HT₂ receptor. In some embodiments, the compound of Formula (I) is an agonist of a serotonin 5-HT_{2A} receptor.

In some embodiments, the compound of Formula (I) is selected from the group consisting of: 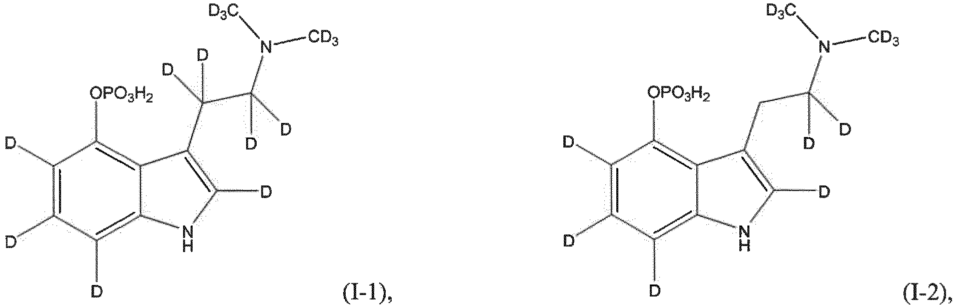 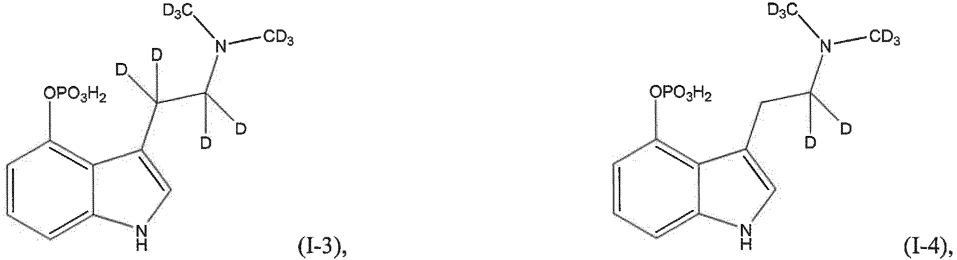 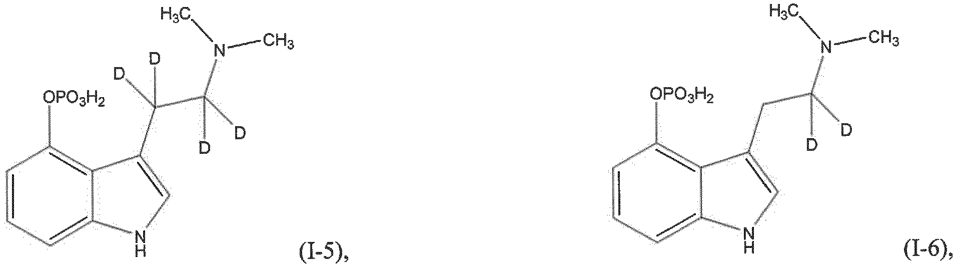 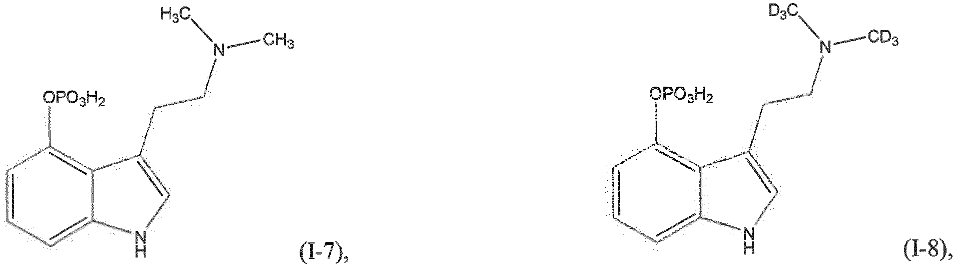 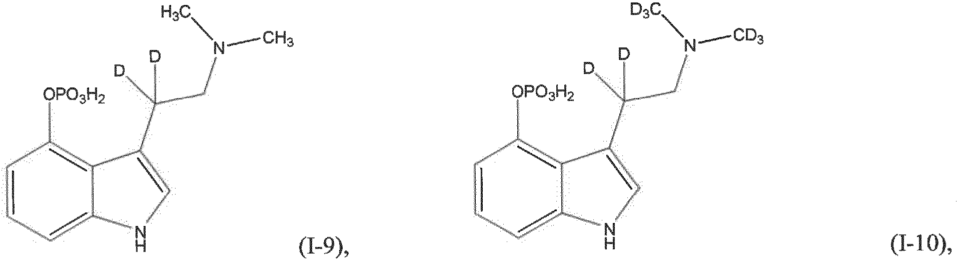 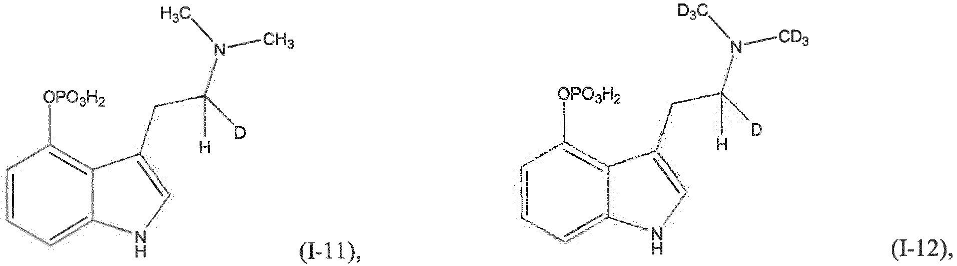 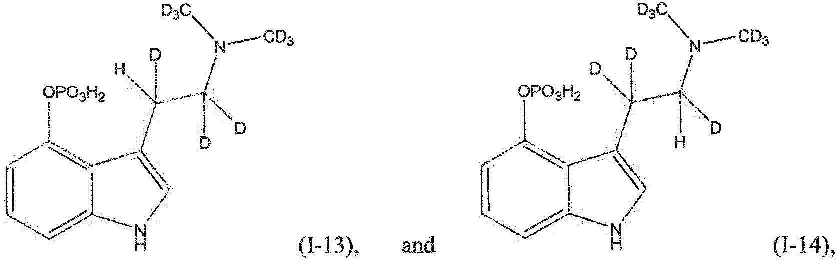 or a pharmaceutically acceptable salt, a polymorph, stereoisomer, a tautomer, or solvate thereof.

The compound number, IUPAC name, and substituent listing for the above-identified compounds are provided in Table 1.

**Table 1. Exemplary compounds of Formula (I)**

| Compound identifier and name | | Formula (I) | | | | | | |
|---|---|---|---|---|---|---|---|---|
| | | X₁,X₂ | Y₁,Y₂ | R₂ | R₅ | R₆ | R₇ | R₈,R₉ |
| **I-1** | 3-(2-(bis(methyl-*d*₃)amino)ethyl-1,1,2,2-*d*₄)-1H-indol-4-yl-2,5,6,7-*d*₄ dihydrogen phosphate | D,D | D,D | D | D | D | D | -CD₃,-CD₃ |
| **I-2** | 3-(2-(bis(methyl-*d*₃)amino)ethyl-2,2-*d*₂)-1H-indol-4-yl-2,5,6,7-*d*₄ dihydrogen phosphate | D,D | H,H | D | D | D | D | -CD₃,-CD₃ |
| **I-3** | 3-(2-(bis(methyl-*d*₃)amino)ethyl-1,1,2,2-*d*₄)-1H-indol-4-yl dihydrogen phosphate | D,D | D,D | H | H | H | H | -CD₃,-CD₃ |
| **I-4** | 3-(2-(bis(methyl-*d*₃)amino)ethyl-2,2-*d*₂)-1H-indol-4-yl dihydrogen phosphate | D,D | H,H | H | H | H | H | -CD₃,-CD₃ |
| **I-5** | 3-(2-(dimethylamino)ethyl-1,1,2,2-*d*₄)-1H-indol-4-yl dihydrogen phosphate | D,D | D,D | H | H | H | H | -CH₃,-CH₃ |
| **I-6** | 3-(2-(dimethylamino)ethyl-2,2-*d*₂)-1H-indol-4-yl dihydrogen phosphate | D,D | H,H | H | H | H | H | -CH₃,-CH₃ |
| **I-7** | 3-(2-(dimethylamino)ethyl)-1H-indol-4-yl dihydrogen phosphate | H,H | H,H | H | H | H | H | -CH₃,-CH₃ |
| **I-8** | 3-(2-(bis(methyl-*d*₃)amino)ethyl)-1H-indol-4-yl dihydrogen phosphate | H,H | H,H | H | H | H | H | -CD₃,-CD₃ |
| **I-9** | 3-(2-(dimethylamino)ethyl-1,1-*d*₂)-1H-indol-4-yl dihydrogen phosphate | H,H | D,D | H | H | H | H | -CH₃,-CH₃ |
| **I-10** | 3-(2-(bis(methyl-*d*₃)amino)ethyl-1,1-*d*₂)-1H-indol-4-yl dihydrogen phosphate | H,H | D,D | H | H | H | H | -CD₃,-CD₃ |
| **I-11** | 3-(2-(dimethylamino)ethyl-2-*d*)-1H-indol-4-yl dihydrogen phosphate | H,D | H,H | H | H | H | H | -CH₃,-CH₃ |
| **I-12** | 3-(2-(bis(methyl-*d*₃)amino)ethyl-2-*d*)-1H-indol-4-yl dihydrogen phosphate | H,D | H,H | H | H | H | H | -CD₃,-CD₃ |
| **I-13** | 3-(2-(bis(methyl-*d*₃)amino)ethyl-1,2,2-*d*₃)-1H-indol-4-yl dihydrogen phosphate | D,D | H,D | H | H | H | H | -CD₃,-CD₃ |
| **I-14** | 3-(2-(bis(methyl-*d*₃)amino)ethyl-1,1,2-*d*₃)-1H-indol-4-yl dihydrogen phosphate | H,D | D,D | H | H | H | H | -CD₃,-CD₃ |

In some embodiments, the compound of Formula (I) is 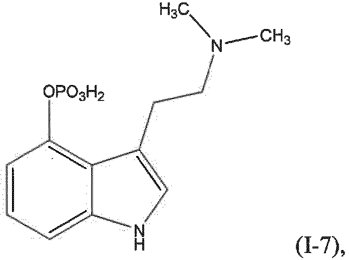 or a pharmaceutically acceptable salt, tautomer, or solvate thereof.

In some embodiments, the compounds of the present disclosure are provided in amorphous form, e.g., as determined by XRPD and/or mDSC. Accordingly, pharmaceutical compositions may be prepared from compounds of Formula (I), in one or more amorphic forms, and may be used for treatment as set forth herein. In some embodiments, the compound of Formula (I) is an amorphous form of 3-(2-(bis(methyl-*d*₃)amino)ethyl-1,1,2,2-*d*₄)-1H-indol-4-yl-2,5,6,7-*d*₄ dihydrogen phosphate (I-1), as determined by X-ray powder diffraction. In some embodiments, the compound of Formula (I) is an amorphous form of 3-(2-(bis(methyl-*d*₃)amino)ethyl-2,2-*d*₂)-1H-indol-4-yl-2,5,6,7-*d*₄ dihydrogen phosphate (I-2), as determined by X-ray powder diffraction. In some embodiments, the compound of Formula (I) is an amorphous form of 3-(2-(bis(methyl-*d*₃)amino)ethyl-1,1,2,2-*d*₄)-1H-indol-4-yl dihydrogen phosphate (**I-3**), as determined by X-ray powder diffraction. In some embodiments, the compound of Formula (I) is an amorphous form of 3-(2-(bis(methyl-*d*₃)amino)ethyl-2,2-*d*₂)-1H-indol-4-yl dihydrogen phosphate (**I-4**), as determined by X-ray powder diffraction. In some embodiments, the compound of Formula (I) is an amorphous form of 3-(2-(dimethylamino)ethyl-1,1,2,2-*d*₄)-1H-indol-4-yl dihydrogen phosphate (**I-5**), as determined by X-ray powder diffraction. In some embodiments, the compound of Formula (I) is an amorphous form of 3-(2-(dimethylamino)ethyl-2,2-*d*₂)-1H-indol-4-yl dihydrogen phosphate (**I-**6), as determined by X-ray powder diffraction. In some embodiments, the compound of Formula (I) is an amorphous form of 3-(2-(dimethylamino)ethyl)-1H-indol-4-yl dihydrogen phosphate **(I-**7), as determined by X-ray powder diffraction. In some embodiments, the compound of Formula (I) is an amorphous form of 3-(2-(bis(methyl-*d*₃)amino)ethyl)-1H-indol-4-yl dihydrogen phosphate **(I-8),** as determined by X-ray powder diffraction. In some embodiments, the compound of Formula (I) is an amorphous form of 3-(2-(dimethylamino)ethyl-1,1-*d*₂)-1H-indol-4-yl dihydrogen phosphate **(I-9),** as determined by X-ray powder diffraction. In some embodiments, the compound of Formula (I) is an amorphous form of 3-(2-(bis(methyl-*d*₃)amino)ethyl-1,1-*d*₂)-1H-indol-4-yl dihydrogen phosphate **(I-10),** as determined by X-ray powder diffraction. In some embodiments, the compound of Formula (I) is an amorphous form of 3-(2-(dimethylamino)ethyl-2-*d*)-1H-indol-4-yl dihydrogen phosphate **(I-11),** as determined by X-ray powder diffraction. In some embodiments, the compound of Formula (I) is an amorphous form of 3-(2-(bis(methyl-*d*₃)amino)ethyl-2-*d*)-1H-indol-4-yl dihydrogen phosphate **(I-12),** as determined by X-ray powder diffraction. In some embodiments, the compound of Formula (I) is an amorphous form of 3-(2-(bis(methyl-*d*₃)amino)ethyl-1,2,2-*d*₃)-1H-indol-4-yl dihydrogen phosphate **(I-13),** as determined by X-ray powder diffraction. In some embodiments, the compound of Formula (I) is an amorphous form of 3-(2-(bis(methyl-*d*₃)amino)ethyl-1,1,2-*d*₃)-1H-indol-4-yl dihydrogen phosphate **(I-14),** as determined by X-ray powder diffraction.

Amorphous forms of the compounds of Formula (I) may be advantageous in terms of higher aqueous solubility and dissolution rates in water, compared to crystalline forms, thereby enabling rapid systemic absorption, higher bioavailability, and better control/more predictable therapeutic onset. Further, in some embodiments, pharmaceutical compositions may be prepared which comprise the amorphous forms of the compounds of Formula (I), e.g., as a solid dispersion (e.g., a solid molecular complex). The solid dispersion (e.g., solid molecular complex) of the present disclosure, such as those set forth herein, may act to stabilize the amorphous forms of the compounds of Formula (I), which tend to be unstable and have a tendency to crystallize. Accordingly, the solid dispersion (e.g., solid molecular complex) can be used to stabilize and deliver these amorphous forms to subjects in need of treatment, e.g., for the treatment of a condition or disease associate with a serotonin 5-HT₂ receptor.

Also disclosed herein is a pharmaceutically acceptable salt of the compound of Formula (I), or a pharmaceutically acceptable polymorph, stereoisomer, a tautomer, or solvate thereof. The acid used to form the pharmaceutically acceptable salt of the compound of Formula (I) may be a monoacid, a diacid, a triacid, a tetraacid, or may contain a higher number of acid groups. The acid groups may be, e.g., a carboxylic acid, a sulfonic acid, a phosphonic acid, or other acidic moieties containing at least one replaceable hydrogen atom. Examples of acids for use in the preparation of the pharmaceutically acceptable (acid addition) salts disclosed herein include, but are not limited to, acetic acid, 2,2-dichloroacetic acid, phenylacetic acid, acylated amino acids, alginic acid, ascorbic acid, L-aspartic acid, sulfonic acids (e.g., benzenesulfonic acid, camphorsulfonic acid, (+)-(1S)-camphor-10-sulfonic acid, ethane-1,2-disulfonic acid, ethanesulfonic acid, 2-hydroxy-ethanesulfonic acid, methanesulfonic acid, naphthalene-2-sulfonic acid, naphthalene-1,5-disulfonic acid, p-toluenesulfonic acid, ethanedisulfonic acid, etc.), benzoic acids (e.g., benzoic acid, 4-acetamidobenzoic acid, 2-acetoxybenzoic acid, salicylic acid, 4-amino-salicylic acid, gentisic acid, etc.), boric acid, (+)-camphoric acid, cinnamic acid, citric acid, cyclamic acid, cyclohexanesulfamic acid, dodecylsulfuric acid, formic acid, fumaric acid, galactaric acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (-)-D-lactic acid, (±)-DL-lactic acid, lactobionic acid, maleic acid, malic acid, (-)-L-malic acid, (+)-D-malic acid, hydroxymaleic acid, malonic acid, (±)-DL-mandelic acid, isethionic acid, 1-hydroxy-2-naphthoic acid, nicotinic acid, nitric acid, orotic acid, oxalic acid, pamoic acid, perchloric acid, phosphoric acid, L-pyroglutamic acid, saccharic acid, succinic acid, sulfuric acid, sulfamic acid, tannic acid, tartaric acids (e.g., DL-tartaric acid, (+)-L-tartaric acid, (-)-D-tartaric acid), thiocyanic acid, propionic acid, valeric acid, and fatty acids (including fatty mono- and di- acids, e.g., adipic (hexandioic) acid, lauric (dodecanoic) acid, linoleic acid, myristic (tetradecanoic) acid, capric (decanoic) acid, stearic (octadecanoic) acid, oleic acid, caprylic (octanoic) acid, palmitic (hexadecenoic) acid, sebacic acid, undecylenic acid, caproic acid, etc.).

Methods for preparing pharmaceutically acceptable salt forms of pharmaceutical compounds are known by those of ordinary skill in the art. In some embodiments, the method includes:
(a) suspending the compound of Formula (I) in a solvent or mixture of solvents;
(b) contacting an acid with the compound of Formula (I) to provide a mixture;
(c) optionally heating the mixture;
(d) optionally cooling the mixture; and
(e) isolating the salt.

Various solvents may be used in the disclosed methods, including one or more protic solvents, one or more aprotic solvents, or mixtures thereof. In some embodiments, the solvent(s) used in the method of preparing the salt is/are a protic solvent(s). In some embodiments, the solvent used in the method of preparing the salt is selected from the group consisting of methanol, ethanol, propanol, isopropanol, butanol, 2-butanol, acetone, butanone, dioxanes (1,4-dioxane), water, tetrahydrofuran (THF), acetonitrile (MeCN), ether solvents (e.g., t-butylmethyl ether (TBME)), hexane, heptane, octane, and combinations thereof. In some embodiments, the solvent is ethanol. In some embodiments, the solvent is 1,4-dioxane. In some embodiments, the solvent is acetonitrile. In some embodiments, the solvent is tetrahydrofuran.

Suitable acids for use in the preparation of pharmaceutically acceptable acid addition salts may include those described heretofore. The acid may be an inorganic acid such as hydrochloric acid, or an organic acid, with organic acids being preferred. In some embodiments, the acid is an organic acid selected from the group consisting of ascorbic acid, citric acid, fumaric acid, maleic acid, malonic acid, (-)-L-malic acid, (+)-L-tartaric acid, methanesulfonic acid, benzenesulfonic acid, toluenesulfonic acid, benzoic acid, salicylic acid, succinic acid, oxalic acid, D-glucuronic acid, glutaric acid salt, and acetic acid. In some embodiments, the acid is an organic acid selected from the group consisting of benzenesulfonic acid, (+)-L-tartaric acid, fumaric acid, acetic acid, citric acid, malonic acid, succinic acid, oxalic acid, benzoic acid, and salicylic acid, with benzenesulfonic acid, succinic acid, and benzoic acid being preferred. In some embodiments, the acid is a fatty acid, such as adipic (hexandioic) acid, lauric (dodecanoic) acid, linoleic acid, myristic (tetradecanoic) acid, capric (decanoic) acid, stearic (octadecanoic) acid, oleic acid, caprylic (octanoic) acid, palmitic (hexadecenoic) acid, sebacic acid, undecylenic acid, caproic acid, etc., with particular mention being made to adipic (hexandioic) acid, lauric (dodecanoic) acid, linoleic acid, myristic (tetradecanoic) acid, capric (decanoic) acid, stearic (octadecanoic) acid, oleic acid, and caprylic (octanoic) acid.

In some embodiments, a stoichiometric (or superstoichiometric) quantity of the acid is contacted with the compound of Formula (I). In some embodiments, a sub-stoichiometric (e.g., 0.5 molar equivalents) quantity of the acid is contacted with the compound of Formula (I). The use of sub-stoichiometric quantities of the acid may be desirable when, for example, the acid contains at least two acidic protons (e.g., two or more carboxylic acid groups) and the target salt is a hemi-acid salt.

In some embodiments, the mixture is heated, e.g., refluxed, prior to cooling.

In some embodiments, the mixture is cooled and the salt is precipitated out of the solution. In some embodiments, the salt is precipitated out of solution in crystalline form. In some embodiments, the salt is precipitated out of solution in amorphous form.

Isolation of the salt may be performed by various well-known isolation techniques, such as filtration, decantation, and the like. In some embodiments, the isolating step includes filtering the mixture.

After isolation, additional crystallization and/or recrystallization steps may also optionally be performed, if desired, for example to increase purity, crystallinity, etc.

In some embodiments, compounds of the present disclosure, e.g., a compound of Formula (I), or any pharmaceutically acceptable salt, polymorph, stereoisomer, or tautomer thereof, is in the form of a solvate. Examples of solvate forms include, but are not limited to, hydrates, methanolates, ethanolates, isopropanolates, etc., with hydrates and ethanolates being preferred. The solvate may be formed from stoichiometric or nonstoichiometric quantities of solvent molecules. Solvates of the compounds herein may be in the form of isolable solvates. In one nonlimiting example, as a hydrate, the compound may be a monohydrate, a dihydrate, etc. Solvates of the compounds herein also include solution-phase forms. Thus, in some embodiments, the present disclosure provides solution-phase compositions of the compounds of the present disclosure, or any pharmaceutically acceptable salts, polymorphs, stereoisomers, or tautomers thereof, which are in solvated form, preferably fully solvated form.

### Solid Dispersion

Disclosed herein is a solid dispersion (e.g., solid molecular complex) which includes a compound of Formula (I), or a pharmaceutically acceptable salt, a polymorph, stereoisomer, a tautomer, or solvate thereof, and a polymer. In some embodiments, a solid dispersion that includes the compound of Formula (I) and a polymer is provided. In some embodiments, a solid molecular complex that includes the compound of Formula (I) and a polymer is provided. In addition to the compound of Formula (I), or a pharmaceutically acceptable salt, a polymorph, stereoisomer, a tautomer, or solvate thereof, and the polymer, the solid dispersion (e.g., solid molecular complex) may optionally contain one or more pharmaceutically acceptable excipients.

The solid dispersion (e.g., solid molecular complex) may comprise a single compound of Formula (I), or a pharmaceutically acceptable salt, a polymorph, stereoisomer, or solvate thereof, or a mixture of compounds of Formula (I), or their salts, polymorphs, stereoisomers, or solvates. The solid dispersion (e.g., solid molecular complex) may be formed from an isotopologue mixture of the disclosed compounds. In some embodiments, a subject compound of Formula (I) may be present in the solid dispersion (e.g., solid molecular complex) at a purity of at least 50% by weight, at least 60% by weight, at least 70% by weight, at least 80% by weight, at least 90% by weight, at least 95% by weight, at least 99% by weight, based on a total weight of isotopologues of the compound of Formula (I) present in the solid dispersion (e.g., solid molecular complex). For example, a solid dispersion (e.g., solid molecular complex) formulated with psilocybin *d-10* (compound **I-3**; 3-(2-(bis(methyl-*d*₃)amino)ethyl-1,1,2,2-*d*₄)-1H-indol-4-yl dihydrogen phosphate), as the subject compound, may additionally contain isotopologues of the subject compound, e.g., psilocybin *d-9,* psilocybin *d-8,* etc., or salt forms, polymorphs, stereoisomers, solvates, or mixtures thereof. In some embodiments, the solid dispersion (e.g., solid molecular complex) is substantially free of other isotopologues of the compound, e.g., the solid dispersion has less than 20, 15, 10, 9, 8, 7, 6, 5, 4, 3, 2 or 1 or 0.5 mole percent of other isotopologues of the compound.

In some embodiments, any position in the compound having deuterium has a minimum deuterium incorporation of at least 10 atom %, at least 20 atom %, at least 25 atom %, at least 30 atom %, at least 40 atom %, at least 45 atom %, at least 50 atom %, at least 60 atom %, at least 70 atom %, at least 80 atom %, at least 90 atom %, at least 95 atom %, at least 99 atom % at the site of deuteration.

The solid dispersion (e.g., solid molecular complex) may be formulated with an enantiomerically pure compound of the present disclosure, e.g., a compound of Formula (I), or a racemic mixture of the compounds. As described herein, a racemic compound of Formula (I) may contain about 50% of the R- and S-stereoisomers based on a molar ratio (about 48 to about 52 mol %, or about a 1:1 ratio)) of one of the isomers. In some embodiments, the solid dispersion may be formed from combining separately produced compounds of the R- and S-stereoisomers in an approximately equal molar ratio (e.g., about 48 to 52%). In some embodiments, the solid dispersion may contain a mixture of separate compounds of the R- and S-stereoisomers in different ratios. In some embodiments, the solid dispersion contains an excess (greater than 50%) of the R-enantiomer. Suitable molar ratios of R/S may be from about 1.5:1, 2:1, 3:1, 4:1, 5:1, 10:1, or higher. In some embodiments, a solid dispersion may contain an excess of the S-enantiomer, with the ratios provided for R/S reversed. Other suitable amounts of R/S may be selected. For example, the R-enantiomer may be enriched, e.g., may be present in amounts of at least about 55% to 100%, or at least 65%, at least 75%, at least 80%, at least 85%, at least 90%, about 95%, about 98%, or 100%. In some embodiments, the S-enantiomer may be enriched, e.g., in amounts of at least about 55% to 100%, or at least 65%, at least 75%, at least 80%, at least 85%, at least 90%, about 95%, about 98%, or 100%. Ratios between all these exemplary embodiments as well as greater than and less than them while still within the disclosure, all are included. Solid dispersions may contain a mixture of the racemate and a separate compound of Formula (I), or a pharmaceutically acceptable salt, a polymorph, stereoisomer, or solvate thereof.

The solid dispersion (e.g., solid molecular complex) may be formulated with one or more polymorphs of the compounds of Formula (I), including crystalline and/or amorphous polymorphs. In some embodiments, the solid dispersion (e.g., solid molecular complex) comprises a compound of Formula (I) (or a pharmaceutically acceptable salt, stereoisomer, a tautomer, or solvate thereof) primarily in amorphous form. In some embodiments, the solid dispersion (e.g., solid molecular complex) comprises a compound of Formula (I) (or a pharmaceutically acceptable salt, stereoisomer, a tautomer, or solvate thereof) in amorphous form. In some embodiments, only the amorphous form of the compound of Formula (I) is present in the solid dispersion, e.g., solid dispersions in which no crystalline forms of the compound of Formula (I) are detectable, for example by XRPD.

In some embodiments, the compound of Formula (I) is stable in the solid dispersion (e.g., solid molecular complex) for at least 3 weeks at 25°C, or for at least 1 month at 25°C, or for at least 2 months at 25°C, or for at least 3 months at 25°C, or for at least 4 months at 25°C, or for at least 5 months at 25°C, or for at least 6 months at 25°C, or for at least 9 months at 25°C, or for at least 12 months at 25°C, or for at least 15 months at 25°C, or for at least 18 months at 25°C, or for at least 24 months at 25°C. In some embodiments, the compound of Formula (I) is immobilized so that it is primarily in amorphous form within the solid dispersion (e.g., solid molecular complex) for at least 2 weeks of storage at 40°C and 75% relative humidity, or for at least 3 weeks of storage at 40°C and 75% relative humidity, or for at least 1 month of storage at 40°C and 75% relative humidity, or for at least 2 months of storage at 40°C and 75% relative humidity, or for at least 3 months of storage at 40°C and 75% relative humidity, or for at least 4 months of storage at 40°C and 75% relative humidity, or for at least 5 months of storage at 40°C and 75% relative humidity, or for at least 6 months of storage at 40°C and 75% relative humidity, or for at least 7 months of storage at 40°C and 75% relative humidity, or for at least 8 months of storage at 40°C and 75% relative humidity, or for at least 9 months of storage at 40°C and 75% relative humidity, or for at least 10 months of storage at 40°C and 75% relative humidity, or for at least 11 months of storage at 40°C and 75% relative humidity, or for at least 12 months of storage at 40° C and 75% relative humidity. Accordingly, the compound of Formula (I) is immobilized so that greater than 50%, or greater than 55%, or greater than 60%, or greater than 65%, or greater than 70%, or greater than 75%, or greater than 80%, or greater than 85%, or greater than 90%, or greater than 95%, or greater than 99% of the compound present in a composition is in amorphous form, as determined for example by XRPD, mDSC, etc.

The compound of Formula (I) may be stable within the solid dispersion (e.g., solid molecular complex), in terms of the compound retaining its biological activity and/or retaining certain physical or chemical properties under certain specified conditions. In some embodiments, the compound of Formula (I) is stable if the activity at the end of the specified period is at least 50%, or at least 60%, or at least 70%, or at least 75%, or at least 80%, or at least 85%, or at least 90%, or at least 95%, or at least 98% of the activity of the compound at the beginning of the specified period. In some embodiments, the compound of Formula (I) in an amorphous form is stable if at least 50%, or at least 60%, or at least 70%, or at least 75%, or at least 80%, or at least 85%, or at least 90%, or at least 95%, or at least 98%, or at least 99% of the compound remains in the amorphous form at the end of the specified period. In further embodiments, an amorphous compound of Formula (I) is stable if it does not form any detectable crystalline peaks in powder XRD profiles during the indicated period. In some embodiments, the specified period is 6 hours, 12 hours, 24 hours, 2 days, 3 days, 4 days, 5 days, 6 days, 1 week, 2 weeks, 3 weeks, 4 weeks, 5 weeks, 6 weeks, 7 weeks, 2 months, 3 months, 4 months, 5 months, 6 months, 7 months, 8 months, 9 months, 10 months, 11 months, 1 year, or longer, or any range therebetween.

In some embodiments, the solid dispersion has a glass transition (Tg) onset of from about 110°C, from about 115°C, from about 120°C, from about 125°C, from about 130°C, from about 135°C, from about 140°C, and up to about 200°C, up to about 195°C, up to about 190°C, up to about 185°C, up to about 180°C, up to about 175°C, up to about 170°C, up to about 165°C, up to about 160°C, up to about 155°C, up to about 150°C, up to about 145°C, as determined by mDSC.

In some embodiments, the solid dispersion has a heat capacity change (ΔCp), in J/(g·°C), of from about 0.1, from about 0.15, from about 0.2, from about 0.25, from about 0.3, from about 0.35, and up to about 0.75, up to about 0.70, up to about 0.65, up to about 0.6, up to about 0.55, up to about 0.5, up to about 0.45, up to about 0.4, as determined by mDSC.

Methods of making solid dispersions (e.g., solid molecular complexes) are also disclosed herein. In some embodiments, the amorphous form of a compound of Formula (I) can be prepared by intermediately transforming a crystalline material into a non-crystalline form, e.g., a melt or a solution. Then, the amorphous material can be prepared by cooling (e.g., quench cooling) of the melt, rapid precipitation from solution, or evaporative techniques, e.g., spray drying or freeze-drying. In some embodiments, the amorphous form is formed by direct solid conversion from the crystalline to the amorphous form, e.g., milling. In some embodiments, the amorphous form can be formed by freeze-drying (lyophilization), spray drying, dehydration, milling, melt quenching, or hot melt extrusion.

In some embodiments, the amorphous state results in the compound of Formula (I) being molecularly dispersed in an inert carrier, e.g., a polymer. In some embodiments, achieving this amorphous state includes one or more of solvent evaporation, spray drying, and melt extrusion. Melt extrusion can use a twin screw extruder to combine an active ingredient (for example, the compound of Formula (I)) with an inert carrier (e.g., a polymer) to form a solid dispersion. Typically, the twin screw extruder is heated to facilitate mixing of the active ingredient with the inert carrier. In some embodiments, the active ingredient (a compound of Formula (I)) is an agonist of a serotonin 5-HT₂ receptor. In some embodiments, the active ingredient (a compound of Formula (I)) is an agonist of a serotonin 5-HT_{2A} receptor.

Amorphous forms of the compound of Formula (I) have improved solubility in water as compared to the crystalline form but are unstable and have a tendency to crystallize. Thus, it is desired to formulate the compound of Formula (I) so that it may stably exist primarily in amorphous form, including in amorphous form.

Solid dispersions that contain the compound of Formula (I) in crystalline form can be prepared through physical mixing processes such as admixing a crystalline compound of Formula (I) with a polymer (admixtures). However, after significant experimentation, the inventors have discovered that such admixture processing is generally not sufficient for the preparation of solid dispersions containing the compound(s) of Formula (I) in amorphous form (or ASDs). Instead, solid dispersions containing amorphous forms of a compound of Formula (I) may be accessed by intermediately transforming a crystalline material into a non-crystalline form, e.g., a melt or a solution, followed by cooling, evaporating, precipitating, or freeze-drying techniques as discussed herein.

In some embodiments, pharmaceutical compositions including the compound of Formula (I) in an amorphous form are provided. In some embodiments, pharmaceutical compositions of the compound of Formula (I), in which the compound of Formula (I) exists stably in amorphous form, may be accomplished, for example, by immobilizing the compound within a matrix formed by a polymer. Accordingly, in some embodiments, the present disclosure provides solid dispersions (e.g., solid molecular complexes) that include the compound of Formula (I). For example, the compound of Formula (I) may be dispersed within a matrix formed by a polymer in its solid state such that it is immobilized in its amorphous form. In some embodiments, the polymer may prevent intramolecular hydrogen bonding or weak dispersion forces between two or more drug molecules (e.g., the compound of Formula (I)). In some embodiments, the solid dispersion provides for a large surface area, thus further allowing for improved dissolution and bioavailability of the compound of Formula (I). In some embodiments, a solid dispersion (e.g., solid molecular complex) includes a therapeutically effective amount of the compound of Formula (I).

In some embodiments, a weight ratio of the compound of Formula (I) within the solid dispersion (e.g., solid molecular complex) to the polymer therein is from about 0.5:9.5, from about 1:9, from about 1.5:8.5, from about 2:8, from about 2.5:7.5, from about 3:7, and up to about 9:1, up to about 8:2, up to about 7.5:2.5, up to about 7:3, up to about 6.5:3.5, up to about 6:4, up to about 5.5:4.5, up to about 5:5, up to about 4.5:5.5, up to about 4:6, up to about 3.7:6.3, up to about 3.5:6.5, or any range therebetween. Other weight ratios above or below these ranges may be utilized, however, in most cases, the weight ratio of the compound of Formula (I) to the polymer in the solid dispersion is equal to or less than 5:5, for example, from about 1.5:8.5 to about 4.5:5.5, from about 2:8 to about 4:6, or about 3:7 to about 3.7:6.3.

In some embodiments, the compound of Formula (I) may be present in the solid dispersion in an amount of from about 0.1 wt.%, from about 0.5 wt.%, from about 1 wt.%, from about 5 wt.%, from about 10 wt.%, from about 15 wt.%, from about 20 wt.%, from about 25 wt.%, from about 30 wt.%, and up to about 90 wt.%, up to about 85 wt.%, up to about 80 wt.%, up to about 75 wt.%, up to about 70 wt.%, up to about 65 wt.%, up to about 60 wt.%, up to about 55 wt.%, up to about 50 wt.%, up to about 45 wt.%, up to about 40 wt.%, up to about 35 wt.%, based on a total weight of the solid dispersion, or any range therebetween. For example, the compound of Formula (I) may be present in the solid dispersion in an amount of from about 10 wt.% to about 70 wt.%, or from about 20 wt.% to about 60 wt.%, or from about 20 wt.% to about 40 wt.%, or about 26 wt.% to about 30 wt.%, based on a total weight of the solid dispersion. In some embodiments, the compound of Formula (I) is present in the solid dispersion in an amount of from about 1 wt.% to about 50 wt.%, or from about 10 wt.% to about 40 wt.%, or from about 20 wt.% to about 35 wt.%, or from about 25 wt.% to about 30 wt.%, based on a total weight of the solid dispersion.

Typically, the solid dispersion (e.g., solid molecular complex) is formulated with a polymer in an amount of not less than about 5 wt.%, based on a total weight of the solid dispersion. In some embodiments, the polymer may be present in the solid dispersion in an amount of from about 5 wt.%, from about 10 wt.%, from about 15 wt.%, from about 20 wt.%, from about 25 wt.%, from about 30 wt.%, from about 35 wt.%, from about 40 wt.%, from about 45 wt.%, from about 50 wt.%, and up to about 95 wt.%, up to about 90 wt.%, up to about 85 wt.%, up to about 80 wt.%, up to about 75 wt.%, up to about 70 wt.%, up to about 65 wt.%, up to about 60 wt.%, up to about 55 wt.%, based on a total weight of the solid dispersion, or any range therebetween. For example, the polymer may be present in the solid dispersion in an amount of from about 20 wt.% to about 95 wt.%, or in an amount of from about 20 wt.% to about 70 wt.%, based on a total weight of the solid dispersion. In some embodiments, a polymer is present in the solid dispersion in an amount of from about 0 wt.% to about 50 wt.%, or from about 5 wt.% to about 60 wt.%, or from about 10 wt.% to about 70 wt.%, based on a total weight of the solid dispersion. In some embodiments, a polymer is present in the solid dispersion in an amount greater than about 10 wt.%, or greater than about 20 wt.%, or greater than about 30 wt.%, or greater than about 40 wt.%, or greater than about 50 wt.%, based on a total weight of the solid dispersion. In some embodiments, the solid dispersion is about 30 wt.% of the compound of Formula (I) and about 70 wt.% of the polymer.

The solid dispersion (e.g., solid molecular complex) may be formulated with a single polymer, or a blend/mixture of different polymers. The polymer may be linear, branched, or crosslinked. The polymer may be a homopolymer or copolymer. In some embodiments, the polymer is a homopolymer. In some embodiments, the polymer is a copolymer. Copolymers may be made formed from two or more, three or more, or four or more monomer species, and may be linear, block, alternating, periodic, statistical/random, stereoblock, gradient, graft, star, or branched copolymers. In some embodiments, the polymer is a synthetic polymer. Examples of the synthetic polymers include, but are not limited to, (1) vinyl polymers such as polyvinyl acetate (PVAc), polyvinyl alcohol (PVA), polyvinylpyrrolidone (PVP) including crosslinked PVP, polyvinyl caprolactam (PVCap), etc.; (2) acrylates such as poly(acrylic) acid, alkyl acrylates (e.g., methyl acrylate, ethyl acrylate, butyl acrylate, etc.); (3) methacrylates, such as EUDRAGIT^{®} type copolymers, poly(methacrylic) acid, alkyl methacrylates (e.g., methylmethacrylate, butylmethacrylate, etc.), amino methacrylate copolymers (e.g., based on N,N-dimethylaminoethyl methacrylate), poly(2-hydroxyethyl methacrylate), etc., for example EUDRAGIT^{®} E PO (EPO; a cationic low viscosity terpolymer based on N,N-dimethylaminoethyl methacrylate-methylmethacrylate-butylmethacrylate; 2:1:1; weight average molecular weight of about 47,000 g/mol; immediate release; soluble below and permeable above pH 5.0; available from Evonik) and EUDRAGIT^{®} L 100-55 (an anionic 1:1 copolymer of methacrylic acid-ethyl acrylate; delayed release; dissolution above pH 5.5; available from Evonik); (4) urethanes; (5) esters; and (6) oxides, such as polyethylene glycol (PEG) and polypropylene glycol (PPG).

In some embodiments, the polymer is a naturally occurring polymer or a derivative of a naturally occurring polymer. Examples of naturally occurring polymers or derivatives of naturally occurring polymers include, but are not limited to, (1) polysaccharides such as chitin, chitosan, dextran, pullulan, gum agar, gum arabic, gum karaya, locust bean gum, gum tragacanth, carrageenans, gum ghatti, guar gum, xanthan gum and scleroglucan; (2) starches such as dextrin and maltodextrin; (3) hydrophilic colloids such as pectin; (4) phosphatides such as lecithin; (5) alginates such as ammonium alginate, sodium, potassium or calcium alginate, propylene glycol alginate, etc.); (6) gelatin; (7) collagen; and (8) cellulose polymers such as ethyl cellulose (EC), methyl cellulose (MC), methylethyl cellulose (MEC), carboxymethyl cellulose (CMC) (e.g., including cross-linked CMC such as sodium croscarmellose), carboxymethyl ethyl cellulose (CMEC), hydroxyethyl cellulose (HEC), hydroxypropyl cellulose (HPC), cellulose acetate (CA), cellulose propionate (CP), cellulose butyrate (CB), cellulose acetate butyrate (CAB), cellulose acetate phthalate (CAP), cellulose acetate trimellitate (CAT), hydroxypropyl methyl cellulose (HPMC), hydroxypropyl methyl cellulose phthalate (HPMCP), hydroxypropyl methyl cellulose acetate succinate (HPMCAS), hydroxypropyl methyl cellulose acetate trimellitate (HPMCAT), and ethylhydroxy ethylcellulose (EHEC).

The polymer used in the solid dispersion may be a blend of any two or more polymers described herein (e.g., HPMC/PVP). In some embodiments, a blended polymer system containing a first polymer (e.g., HPMC) and a second polymer (e.g., PVP) is used. A weight ratio of the first polymer to the second polymer typically ranges from at least 1:99, at least 5:95, at least 10:90, at least 15:85, at least 20:80, at least 25:75, at least 30:70, at least 35:65, at least 40:60, at least 45:55, at least 50:50, and up to 99:1, up to 95:5, up to 90:10, up to 85:15, up to 80:20, up to 75:25, up to 70:30, up to 65:35, up to 60:40, up to 55:45.

The polymer used in the solid dispersion may be a copolymer of any two or more monomer species constituting the polymers described herein (e.g., copolymer of vinyl pyrrolidone and vinyl acetate, copovidone or PVP-VAc), aminomethacrylate copolymers, polyethylene glycol-polyvinyl acetate-polyvinylcaprolactame-based graft copolymer (PVAc-PVCap-PEG), acrylate and/or methacrylate copolymers, etc.). In some embodiments, a copolymer derived from a first monomer (e.g., vinyl pyrrolidone, VP) and a second monomer (e.g., vinyl acetate (VAc)) is used. A weight ratio of the first monomer to the second monomer in the copolymer typically ranges from at least 1:99, at least 5:95, at least 10:90, at least 15:85, at least 20:80, at least 25:75, at least 30:70, at least 35:65, at least 40:60, at least 45:55, at least 50:50, and up to 99:1, up to 95:5, up to 90:10, up to 85:15, up to 80:20, up to 75:25, up to 70:30, up to 65:35, up to 60:40, up to 55:45. Exemplary copolymers may include, but are not limited to, EUDRAGIT^{®} type copolymers, KOLLIDON^{®} VA 64 (a 60:40 copolymer of VP:VAc, 45,000-75,000 g/mol, available from BASF) and VIVAPHARM ^{®} PVP/VA 64 (a 6:4 linear random copolymer of VP:VAc, available from JRS Pharma).

The polymer may be a nonionic polymer or an ionic polymer (cationic, anionic, or contains a mixture of cationic and anionic monomer units). In some embodiments, the solid dispersion (e.g., solid molecular complex) comprises the compound of Formula (I) dispersed in a nonionic polymer. This may be accomplished by various means, including: (A) melting the polymer and dissolving the compound, and optionally any pharmaceutically acceptable excipient(s), in the polymer and then cooling the mixture; or (B) dissolving both the compound of Formula (I) and the polymer in a solvent (e.g., water, an organic solvent, or mixtures thereof), optionally with one or more pharmaceutically acceptable excipients, and removing/evaporating the solvent, for example, through lyophilization, spray drying techniques, in a rotary evaporator, etc. The resulting solid dispersion may comprise the compound of Formula (I) dispersed in the polymer primarily in amorphous form, including in amorphous form.

In some embodiments, the solid dispersion (e.g., solid molecular complex) comprises the compound of Formula (I) dispersed in an ionic polymer. Such solid dispersions may in some instances result in increased stability of the compound of Formula (I). This may be accomplished by various means, including the methods described above for use in forming a dispersion in a nonionic polymer. Because ionic polymers have pH dependent solubility in aqueous systems, the resulting solid dispersion of the compound of Formula (I) and the polymer may be formulated for stability at low pH in the stomach and to release the compound of Formula (I) in the intestine at higher pH (e.g., when an anionic polymer is used), and vice vera, may be formulated for stability at high pH but release the compound of Formula (I) in the stomach at lower pH (e.g., when a cationic polymer is used). In some embodiments, the compound of Formula (I) in such solid dispersions with an ionic polymer may thus be less capable of separating from the polymer and may be immobilized by the polymer in its amorphous form. Examples of such ionic polymers include hydroxypropyl methyl cellulose acetate succinate (HPMCAS), hydroxypropyl methyl cellulose phthalate (HPMCP), and methacrylic acid copolymers. In some embodiments, a polymer is used that is capable of immobilizing the compound of Formula (I) so that it exists primarily in an amorphous form for an extended period of time.

In some embodiments, the polymer is at least one selected from the group consisting of a vinyl polymer, a methacrylate, a polysaccharide, gelatin, and a cellulose polymer, or a blend or a copolymer thereof. In some embodiments, the polymer is at least one selected from the group consisting of gelatin, polyvinyl acetate, polyvinyl alcohol, polyvinylpyrrolidone, pullulan, a cellulose polymer described herein (e.g., HPMC, HPMCAS, HPMCP, etc.), a methacrylate copolymer, including blends and copolymers thereof. In some embodiments, the pharmaceutical composition includes a solid dispersion (e.g., solid molecular complex) comprising the compound of Formula (I) dispersed in a gelatin matrix (as the polymer component), and optionally a pharmaceutically acceptable excipient such as a non-reducing sugar (e.g., mannitol) and/or a pH modifier (e.g., sodium hydroxide). In some embodiments, the pharmaceutical composition includes a solid dispersion (e.g., solid molecular complex) comprising the compound of Formula (I) dispersed within a matrix formed by a cellulose polymer described herein. In some embodiments, the cellulose polymer is HPMC. In some embodiments, the cellulose polymer is HPMCAS. In some embodiments, the pharmaceutical composition includes a solid dispersion (e.g., solid molecular complex) comprising the compound of Formula (I) dispersed within a matrix formed by a cellulose polymer described herein and polyvinylpyrrolidone. In some embodiments, the solid dispersion comprises a blend of HPMC and PVP as the polymer component.

The disclosed solid dispersions formulated with one or more compounds of Formula (I) in amorphous form are advantageous in that the amorphous form of the compound(s) of Formula (I) provides consistent dissolution kinetics for predictable pharmacokinetic behavior and clinical outcomes, yet, the release kinetics from the solid dispersion can be tuned/controlled by selection of an appropriate polymer or polymer blend. For example, the use of hydroxypropyl methyl cellulose acetate succinate (HPMCAS) may provide solid dispersions with extended-release profiles, while the use of other polymers, such as gelatin, may be used to provide solid dispersions with immediate/rapid release profiles. In some embodiments, the solid dispersion (e.g., solid molecular complex) comprises, as polymer component, hydroxypropyl methyl cellulose acetate succinate (HPMCAS), examples of which include, but are not limited to, AQUASOLVE^{™} HPMCAS L, M, or H grade, such as AQUASOLVE^{™} HPMCAS MF (7-11% acetyl, 10-14% succinoyl, 21-25% methoxyl, 5-9% hydroxypropoxy; viscosity of 2.4-3.6 mPa·s as 2% solution in water at 20°C; less than 10 µm mean particle size), available from Ashland; and AQOAT^{®} polymers such as AQOAT^{®} AS-MG (9% acetyl, 11% succinoyl; 1,000 µm mean particle size; dissolution pH≥6.0), available from Shin-Etsu Chemical Co. Ltd.

Further, the release kinetics of the compound of Formula (I) from the solid dispersion can also be tuned/controlled by selection of a suitable molecular weight of polymer. In some embodiments, the solid dispersion comprises a polymer(s) having a low viscosity grade, e.g., a low molecular weight, such as a weight average molecular weight of less than or equal to 100,000 g/mol, less than or equal to 90,000 g/mol, less than or equal to 80,000 g/mol, less than or equal to 70,000 g/mol, less than or equal to 60,000 g/mol, less than or equal to 50,000 g/mol, less than or equal to 40,000 g/mol, less than or equal to 30,000 g/mol, less than or equal to 20,000 g/mol, less than or equal to 15,000 g/mol. Typically, the lower limit of weight average molecular weight for low viscosity grade polymers may be from 1,000 g/mol, from 2,000 g/mol, from 4,000 g/mol, from 6,000 g/mol, from 8,000 g/mol, from 10,000 g/mol, from 12,000 g/mol, from 14,000 g/mol. In some embodiments, the low molecular weight polymer is a low molecular weight hydroxypropyl methyl cellulose (HPMC) polymer, having a molecular weight within the above recite range, alone or as a polymer blend (e.g., blended with PVP). Examples of a low molecular weight hydroxypropyl methyl cellulose (HPMC) polymers which can be used herein include, but are not limited to, AFFINISOL^{™} HPMC HME 15LV (water soluble; amorphous HPMC polymer with a molecular weight of less than 100kDa; bulk density of 0.42 g/cc; D(0.5) of 104.49 µm), METHOCEL^{™} E3 LV (2910 substitution type: 28-30% methoxy substitution, 7-12% hydroxypropyl substitution; viscosity of 4.0-6.0 mPa·s as 2% solution in water at 20°C), METHOCEL^{™} E6 premium LV (70,000-80,000 g/mol, 2910 substitution type: 28-30% methoxy substitution, 7-12% hydroxypropyl substitution; viscosity of 4.8-7.2 mPa·s as 2% solution in water at 20°C), each available from DuPont, and PHARMACOAT^{®} 606 (2910 substitution type: 28-30% methoxy substitution, 7-12% hydroxypropyl substitution; viscosity of 6.0 mPa·s as 2% solution in water at 20°C), available from Shin-Etsu Chemical Co. Ltd. Examples of a PVP polymer which can be used herein include, but are not limited to, KOLLIDON^{®} 12PF (weight average molecular weight of 2,500 g/mol; bulk density of 400-600 g/L; D(0.5) of 35 µm ± 5 µm) and KOLLIDON^{®} 30 (also called PVP K-30, amorphous, water-soluble polyvinylpyrrolidone with a weight average molecular weight of 44,000 - 54,000 g/mol) each available from BASF. The selection of a low molecular weight polymer may provide solid dispersions adapted for immediate release or fast release of the compound of Formula (I). For example, immediate release may refer to dosage forms which release greater than 80 wt.% of the active ingredient within about 1 minute following administration, while the phrase fast release may refer to dosage forms in which the release of 80 wt.% of the active ingredient takes place in a range of about 1 minute to about 5 minutes following administration. While not limited to specific manufacturing techniques, solid dispersions comprising a low molecular weight polymer or polymer blend may be advantageously suited for freeze drying or spray drying preparation methods.

In some embodiments, the solid dispersion comprises a polymer having a high viscosity grade, e.g., a high molecular weight, such as a weight average molecular weight of at least 150,000 g/mol, at least 200,000 g/mol, at least 250,000 g/mol, at least 300,000 g/mol, at least 350,000 g/mol, at least 400,000 g/mol, at least 450,000 g/mol, at least 500,000 g/mol, at least 550,000 g/mol, at least 600,000 g/mol, at least 650,000 g/mol, at least 700,000 g/mol, at least 750,000 g/mol, at least 800,000 g/mol, at least 850,000 g/mol, at least 900,000 g/mol, at least 950,000 g/mol, at least 1,000,000 g/mol. The upper limit of molecular weight for high viscosity grade polymers is not particularly limited, but is typically up to 5,000,000 g/mol, 4,000,000 g/mol, 3,000,000 g/mol, or 2,000,000 g/mol. In some embodiments, the high molecular weight polymer is a high molecular weight hydroxypropyl methyl cellulose (HPMC) polymer, having a molecular weight within the above recite range, alone or as a polymer blend (e.g., blended with PVP). Examples of a high molecular weight hydroxypropyl methyl cellulose (HPMC) polymer which can be used herein include, but are not limited to, AFFINISOL^{™} HPMC HME 100LV or HPMC HME 4M, each available from DuPont; METHOCEL^{™} K100LV (164,000 g/mol), METHOCEL^{™} K4M (400,000 g/mol), METHOCEL^{™} K15M (575,000 g/mol), each available from Colorcon, Inc.; BENECEL^{™} K35M Pharm (2208 substitution type; 675,000 g/mol) and BENECEL^{™} K100LV PH PRM (2208 substitution type; 164,000 g/mol), each available from Ashland. High molecular weight polymers or polymer blends may provide solid dispersions adapted for either fast release or extended-release dosage forms, but are particularly well suited for extended-release applications where it is desirable to release the compound of Formula (I) over extended periods of time, such as for example over 10 minutes, 20 minutes, 30 minutes, 1 hour, 2 hours, 4 hours, 6 hours, 8 hours, 10 hours, 12 hours, or any range in between, or longer. For example, extended-release may refer to dosage forms in which the release of 80 wt.% of the active ingredient takes place in a range of about 5 minutes or longer, 10 minutes or longer, 15 minutes or longer, etc. following administration. While not limited to specific manufacturing techniques, solid dispersions comprising a high molecular weight polymer or polymer blend may be advantageously suited for hot melt extrusion, spray drying, or freeze drying preparation methods.

In some embodiments, the pharmaceutical composition comprises a solid dispersion (e.g., solid molecular complex) that includes the compound of Formula (I) dispersed within a matrix formed by gelatin. Various grades (e.g., various bloom numbers) and sources of gelatin, including gelatin derived from fish or mammalian sources (e.g., bovine), may be used herein. An example of a gelatin includes, but is not limited to, fish gelatin (super fine), available from Ajinomoto, USA. In some embodiments, the solid dispersion (e.g., solid molecular complex) further includes a non-reducing sugar, e.g., mannitol and/or a pH modifier (e.g., sodium hydroxide). The weight ratio of the compound of Formula (I) within the solid dispersion (e.g., solid molecular complex) to the gelatin (polymer) therein is generally within the range set forth herein, for example, from about 1:9 to about 5:5, from about 2:8 to about 4:6, from about 3:7 to about 3.7:6.3.

Various optional pharmaceutically acceptable excipients can be mixed, ground, granulated, or otherwise incorporated into the solid dispersion as described herein to form a material suitable for a particular dosage form or administration route. Potentially beneficial excipients may fall generally into the following classes: other matrix materials, fillers, or diluents; surface active agents; drug complexing agents or solubilizing agents; disintegrants, binders, lubricants, and pH modifiers (e.g., acids, bases, or buffers such as phosphate or citrate salts/buffers). To the extent of any overlap with the polymer component of the solid dispersion (e.g., solid molecular complex), the optional excipients which are considered to be part of the solid dispersion, are considered to be separate and distinct from the polymer in the solid dispersions herein.

Examples of other matrix materials, fillers, or diluents include, but are not limited to, lactose, mannitol, xylitol, microcrystalline cellulose, and calcium diphosphate.

Examples of surface active agents include, but are not limited to, sodium lauryl sulfate and polysorbate 80.

Examples of drug complexing agents or solubilizing agents include, but are not limited to, caffeine, xanthene, gentisic acid, cylodextrins. sodium phosphate, natural amino acids, acacia, cholesterol, diethanolamine (adjunct), glyceryl monostearate, lanolin alcohols, mono- and diglycerides, monoethanolamine (adjunct), lecithin, oleic acid (adjunct), oleyl alcohol (stabilizer), polyoxyethylene 50 stearate, polyoxyl 35 castor oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, diacetate, monostearate, sodium lauryl sulfate, sodium stearate, sorbitan monolaurate, sorbitan monooleate, sorbitan monopalmitate, sorbitan monostearate, stearic acid, trolamine, and emulsifying wax.

Examples of disintegrants include, but are not limited to, sodium starch gycolate and calcium carbonate.

Examples of binders include microcrystalline cellulose and sugars, such as sucrose, mannitol, glucose, dextrose, molasses, and lactose.

Examples of lubricants include, but are not limited to, magnesium stearate and calcium stearate.

Examples of pH modifiers include, but are not limited to, acids such as citric acid, acetic acid, ascorbic acid, lactic acid, aspartic acid, succinic acid, phosphoric acid, and the like; bases such as sodium acetate, potassium acetate, calcium oxide, magnesium oxide, trisodium phosphate, sodium hydroxide, calcium hydroxide, aluminum hydroxide, and the like, and buffers generally comprising mixtures of acids and the salts of said acids.

The pharmaceutical composition may, in addition to the solid dispersion (e.g., solid molecular complex), also optionally comprise therapeutically inert, inorganic or organic excipients (for example, pharmaceutically acceptable excipients) as a separate and distinct component of the dosage form from the solid dispersion. Thus, pharmaceutically acceptable excipients which are dispersed within the solid dispersion may be differentiated from pharmaceutically acceptable excipients which are not dispersed within the solid dispersion but are nonetheless present in the pharmaceutical composition, even though the same chemicals, compounds, or materials may be used for either. For purposes of illustration, a pharmaceutical composition in tablet form may contain lower and upper layers comprising one or more pharmaceutical acceptable excipients, which surround or sandwich a core layer formed from a solid dispersion comprising the compound of Formula (I) and one or more pharmaceutical acceptable excipients dispersed with a polymer. Here, the pharmaceutically acceptable excipient(s) of the lower and upper layers would be considered a distinct component of the dosage form from the pharmaceutically acceptable excipient(s) present and dispersed within the core layer (excipients within the solid dispersion). In another example, a solid dispersion comprising the compound of Formula (I) and a pharmaceutical acceptable excipient dispersed with a polymer may be coated with a pharmaceutically acceptable excipient, with the pharmaceutically acceptable excipient of the coating being a separate component from the pharmaceutically acceptable excipient present within the solid dispersion.

"Pharmaceutically acceptable excipients" may be excipients approved by a regulatory agency of the Federal or a state government or listed in the U.S. Pharmacopeia or other generally recognized pharmacopeia for use in mammals, such as humans. The term "excipient" herein refers to a vehicle, diluent, adjuvant, carrier, or any other auxiliary or supporting ingredient with which the solid dispersion containing the active ingredient (e.g., a compound of Formula (I)) is formulated for administration to a mammal. Such pharmaceutical excipients can be liquids, such as water and oils, including those of petroleum, animal, vegetable or synthetic origin, such as peanut oil, soybean oil, mineral oil, sesame oil and the like. The pharmaceutical excipients can be water, saline, gum acacia, gelatin, starch paste, talc, keratin, colloidal silica, urea, and the like. The pharmaceutical excipients can include one or more gases, e.g., to act as a carrier for administration via inhalation. In addition, auxiliary, stabilizing, thickening, lubricating, taste masking, coloring agents, and other pharmaceutical additives may be included in the disclosed compositions, such as any of those set forth herein. The pharmaceutical composition may thus be formulated with additional agents such as preserving agents, solubilizing agents, stabilizing agents, wetting agents, emulsifying agents, sweetening agents, coloring agents, flavoring agents, salts for varying the osmotic pressure, buffers, coating agents and antioxidants. The pharmaceutical composition may also contain additional therapeutically active ingredients or more than one therapeutically active ingredient/polymer complex (e.g., a solid dispersion, for example a solid molecular complex).

In some embodiments, the pharmaceutical composition includes the solid dispersion (e.g., solid molecular complex) suspended in an aqueous vehicle containing hydroxypropylcellulose (HPC). In some embodiments, the vehicle contains about 2% by weight HPC. In some embodiments, the pharmaceutical composition includes colloidal silicon dioxide (silica). In some embodiments, the addition of colloidal silicon dioxide may further improve the stability of the solid dispersion (e.g., solid molecular complex). In some embodiments, the pharmaceutical composition includes at least about 0.5% by weight colloidal silicon dioxide.

Pharmaceutical compositions are provided herein which comprise about 0.1 to about 1000 mg, about 1 to about 500 mg, about 2 to about 100 mg, about 1 mg, about 2 mg, about 3 mg, about 5 mg, about 10 mg, about 20 mg, about 30 mg, about 40 mg, about 50 mg, about 100 mg, about 500 mg of one or more compounds of Formula (I) as disclosed herein. The quantity of compound of Formula (I) (on active basis) in a unit dose preparation may be varied or adjusted within the above ranges as deemed appropriate using sound medical judgment, according to the particular application, administration route, potency of the active component, etc. The pharmaceutical composition can, if desired, also contain other compatible therapeutic agents. The term "unit dosage form" or "unit dose" will be used herein to refer to compositions formulated with an amount of an active pharmaceutical ingredient (API) in a dose for administration as a single dose to a target individual. The unit dosage form may be adapted, depending on the nature of the active ingredient, the indication, the disease stage and various other factors known per se for once, twice, thrice or any other number of daily, weekly, or monthly administrations.

In some embodiments, the pharmaceutical compositions disclosed herein are adapted for oral and/or intraoral administration such as through the mucosal linings of the oral cavity, e.g., buccal, lingual, and sublingual administration. Intraoral dosage forms allow for pre-gastric absorption of the compounds herein, e.g., when administered intraorally through the mucosal linings of the oral cavity, e.g., buccal, lingual, and sublingual administration, for increased bioavailability and faster onset compared to oral administration through the gastrointestinal tract. In some embodiments, the pharmaceutical compositions disclosed herein are in orodispersible dosage forms (ODFs). ODFs can be prepared by different techniques, such as freeze-drying (lyophilization), molding, spray drying, mass extrusion or compressing. Preferably, the ODFs are prepared by lyophilization. ODFs encompass solid dosage forms that disintegrate or dissolve in the mouth within about 90 seconds, 60 seconds, 30 seconds, 20 seconds, 10 seconds, 5 seconds, 2 seconds or less after being received in the oral cavity. In some embodiments, an orodispersible dosage form disperses in the mouth within 10, 9, 8, 7, 6, 5, 4, 3, 2, or even within 1 second. In some embodiments, the pharmaceutical compositions are in the form of orodispersible dosage forms having a disintegration time according to the United States Phamacopeia (USP) disintegration test <701> of not more than about 30 seconds, not more than about 20, not more than about 10 seconds, not more than about 5 seconds, not more than about 2 seconds. Orodispersible dosage forms having longer disintegration times according to the United States Phamacopeia (USP) disintegration test <701>, such as when adapted for extended-release, for example 2 minutes, 3 minutes, 4 minutes, 5 minutes, 10 minutes, 15 minutes, 20 minutes, 25 minutes, 30 minutes, 45 minutes, 60 minutes, or any range therebetween, or longer, are also contemplated.

In some embodiments, the pharmaceutical compositions are in the form of fast dissolving tablets (FDTs), also called oral disintegrating tablets (or orodispersible tablets) (ODTs) or fast dispersible tablets. Fast dissolving tablets can be prepared by different techniques, such as freeze-drying (lyophilization), molding, spray drying, mass extrusion or compressing. In some embodiments, fast dissolving tablets are prepared by lyophilization. In some embodiments, fast dissolving tablet refers to forms which disintegrate in less than about 90 seconds, in less than about 60 seconds, in less than about 30 seconds, in less than about 20, in less than about 10 seconds in the oral cavity, in less than about 5 seconds, or in less than about 2 seconds after being received in the oral cavity. In some embodiments, fast dissolving tablet refers to forms which dissolve in less than about 90 seconds, in less than about 60 seconds, in less than about 30 seconds, in less than about 20, in less than about 10 seconds in the oral cavity, in less than about 5 seconds, or in less than about 2 seconds after being received in the oral cavity.

In some embodiments, the pharmaceutical compositions are in the form of lyophilized FDTs. In some embodiments, the lyophilized FDTs are created by creating a porous matrix by subliming the water from pre-frozen aqueous formulation of the drug containing matrix-forming agents and other excipients such as lyoprotectants, preservatives, pH modifiers, and flavors. In some embodiments, the FDTs comprise two component frameworks of a lyophilized matrix system that work together to ensure the development of a successful formulation. In some embodiments, the first component is a water-soluble polymer such as gelatin, dextran, alginate, and maltodextrin. This component maintains the shape and provides mechanical strength to the tablets (binder). In some embodiments, the second constituent is matrix-supporting/disintegration-enhancing agents such as sucrose, lactose, mannitol, xylitol, microcrystalline cellulose, and/or calcium diphosphate, which acts by cementing the porous framework, provided by the water-soluble polymer and accelerates the disintegration of the FDT. In some embodiments, the lyophilized FDT includes gelatin and mannitol. In some embodiments, the lyophilized FDTs include gelatin, mannitol, and one or more of a lyoprotectant, a preservative, an antioxidant, a stabilizing agent, a solubilizing agent, a flavoring agent, a pH modifier, etc., with particular mention being made to sodium hydroxide. A non-limiting example of an FDT formulation is Zydis^{®} orally dispersible tablets (available from Catalent). In some embodiments, the FDT formulation (e.g., Zydis^{®} orally dispersible tablets) includes a solid dispersion formed from (a) one or more water-soluble polymers, such as gelatin, (b) one or more matrix materials, fillers, or diluents, such as mannitol, (c) a compound of Formula (I), or a pharmaceutically acceptable salt, polymorph, stereoisomer, or solvate thereof, and optionally (d) a lyoprotectant, a preservative, an antioxidant, a stabilizing agent, a solubilizing agent, a pH modifier, and/or a flavoring agent. In some embodiments, the FDT formulation (e.g., Zydis^{®} orally dispersible tablets) includes gelatin, mannitol, a compound of Formula (I), or a pharmaceutically acceptable salt, polymorph, stereoisomer, tautomer, or solvate thereof, and sodium hydroxide.

In some embodiments, the lyophilized FDTs include a cellulose polymer described herein (e.g., HPMC), either alone or as a polymer blend, e.g., with polyvinylpyrrolidone.

In some embodiments, the pharmaceutical compositions are in the form of lyophilized wafers. In some embodiments, the pharmaceutical compositions are in the form of lyophilized wafers protected for the long-term storage by a specialty packaging excluding moisture, oxygen and light. In some embodiments, the lyophilized wafers are created by creating a porous matrix by subliming the water from pre-frozen aqueous formulation of the drug containing matrix-forming agents and other excipients such as lyoprotectants, preservatives, pH modifiers, and flavors. In some embodiments, the lyophilized wafer includes a thin water-soluble film matrix. In some embodiments, the wafers comprise two component frameworks of a lyophilized matrix system that work together to ensure the development of a successful formulation. In some embodiments, the first component is water-soluble polymers such as gelatin, dextran, alginate, and maltodextrin. This component maintains the shape and provides mechanical strength to the tablets (binder). In some embodiments, the second constituent is matrix-supporting/disintegration-enhancing agents such as sucrose, lactose, mannitol, xylitol, microcrystalline cellulose, and/or calcium diphosphate, which acts by cementing the porous framework, provided by the water-soluble polymer and accelerates the disintegration of the wafer. In some embodiments, the lyophilized wafers include gelatin and mannitol. In some embodiments, the lyophilized wafers include gelatin, mannitol, and one or more of a lyoprotectant, a preservative, an antioxidant, a stabilizing agent, a solubilizing agent, a flavoring agent, a pH modifier, etc., with particular mention being made to sodium hydroxide. In some embodiments, the lyophilized wafer includes a cellulose polymer described herein (e.g., HPMC), either alone or as a polymer blend, e.g., with polyvinylpyrrolidone.

In some embodiments, the wafer can comprise a monolayer, bilayer, or trilayer. In some embodiments, the monolayer wafer contains the solid dispersion of polymer, an active ingredient (e.g., a compound of Formula (I)), and optionally one or more excipients. In some embodiments, the bilayer wafer contains one or more excipients, such as a solubilizing agent, in a first layer and a solid dispersion comprising an active ingredient and polymer in the second layer. This configuration allows the active ingredient to be stored separately from the excipients and can increase the stability of the active ingredient and optionally increase the shelf life of the pharmaceutical composition compared to the case where the excipients and the active ingredient were contained in a single layer. For tri-layer wafers, each of the layers may be different or two of the layers, such as the upper and lower layers, may have substantially the same composition. In some embodiments, the lower and upper layers surround a core layer containing the solid dispersion comprising the active ingredient. In some embodiments, the lower and upper layers may contain one or more excipients, such as a solubilizing agent. In some embodiments, the lower and upper layers have the same composition. Alternatively, the lower and upper layers may contain different excipients or different amounts of the same excipient. The core layer typically contains the solid dispersion formed from polymer(s), active ingredient, optionally with one or more excipients.

Examples of pharmaceutically acceptable lyoprotectants include, but are not limited to, disaccharides such as sucrose and trehalose, anionic polymers such as sulfobutylether-β-cyclodextrin (SBECD) and hyaluronic acid, and hydroxylated cyclodextrins.

Examples of pharmaceutically acceptable preservatives include, but are not limited to, glycerin, methyl and propylparaben, benzoic acid, sodium benzoate and alcohol.

Examples of pharmaceutically acceptable antioxidants, which may act to further enhance stability of the composition, include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of pharmaceutically acceptable stabilizing agents include, but are not limited to, fatty acids, fatty alcohols, alcohols, long chain fatty acid esters, long chain ethers, hydrophilic derivatives of fatty acids, polyvinylpyrrolidones, polyvinyl ethers, polyvinyl alcohols, hydrocarbons, hydrophobic polymers, moisture-absorbing polymers, glycerol, methionine, monothioglycerol, ascorbic acid, citric acid, polysorbate, arginine, cyclodextrins, microcrystalline cellulose, modified celluloses (e.g., carboxymethylcellulose, sodium salt), sorbitol, and cellulose gel.

Examples of pharmaceutically acceptable solubilizing agents (or dissolution aids) include, but are not limited to, citric acid, hydroxypropylcellulose, hydroxypropylmethylcellulose, sodium stearyl fumarate, methacrylic acid copolymer LD, methylcellulose, sodium lauryl sulfate, polyoxyl 40 stearate, purified shellac, sodium dehydroacetate, fumaric acid, DL-malic acid, L-ascorbyl stearate, L-asparagine acid, adipic acid, aminoalkyl methacrylate copolymer E, propylene glycol alginate, casein, casein sodium, a carboxyvinyl polymer, carboxymethylethylcellulose, powdered agar, guar gum, succinic acid, copolyvidone, cellulose acetate phthalate, tartaric acid, dioctylsodium sulfosuccinate, zein, powdered skim milk, sorbitan trioleate, lactic acid, aluminum lactate, ascorbyl palmitate, hydroxyethylmethylcellulose, hydroxypropylmethylcelluloseacetate succinate, polyoxyethylene (105) polyoxypropylene (5) glycol, polyoxyethylene hydrogenated castor oil 60, polyoxyl 35 castor oil, poly(sodium 4-styrenesulfonate), polyvinylacetaldiethylamino acetate, polyvinyl alcohol, maleic acid, methacrylic acid copolymer S, lauromacrogol, sulfuric acid, aluminum sulfate, phosphoric acid, calcium dihydrogen phosphate, sodium dodecylbenzenesulfonate, a vinyl pyrrolidone-vinyl acetate copolymer, sodium lauroyl sarcosinate, acetyl tryptophan, sodium methyl sulfate, sodium ethyl sulfate, sodium butyl sulfate, sodium octyl sulfate, sodium decyl sulfate, sodium tetradecyl sulfate, sodium hexadecyl sulfate, and sodium octadecyl sulfate.

Flavoring agents include natural flavors extracted from plants, such as fruits, and synthetic blends of compounds which produce a pleasant taste sensation or taste masking effect. Examples of flavoring agents include, but are not limited to, aspartame, saccharin (as sodium, potassium or calcium saccharin), cyclamate (as a sodium, potassium or calcium salt), sucralose, acesulfame-K, thaumatin, neohisperidin, dihydrochalcone, ammoniated glycyrrhizin, dextrose, maltodextrin, fructose, levulose, sucrose, glucose, wild orange peel, citric acid, tartaric acid, oil of wintergreen, oil of peppermint, methyl salicylate, oil of spearmint, oil of sassafras, oil of clove, cinnamon, anethole, menthol, thymol, eugenol, eucalyptol, lemon, lime, and lemon-lime.

Cyclodextrins such as α-cyclodextrin, β-cyclodextrin, γ-cyclodextrin, hydroxyethyl β-cyclodextrin, hydroxypropyl γ-cyclodextrin, sulfated β-cyclodextrin, sulfated α-cyclodextrin, sulfobutyl ether β-cyclodextrin, or other solubilized derivatives can also be advantageously used to enhance delivery of compositions described herein.

Disclosed herein are pharmaceutical compositions in modified release dosage forms, which comprise solid dispersions as disclosed herein and one or more release controlling excipients as described herein. Suitable modified release dosage vehicles include, but are not limited to, hydrophilic or hydrophobic matrix devices, water-soluble separating layer coatings, enteric coatings, osmotic devices, multiparticulate devices, and combinations thereof. The pharmaceutical compositions may also comprise non-release controlling excipients.

Further disclosed herein are pharmaceutical compositions in enteric coated dosage forms, which comprise solid dispersions as disclosed herein and one or more release controlling excipients for use in an enteric coated dosage form. The pharmaceutical compositions may also comprise non-release controlling excipients.

Further disclosed herein are pharmaceutical compositions in effervescent dosage forms, which comprise solid dispersions as disclosed herein and one or more release controlling excipients for use in an effervescent dosage form. The pharmaceutical compositions may also comprise non-release controlling excipients.

Additionally disclosed are pharmaceutical compositions in a dosage form that has an instant releasing component and at least one delayed releasing component, and is capable of giving a discontinuous release of the active ingredient (e.g., a compound of Formula (I)) in the form of at least two consecutive pulses separated in time from about 0.1 up to about 24 hours (e.g., about 0.1, 0.5, 1, 2, 4, 6, 8, 10, 12, 14, 16, 18, 10, 22, or 24 hours). The pharmaceutical compositions comprise a solid dispersion as disclosed herein and one or more release controlling and non-release controlling excipients, such as those excipients suitable for a disruptable semipermeable membrane and as swellable substances.

Disclosed herein also are pharmaceutical compositions in a dosage form for oral administration to a subject, which comprise a solid dispersion as disclosed herein and one or more pharmaceutically acceptable excipients, enclosed in an intermediate reactive layer comprising a gastric juice-resistant polymeric layered material partially neutralized with alkali and having cation exchange capacity and a gastric juice-resistant outer layer.

In some embodiments, the pharmaceutical compositions are in the form of immediate-release capsules for oral administration, and may further comprise cellulose, iron oxides, lactose, magnesium stearate, and sodium starch glycolate.

In some embodiments, the pharmaceutical compositions are in the form of delayed-release capsules for oral administration, and may further comprise cellulose, ethylcellulose, gelatin, hypromellose, iron oxide, and titanium dioxide.

In some embodiments, the pharmaceutical compositions are in the form of enteric coated delayed-release tablets for oral administration, and may further comprise carnauba wax, crospovidone, diacetylated monoglycerides, ethylcellulose, hydroxypropyl cellulose, hypromellose phthalate, magnesium stearate, mannitol, sodium hydroxide, sodium stearyl fumarate, talc, titanium dioxide, and yellow ferric oxide.

In some embodiments, the pharmaceutical compositions are in the form of enteric coated delayed-release tablets for oral administration, and may further comprise calcium stearate, crospovidone, hydroxypropyl methylcellulose, iron oxide, mannitol, methacrylic acid copolymer, polysorbate 80, povidone, propylene glycol, sodium carbonate, sodium lauryl sulfate, titanium dioxide, and triethyl citrate.

The pharmaceutical compositions disclosed herein may be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

In the case wherein the patient's condition does not improve, upon the doctor's discretion the compounds may be administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disorder is retained. Patients can, however, require intermittent treatment on a long-term basis upon any recurrence of symptoms.

The compounds as disclosed herein may be administered alone or in combination with one or more other active ingredients. Pharmaceutical compositions comprising a compound disclosed herein may be formulated in various dosage forms for oral, parenteral, and topical administration. The pharmaceutical compositions may also be formulated as a modified release dosage form, including delayed-, extended-, prolonged-, sustained-, pulsatile-, controlled-, accelerated-, fast-, targeted-, programmed-release, and gastric retention dosage forms. These dosage forms can be prepared according to conventional methods and techniques known to those skilled in the art (*see, Remington: The Science and Practice of Pharmacy,* supra; Modified-Release Drug Delivery Technology, Rathbone et al., Eds., Drugs and the Pharmaceutical Science, Marcel Dekker, Inc.: New York, N.Y., 2002; Vol. 126).

Any of the pharmaceutical compositions described herein can comprise a solid dispersion (e.g., a solid molecular complex) comprising a compound of Formula (I), or a pharmaceutically acceptable salt, a polymorph, stereoisomer, a tautomer, or solvate thereof, and a polymer.

### A. Oral Administration

The pharmaceutical compositions disclosed herein may be provided in solid, semisolid, or liquid dosage forms for oral administration. As used herein, oral administration includes gastric (enteral) delivery, for example whereby the medication is taken by mouth and swallowed, as well as intraoral administration such as through the mucosal linings of the oral cavity, e.g., also includes buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the solid dispersions (e.g., solid molecular complex) containing the active ingredient(s) and a polymer(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remains intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, Pa.); and mixtures thereof. Suitable fillers include, but are not limited to, talc, calcium carbonate, microcrystalline cellulose, powdered cellulose, dextrates, kaolin, mannitol, silicic acid, sorbitol, starch, pre-gelatinized starch, and mixtures thereof. The binder or filler may be present from about 50 to about 99% by weight in the pharmaceutical compositions disclosed herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Veegum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as corn starch, potato starch, tapioca starch, and pre-gelatinized starch; clays; aligns; and mixtures thereof. The amount of disintegrant in the pharmaceutical compositions disclosed herein varies upon the type of formulation, and is readily discernible to those of ordinary skill in the art. The pharmaceutical compositions disclosed herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, corn oil, and soybean oil; zinc stearate; ethyl oleate; ethyl laureate; agar; starch; lycopodium; silica or silica gels, such as AEROSIL^{®} 200 (W.R. Grace Co., Baltimore, Md.) and CAB-O-SIL^{®} (Cabot Co. of Boston, Mass.); and mixtures thereof. The pharmaceutical compositions disclosed herein may contain about 0.1 to about 5% by weight of a lubricant.

Suitable glidants include colloidal silicon dioxide, CAB-O-SIL^{®} (Cabot Co. of Boston, Mass.), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many excipients may serve several functions, even within the same formulation.

The pharmaceutical compositions disclosed herein may be disclosed as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the solid dispersions comprising the active ingredient, e.g., in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The dosage form may be an immediate release (IR) or fast release dosage form, examples of which include, but are not limited to, an immediate release (IR) tablet or an immediate release (IR) capsule. In addition to the solid dispersion of the present disclosure, which contains active ingredient (e.g., a compound of Formula (I)) dispersed in a polymer, dosage forms adapted for immediate or fast release may also include one or more pharmaceutically acceptable excipients which readily disperse, dissolve, or otherwise breakdown in the gastric environment so as not to delay or prolong dissolution/absorption of the active. Examples of pharmaceutically acceptable excipients for immediate or fast release dosage forms include, but are not limited to, one or more binders/granulators, matrix materials, fillers, diluents, disintegrants, dispersing agents, solubilizing agents, lubricants, and/or performance modifiers. In some embodiments, the immediate or fast release (IR) dosage form is an immediate release (IR) or fast release tablet comprising one or more of the following pharmaceutically acceptable excipients: microcrystalline cellulose, sodium carboxymethylcellulose, magnesium stearate, mannitol, crospovidone, and sodium stearyl fumarate. In some embodiments, the immediate release (IR) or fast release dosage form comprises microcrystalline cellulose, sodium carboxymethylcellulose, and magnesium stearate as pharmaceutically acceptable excipients. In some embodiments, the immediate release (IR) or fast release dosage form comprises mannitol, crospovidone, and sodium stearyl fumarate as pharmaceutically acceptable excipients.

The pharmaceutical compositions disclosed herein may be disclosed as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the solid dispersion containing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms disclosed herein may be encapsulated in a capsule. Suitable liquid and semisolid dosage forms include solutions and suspensions in propylene carbonate, vegetable oils, or triglycerides. The capsules may also be coated as known by those of skill in the art in order to modify or sustain dissolution of the active ingredient.

The pharmaceutical compositions disclosed herein may be disclosed in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde (the term "lower" means an alkyl having between 1 and 6 carbon atoms), e.g., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) disclosed herein, and a dialkylated mono- or poly-alkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates. In some embodiments, examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Cyclodextrins such as α-cyclodextrin, β-cyclodextrin, γ-cyclodextrin, hydroxyethyl β-cyclodextrin, hydroxypropyl γ-cyclodextrin, sulfated β-cyclodextrin, sulfated α-cyclodextrin, sulfobutyl ether β-cyclodextrin, or other solubilized derivatives can also be advantageously used to enhance delivery of compositions described herein.

The pharmaceutical compositions disclosed herein for oral administration may be also disclosed in the forms of liposomes, micelles, microspheres, or nanosystems.

The pharmaceutical compositions disclosed herein may be disclosed as non-effervescent or effervescent, granules and powders, to be reconstituted into a liquid dosage form. Pharmaceutically acceptable excipients used in the non-effervescent granules or powders may include diluents, sweeteners, and wetting agents. Pharmaceutically acceptable excipients used in the effervescent granules or powders may include organic acids and a source of carbon dioxide.

Coloring and flavoring agents can be used in all of the above dosage forms.

The pharmaceutical compositions disclosed herein may be co-formulated with other active ingredients which do not impair the desired therapeutic action, or with substances that supplement the desired action, such as drotrecogin-α, and hydrocortisone.

B. Parenteral Administration

The pharmaceutical compositions disclosed herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions disclosed herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see, Remington: The Science and Practice of Pharmacy,* supra).

The pharmaceutical compositions intended for parenteral administration may include one or more pharmaceutically acceptable excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH modifiers, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (e.g., polyethylene glycol 300 and polyethylene glycol 400), propylene glycol, glycerin, N-methyl-2-pyrrolidone, dimethylacetamide, and dimethylsulfoxide.

Suitable antimicrobial agents or preservatives include, but are not limited to, phenols, cresols, mercurials, benzyl alcohol, chlorobutanol, methyl and propyl p-hydroxybenzates, thimerosal, benzalkonium chloride, benzethonium chloride, methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH modifiers include, but are not limited to, sodium hydroxide, hydrochloric acid, citric acid, and lactic acid. Suitable complexing agents include, but are not limited to, cyclodextrins, including ca-cyclodextrin, β-cyclodextrin, hydroxypropyl-3-cyclodextrin, sulfobutylether-β-cyclodextrin, and sulfobutylether 7-O-cyclodextrin (CAPTISOL^{®}, CyDex, Lenexa, Kans.).

The pharmaceutical compositions disclosed herein may be formulated for single or multiple dosage administration. The single dosage formulations are packaged in an ampule, a vial, or a syringe. The multiple dosage parenteral formulations must contain an antimicrobial agent at bacteriostatic or fungistatic concentrations. All parenteral formulations must be sterile, as known and practiced in the art.

In some embodiments, the pharmaceutical compositions are disclosed as ready-to-use sterile solutions. In some embodiments, the pharmaceutical compositions are disclosed as sterile dry soluble products, including lyophilized powders and hypodermic tablets, to be reconstituted with a vehicle prior to use. In some embodiments, the pharmaceutical compositions are disclosed as ready-to-use sterile suspensions. In some embodiments, the pharmaceutical compositions are disclosed as sterile dry insoluble products to be reconstituted with a vehicle prior to use. In some embodiments, the pharmaceutical compositions are disclosed as ready-to-use sterile emulsions.

The pharmaceutical compositions may be formulated as a suspension, solid, semi-solid, or thixotropic liquid, for administration as an implanted depot. In some embodiments, the pharmaceutical compositions disclosed herein are dispersed in a solid inner matrix, which is surrounded by an outer polymeric membrane that is insoluble in body fluids but allows the active ingredient in the pharmaceutical compositions to diffuse through.

Suitable inner matrixes include polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### C. Topical Administration

The pharmaceutical compositions disclosed herein may be administered topically to the skin, orifices, or mucosa. Topical administration, as described herein, includes (intra)dermal, conjuctival, intracorneal, intraocular, ophthalmic, auricular, transdermal, nasal, vaginal, uretheral, respiratory, and rectal administration.

The pharmaceutical compositions disclosed herein may be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions disclosed herein may also comprise liposomes, micelles, microspheres, nanosystems, and mixtures thereof.

Pharmaceutically acceptable excipients suitable for use in the topical formulations disclosed herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The ointments, pastes, creams and gels may contain, in addition to an active ingredient(s), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an active ingredient(s), excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal delivery devices (e.g., patches) have the added advantage of providing controlled delivery of active ingredient(s) to the body. That is, solid dispersions comprising the active ingredient(s) of the present disclosure (e.g., a compound of Formula (I)) can be administered *via* a transdermal patch at a steady state concentration, whereby the active ingredient(s) is gradually administered over time, thus avoiding drug spiking and adverse events/toxicity associated therewith.

Transdermal patch dosage forms herein may be formulated with various amounts of the active ingredient(s), depending on the disease/condition being treated, the active ingredient(s) employed, the permeation and size of the transdermal delivery device, the release time period, etc. For example, a unit dose preparation may be varied or adjusted e.g., from 5 mg, 10 mg, 15 mg, 20 mg, 25 mg, 30 mg, 35 mg, 40 mg, 45 mg, 50 mg, to 100 mg, 95 mg, 90 mg, 85 mg, 80 mg, 75 mg, 70 mg, 65 mg, 60 mg, 55 mg of the compound of Formula (I), or otherwise as deemed appropriate using sound medical judgment, according to the particular application and the potency of the active ingredient.

Transdermal patches formulated with the solid dispersion may be suitable for microdosing to achieve durable therapeutic benefits, with decreased toxicity. In some embodiments, the compound of Formula (I) may be administered *via* a transdermal patch at serotonergic, but sub-psychoactive concentrations, for example, over an extended period such as over a 8, 24, 48, 72, 84, 96, or 168 hour time period.

In addition to the solid dispersion of the present disclosure, and any optional pharmaceutically acceptable excipient(s), the transdermal patch may also include one or more of a pressure sensitive adhesive layer, a backing, and a release liner, as is known to those of ordinary skill in the art.

In some embodiments, the solid dispersion is dissolved/dispersed directly into a polymer matrix forming the pressure sensitive adhesive layer. In some embodiments, the compound of Formula (I) (or a pharmaceutically acceptable salt, a polymorph, stereoisomer, a tautomer, or solvate thereof) of the present disclosure may be dissolved/dispersed directly into a polymer matrix forming the pressure sensitive adhesive layer, that is, where the pressure sensitive adhesive layer acts as the polymer component of the solid dispersion. Such transdermal patches are called drug-in-adhesive (DIA) patches. Preferred DIA patch forms are those in which the active ingredient(s) is distributed uniformly throughout a pressure sensitive adhesive polymer matrix. In some embodiments, the active ingredient(s) may be provided in a solid dispersion in a layer which is separate from the pressure sensitive adhesive layer. In any case, the transdermal patch dosage forms may optionally be formulated with suitable excipient(s) such as carriers, permeation agents/absorption enhancers, humectants, etc. to increase the flux across the skin.

Examples of carrier agents may include, but are not limited to, C₈-C₂₂ fatty acids, such as oleic acid, undecanoic acid, valeric acid, heptanoic acid, pelargonic acid, capric acid, lauric acid, and eicosapentaenoic acid; C₈-C₂₂ fatty alcohols such as octanol, nonanol, oleyl alcohol, decyl alcohol and lauryl alcohol; lower alkyl esters of C₈-C₂₂ fatty acids such as ethyl oleate, isopropyl myristate, butyl stearate, and methyl laurate; di(lower)alkyl esters of C₆-C₂₂ diacids such as diisopropyl adipate; monoglycerides of C₈-C₂₂ fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; glycerol; ethyl acetate; acetoacetic ester; N-alkylpyrrolidone; cyclodextrins, such as α-cyclodextrin, β-cyclodextrin, γ-cyclodextrin, or derivatives such as 2-hydroxypropyl-β-cyclodextrin; and terpenes/terpenoids, such as limonene, linalool, myrcene, pinene such as α-pinene, caryophyllene, citral, eucolyptol, and the like; including mixtures thereof.

Examples of permeation agents/absorption enhancers include, but are not limited to, sulfoxides, such as dodecylmethylsulfoxide, octyl methyl sulfoxide, nonyl methyl sulfoxide, decyl methyl sulfoxide, undecyl methyl sulfoxide, 2-hydroxydecyl methyl sulfoxide, 2-hydroxy-undecyl methyl sulfoxide, 2-hydroxydodecyl methyl sulfoxide, and the like; surfactant-lecithin organogel (PLO), such as those formed from an aqueous phase with one or more of poloxamers, CARBOPOL and PEMULEN, a lipid phase formed from one or more of isopropyl palmitate and PPG-2 myristyl ether propionate, and lecithin; fatty acids, esters, and alcohols, such as oleyloleate and oleyl alcohol; keto acids such as levulinic acid; glycols and glycol ethers, such as diethylene glycol monoethyl ether; including mixtures thereof.

Examples of humectants/crystallization inhibitors include, but are not limited to, polyvinylpyrrolidone-co-vinyl acetate, polymethacrylate, and mixtures thereof.

The pressure sensitive adhesive layer may be formed from polymers including, but not limited to, acrylics (polyacrylates including alkyl acrylics), polyvinyl acetates, natural and synthetic rubbers (e.g., polyisobutylene), ethylenevinylacetate copolymers, polysiloxanes, polyurethanes, plasticized polyether block amide copolymers, plasticized styrene-butadiene rubber block copolymers, and mixtures thereof. The pressure-sensitive adhesive layer used in the transdermal patch of the present disclosure may be formed from an acrylic polymer pressure-sensitive adhesive, preferably an acrylic copolymer pressure sensitive adhesive. The acrylic copolymer pressure sensitive adhesive may be obtained by copolymerization of one or more alkyl (meth)acrylates (e.g., 2-ethylhexyl acrylate); aryl (meth)acrylates; arylalkyl (meth)acrylate; and (meth)acrylates with functional groups such as hydroxyalkyl (meth)acrylates (e.g., hydroxyethyl acrylate, 2-hydroxypropyl acrylate, 3-hydroxypropyl acrylate, 4-hydroxybutyl acrylate, 2-hydroxyethyl methacrylate, 2-hydroxypropyl methacrylate, 3-hydroxypropyl methacrylate, and 4-hydroxybutyl methacrylate), carboxylic acid containing (meth)acrylates (e.g., acrylic acid), and alkoxy (meth)acrylates (e.g., methoxyethyl acrylate); optionally with one or more copolymerizable monomers (e.g., vinyl pyrrolidone, vinyl acetate, etc.). Specific examples of acrylic pressure-sensitive adhesives may include, but are not limited to, DURO-TAK products (Henkel) such as DURO-TAK 87-900A, DURO-TAK 87-9301, DURO-TAK 87-4098, DURO-TAK 87-2074, DURO-TAK 87-235A, DURO-TAK 87-2510, DURO-TAK 87-2287, DURO-TAK 87-4287, DURO-TAK 87-2516, DURO-TAK 387-2052, and DURO-TAK 87-2677.

The backing used in the transdermal patch of the present disclosure may include flexible backings such as films, nonwoven fabrics, Japanese papers, cotton fabrics, knitted fabrics, woven fabrics, and laminated composite bodies of a nonwoven fabric and a film. Such a backing is preferably composed of a soft material that can be in close contact with a skin and can follow skin movement and of a material that can suppress skin rash and other discomforts following prolonged use of the patch. Examples of the backing materials include, but are not limited to, polyethylene, polypropylene, polyethylene terephthalate, polybutylene terephthalate, polyethylene naphthalate, polystyrene, nylon, cotton, acetate rayon, rayon, a rayon/polyethylene terephthalate composite body, polyacrylonitrile, polyvinyl alcohol, acrylic polyurethane, ester polyurethane, ether polyurethane, a styrene-isoprene-styrene copolymer, a styrene-butadiene-styrene copolymer, a styrene-ethylene-propylene-styrene copolymer, styrene-butadiene rubber, an ethylene-vinyl acetate copolymer, or cellophane, for example. Preferred backings do not adsorb or release the active ingredient(s). In order to suppress the adsorption and release of the active ingredient(s), to improve transdermal absorbability of the active ingredient(s), and to suppress skin rash and other discomforts, the backing preferably includes one or more layers composed of the material above and has a water vapor permeability. Specific examples of backings may include, but are not limited to, 3M COTRAN products such as 3M COTRAN ethylene vinyl acetate membrane film 9702, 3M COTRAN ethylene vinyl acetate membrane film 9716, 3M COTRAN polyethylene membrane film 9720, 3M COTRAN ethylene vinyl acetate membrane film 9728, and the like.

The release liner used in the transdermal patch of the present disclosure may include, but is not limited to, a polyester film having one side or both sides treated with a release coating, a polyethylene laminated high-quality paper treated with a release coating, and a glassine paper treated with a release coating. The release coating may be a fluoropolymer, a silicone, a fluorosilicone, or any other release coating known to those of ordinary skill in the art. The release liner may have an uneven surface in order to easily take out the transdermal patch from a package. Examples of release liners may include, but are not limited to SCOTCHPAK products from 3M such as 3M SCOTCHPAK 9744, 3M SCOTCHPAK 9755, 3M SCOTCHPAK 9709, and 3M SCOTCHPAK 1022.

Other layers such as abuse deterrent layers formulated with one or more irritants (e.g., sodium lauryl sulfate, poloxamer, sorbitan monoesters, glyceryl monooleates, spices, etc.), may also be employed.

Methods disclosed herein using a transdermal patch dosage form provide for systemic delivery of small doses of active ingredient(s), preferably over extended periods of time such as up to 168 hour time periods, for example from 2 to 96 hours, or 4 to 72 hours, or 8 to 24 hours, or 10 to 18 hours, or 12 to 14 hours. In particular, the compound(s) of Formula (I) of the present disclosure can be delivered in small, steady, and consistent doses such that deleterious or undesirable side-effects can be avoided. In some embodiments, the compounds of Formula (I) of the present disclosure are administered transdermally at serotonergic, but sub-psychoactive concentrations.

Therefore, provided herein are methods of treating a disease or disorder associated with a serotonin 5-HT₂ receptor, such as a central nervous system (CNS) disorder, a psychological disorder, or an autonomic nervous system (ANS), comprising administering the solid dispersion od the present disclosure *via* a transdermal patch. Here, the compound of Formula (I) is capable of diffusing from the polymer matrix of the transdermal patch (e.g., from the pressure sensitive adhesive layer or from a separate polymer layer) across the skin of the subject and into the bloodstream of the subject.

An exemplary drug-in-adhesive (DIA) patch formulation may comprise 5 to 30 wt.% of a compound of Formula (I) (psilocybin, psilocybin-*d*₁₀ etc.), 5 to 35 wt.% polymer/crystallization inhibitor (e.g., HPMC, HPMCAS, polyvinylpyrrolidone-co-vinyl acetate, polymethacrylate, etc.), 30 to 70 wt.% pressure sensitive adhesive (e.g., DURO-TAK 387-2052, DURO-TAK 87-2677, and DURO-TAK 87-4098), 1 to 10 wt.% permeation agents/absorption enhancers (e.g., oleyloleate, oleyl alcohol, levulinic acid, diethylene glycol monoethyl ether, etc.), each based on a total weight of the DIA patch formulation, though it should be understood that many variations are possible in light of the teachings herein.

Automatic injection devices offer a method for delivery of the pharmaceutical compositions disclosed herein to patients. The compositions disclosed herein may be administered to a patient using automatic injection devices through a number of known devices, a non-limiting list of which includes transdermal, subcutaneous, and intramuscular delivery.

In some transdermal, subcutaneous, or intramuscular applications, a composition disclosed herein is absorbed through the skin. Passive transdermal patch devices often include an absorbent layer or membrane that is placed on the outer layer of the skin. The membrane typically contains a dose of a substance that is allowed to be absorbed through the skin to deliver the composition to the patient. Typically, only substances that are readily absorbed through the outer layer of the skin may be delivered with such transdermal patch devices.

Other automatic injection devices disclosed herein are configured to provide for increased skin permeability to improve delivery of the disclosed compositions. Non-limiting examples of structures used to increase permeability to improve transfer of a composition into the skin, across the skin, or intramuscularly include the use of one or more microneedles, which in some embodiments may be coated with a composition disclosed herein. Alternatively, hollow microneedles may be used to provide a fluid channel for delivery of the disclosed compositions below the outer layer of the skin. Other devices disclosed herein include transdermal delivery by iontophoresis, sonophoresis, reverse iontophoresis, or combinations thereof, and other technologies known in the art to increase skin permeability to facilitate drug delivery.

The pharmaceutical compositions may also be administered topically by electroporation, iontophoresis, phonophoresis, sonophoresis and microneedle or needle-free injection, such as POWDERJECT^{™} (Chiron Corp., Emeryville, Calif.), and BIOJECT^{™} (Bioject Medical Technologies Inc., Tualatin, Oreg.).

The pharmaceutical compositions disclosed herein may be disclosed in the forms of ointments, creams, and gels. Suitable ointment vehicles include oleaginous or hydrocarbon vehicles, including such as lard, benzoinated lard, olive oil, cottonseed oil, and other oils, white petrolatum; emulsifiable or absorption vehicles, such as hydrophilic petrolatum, hydroxystearin sulfate, and anhydrous lanolin; water-removable vehicles, such as hydrophilic ointment; water-soluble ointment vehicles, including polyethylene glycols of varying molecular weight; emulsion vehicles, either water-in-oil (W/O) emulsions or oil-in-water (O/W) emulsions, including cetyl alcohol, glyceryl monostearate, lanolin, and stearic acid (*see,* Remington: The Science and Practice of Pharmacy, supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, Carbopol^{®}; hydrophilic polymers, such as polyethylene oxides, polyoxyethylene-polyoxypropylene copolymers, and polyvinyl alcohol; cellulosic polymers, such as hydroxypropyl cellulose, hydroxyethyl cellulose, hydroxypropyl methylcellulose, hydroxypropyl methyl cellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions disclosed herein may be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: The Science and Practice of Pharmacy, supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable excipients utilized in rectal and vaginal suppositories include bases or vehicles, such as stiffening agents, which produce a melting point in the proximity of body temperature, when formulated with the pharmaceutical compositions disclosed herein; and antioxidants as described herein, including bisulfite and sodium metabisulfite. Suitable vehicles include, but are not limited to, cocoa butter *(theobroma* oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to about 3 g.

The pharmaceutical compositions disclosed herein may be administered ophthalmically in the forms of solutions, suspensions, ointments, emulsions, gel-forming solutions, powders for solutions, gels, ocular inserts, and implants.

### D. Intranasal and Inhalation Administration

The pharmaceutical compositions disclosed herein may be administered intranasally or by inhalation to the respiratory tract. The pharmaceutical compositions may be disclosed in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be disclosed as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient disclosed herein, a propellant as solvent; and/or a surfactant, such as sorbitan trioleate, oleic acid, or an oligolactic acid.

Administration may also be carried out with a dry powder inhaler (DPI). In such DPI devices, the solid dispersion itself can form the powder or the powder can be formed from pharmaceutical compositions of the solid dispersion and additional pharmaceutically acceptable excipients. Pharmaceutically acceptable excipients used to formulate such carrier powders are known in the art *(see,* e.g., H. Hamishehkar, et al., "The Role of Carrier in Dry Powder Inhaler", Recent Advances in Novel Drug Carrier Systems, pp.39-66, (2012).). In either case, the active ingredient (e.g., a compound of Formula (I)) is releasably bound within the solid dispersion such that upon inhalation, the moisture in the lungs releases the active ingredient from the solid dispersion to make the active ingredient available for systemic absorption. In some embodiments, the active ingredient is delivered by use of a dry powder inhaler (DPI).

DPI is generally formulated with powders or powder mixtures with coarse carrier particles and micronized drug particles with aerodynamic particle diameters of 1-5 µm (*see* Iida et al., "Preparation of dry powder inhalation by surface treatment of lactose carrier particles." Chem Pharm Bull, 2003, 51(1): 1-5). Carrier particles are often used to improve drug particle flowability, thus improving dosing accuracy and minimizing the dose variability observed with drug formulations alone while making them easier to handle during manufacturing operations. Carrier particles should have several characteristics such as physico-chemical stability, biocompatibility and biodegradability, compatible with the drug substance and must be inert, available and economical. The choice of carrier particle (both content and size) is well within the purview of one of ordinary skill in the art. The most common carrier particles are made of lactose or other sugars, with α-lactose monohydrate being the most common lactose grade used in the inhalation field for such particulate carriers.

The pharmaceutical compositions disclosed herein may be micronized to a size suitable for delivery by inhalation, such as about 50 micrometers or less, or about 10 micrometers or less. Particles of such sizes may be prepared using a comminuting method known to those skilled in the art, such as spiral jet milling, fluid bed jet milling, supercritical fluid processing to form nanoparticles, high pressure homogenization, or spray drying. In some embodiments, dry powders for use in DPI administration are produced using spray drying techniques.

Capsules, blisters, and cartridges for use in an inhaler or insufflator may be formulated to contain a powder mix of the pharmaceutical compositions disclosed herein; a suitable powder base, such as lactose or starch; and a performance modifier, such as l-leucine, mannitol, or magnesium stearate. The lactose may be anhydrous or in the form of the monohydrate. Other suitable excipients include, but are not limited to, dextran, glucose, maltose, sorbitol, xylitol, fructose, sucrose, and trehalose. The pharmaceutical compositions disclosed herein for inhaled/intranasal administration may further comprise a suitable flavor, such as menthol and levomenthol, or sweeteners, such as saccharin or saccharin sodium.

The pharmaceutical compositions disclosed herein for topical administration may be formulated to be immediate release or modified release, including delayed-, sustained-, pulsed-, controlled-, targeted, and programmed release.

### E. Modified Release

The pharmaceutical compositions disclosed herein may be formulated as a modified release dosage form. As used herein, the term "modified release" refers to a dosage form in which the rate or place of release of the active ingredient(s) is different from that of an immediate dosage form when administered by the same route. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphorism of the active ingredient(s).

### 1. Matrix Controlled Release Devices

The pharmaceutical compositions disclosed herein in a modified release dosage form may be fabricated using a matrix controlled release device known to those skilled in the art (*see,* Takada et al in "Encyclopedia of Controlled Drug Delivery," Vol. 2, Mathiowitz ed., Wiley, 1999).

In some embodiments, the pharmaceutical compositions disclosed herein in a modified release dosage form is formulated using an erodible matrix device, which is water-swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinylpyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Evonik); poly(2-hydroxyethylmethacrylate); polylactides; copolymers of L-glutamic acid and ethyl-L-glutamate; degradable lactic acid-glycolic acid copolymers; poly-D-(-)-3-hydroxybutyric acid; and other acrylic acid derivatives, such as homopolymers and copolymers of butylmethacrylate, methylmethacrylate, ethylmethacrylate, ethylacrylate, (2-dimethylaminoethyl)methacrylate, and (trimethylaminoethyl)methacrylate chloride.

In further embodiments, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device include, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinylacetate copolymers, ethylene/propylene copolymers, ethylene/ethyl acrylate copolymers, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethyleneterephthalate, natural rubber, silicone rubbers, polydimethylsiloxanes, silicone carbonate copolymers, and; hydrophilic polymers, such as ethyl cellulose, cellulose acetate, crospovidone, and cross-linked partially hydrolyzed polyvinyl acetate, and fatty compounds, such as carnauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingredient(s) versus the polymer, and other excipients in the pharmaceutical compositions.

The pharmaceutical compositions disclosed herein in a modified release dosage form may be prepared by methods known to those skilled in the art, including direct compression, dry or wet granulation followed by compression, melt-granulation followed by compression.

### 2. Osmotic Controlled Release Devices

The pharmaceutical compositions disclosed herein in a modified release dosage form may be fabricated using an osmotic controlled release device, including a one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents water-swellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

Another class of osmotic agents are osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol, organic acids, such as ascorbic acid, benzoic acid, fumaric acid, citric acid, maleic acid, sebacic acid, sorbic acid, adipic acid, edetic acid, glutamic acid, p-toluenesulfonic acid, succinic acid, and tartaric acid; urea; and mixtures thereof.

Osmotic agents of different dissolution rates may be employed to influence how rapidly the active ingredient(s) is initially delivered from the dosage form. For example, amorphous sugars, such as Mannogeme EZ (SPI Pharma, Lewes, Del.) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core may also include a wide variety of other excipients as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxylated ethylene-vinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

A semipermeable membrane may also be a hydrophobic microporous membrane, wherein the pores are substantially filled with a gas and are not wetted by the aqueous medium but are permeable to water vapor, as disclosed in U.S. Pat. No. 5,798,119. Such hydrophobic but water-vapor permeable membranes are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes.

The delivery port(s) on the semipermeable membrane may be formed post-coating by mechanical or laser drilling. Delivery port(s) may also be formed in situ by erosion of a plug of water-soluble material or by rupture of a thinner portion of the membrane over an indentation in the core. In addition, delivery ports may be formed during coating process, as in the case of asymmetric membrane coatings of the type disclosed in U.S. Pat. Nos. 5,612,059 and 5,698,220.

The total amount of the active ingredient(s) released and the release rate can be substantially modulated via the thickness and porosity of the semipermeable membrane, the composition of the core, and the number, size, and position of the delivery ports.

The pharmaceutical compositions in an osmotic controlled-release dosage form may further comprise additional conventional excipients as described herein to promote performance or processing of the formulation.

The osmotic controlled-release dosage forms can be prepared according to conventional methods and techniques known to those skilled in the art (*see, Remington: The Science and Practice of Pharmacy,* supra; Santus and Baker, J. Controlled Release 1995, 35, 1-21; Verma et al., Drug Development and Industrial Pharmacy 2000, 26, 695-708; Verma et al., J. Controlled Release 2002, 79, 7-27).

In some embodiments, the pharmaceutical compositions disclosed herein are formulated as AMT controlled-release dosage form, which comprises an asymmetric osmotic membrane that coats a core comprising the active ingredient(s) and other pharmaceutically acceptable excipients. The AMT controlled-release dosage forms can be prepared according to conventional methods and techniques known to those skilled in the art, including direct compression, dry granulation, wet granulation, and a dip-coating method.

In some embodiments, the pharmaceutical compositions disclosed herein are formulated as ECS controlled-release dosage form, which comprises an osmotic membrane that coats a core comprising the active ingredient(s), a polymer (e.g., hydroxylethyl cellulose), and optionally other pharmaceutically acceptable excipients.

### 3. Multiparticulate Controlled Release Devices

The pharmaceutical compositions disclosed herein in a modified release dosage form may be fabricated a multiparticulate controlled release device, which comprises a multiplicity of particles, granules, or pellets, ranging from about 10 µm to about 3 mm, about 50 µm to about 2.5 mm, or from about 100 µm to about 1 mm in diameter. Such multiparticulates may be made by the processes know to those skilled in the art, including wet- and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients as described herein may be blended with the pharmaceutical compositions to aid in processing and forming the multiparticulates. The resulting particles may themselves constitute the multiparticulate device or may be coated by various film-forming materials, such as enteric polymers, water-swellable, and water-soluble polymers. The multiparticulates can be further processed as a capsule or a tablet.

### 4. Targeted Delivery

The pharmaceutical compositions disclosed herein may also be formulated to be targeted to a particular tissue, receptor, or other area of the body of the subject to be treated, including liposome-, resealed erythrocyte-, and antibody-based delivery systems.

### Method of Making Solid Dispersions (e.g.. Solid Molecular Complexes)

Also provided are methods of making solid dispersions (e.g., solid molecular complexes) as disclosed herein and pharmaceutical compositions comprising the solid dispersions (e.g., solid molecular complexes). In some embodiments, the compound of Formula (I) may be microprecipitated with a polymer as disclosed herein. Methods of making the solid dispersion, or pharmaceutical composition, may be accomplished by any means known in the art, for example: spray drying; freeze-drying (lyophilization); solvent-controlled precipitation; pH-controlled precipitation; hot melt extrusion; and supercritical fluid technology. Each of these methods is described in more detail below.

After forming the solid dispersion using the various methods, it can be recovered by procedures known to those skilled in the art, for example by filtration, conveying to a collector, centrifugation, washing, etc. The recovered solid dispersion can be subjected to drying or additional drying steps (e.g., in air, an oven, or a vacuum) and the resulting solid can be optionally milled, pulverized or micronized to a fine powder by means known in the art. The powder form of the solid dispersion can then be used as is (can be used per se as the pharmaceutical composition) or combined with a pharmaceutically acceptable excipient to form a pharmaceutical composition.

### 1. Spray Drying

Solid dispersions can be obtained by spray drying a liquid mixture comprising an active ingredient (e.g., a compound of Formula (I) or a pharmaceutically acceptable salt, a polymorph, stereoisomer, a tautomer, or solvate thereof), a suitable polymer(s), an optional pharmaceutically acceptable excipient, and an appropriate solvent system. The solvent system may be a single solvent or mixture of solvents, including organic solvents having a low boiling point (e.g., ethanol, methanol, acetone, dichloromethane (DCM), methyl acetate, ethyl acetate, isopropyl acetate, 2-butanone, methanol, 1-propanol, propan-2-ol, acetonitrile, chloroform, etc.), solvents including organic solvents with a medium/high boiling point (e.g., water, acetic acid, 3-pentanone, 4-methyl-2-pentanone, dimethylsulfoxide, dimethylformamide, etc.), or mixtures thereof. In some embodiments, the solvent system is a mixture of organic solvents, e.g., dichloromethane and methanol. While not limited thereto, a % solid loading of the liquid mixture typically ranges from about 0.1 wt.%, from about 0.5 wt.%, from about 1 wt.%, from about 1.5 wt.%, from about 2 wt.%, and up to about 5 wt.%, up to about 4.5 wt.%, up to about 4 wt.%, up to about 3.5 wt.%, up to about 3 wt.%, up to about 2.5 wt.%.

Spray drying is a process that converts the liquid mixture to a dried particulate form, through atomization of the liquid mixture and removal of the solvent. Atomization may be done, for example, through a nozzle or on a rotating disk. By means of spray drying, the solvent is evaporated by flash evaporation, for example at a temperature close to the boiling point thereof, leaving the compound of Formula (I) precipitated in a matrix formed by the polymer. Optionally, a secondary drying process such as fluidized bed drying or vacuum drying, may be used to reduce residual solvents to pharmaceutically acceptable levels.

Typically, spray drying involves contacting a highly dispersed liquid mixture and a sufficient volume of hot air or gas to produce evaporation and drying of the liquid droplets. The liquid mixture to be spray dried can be any solution, suspension, coarse suspension, slurry, colloidal dispersion, or paste that may be atomized using the selected spray drying apparatus. In a standard procedure, the liquid mixture is sprayed into a current of warm filtered air or gas that evaporates the solvent and conveys the dried product to a collector (e.g., a cyclone or directly to a membrane filter bag). The spent air may then be exhausted with the solvent, or alternatively the spent air may be sent to a condenser to capture and optionally recycle the solvent. A commercially available spray dry apparatus may be used to conduct the spray drying. For example, commercial spray dryers are manufactured by Buchi Ltd., and NIRO^{®} and PHARMASD^{™} spray dryers from GEA *(see,* US 2004/0105820; US 2003/0144257). Spray dyers-spray chillers/congealers such as PROCEPT 4M8-Trix available from Procept, may also be used. For example, a pressure nozzle, a two-fluid electrosonic nozzle, a two-fluid nozzle, a three-fluid nozzle, a cooled nozzle, a heated nozzle, an ultrasonic nozzle, or a rotary atomizer can be used.

Techniques and methods for spray drying may be found in Perry's Chemical Engineering Handbook, 6th Ed., R. H. Perry, D. W. Green & J. O. Maloney, eds.), McGraw-Hill book co. (1984); and Marshall "Atomization and Spray Drying" 50, Chem. Eng. Prog. Monogr. Series 2 (1954). In general, the spray drying may be conducted with an inlet temperature of from about 40°C, from about 45°C, from about 50°C, from about 60°C, from about 70°C, to about 200°C, to about 150°C, to about 100°C, to about 75°C, e.g., about 50°C. The spray drying may generally be conducted with an outlet temperature of from about 15°C, from about 20°C, from about 25°C, to about 100° C, to about 75°C, to about 50°C, to about 40°C, to about 30°C, e.g., about 27°C.

Removal of the solvent may optionally involve a subsequent drying step, such as tray drying, fluid bed drying (e.g., from about room temperature to about 100°C., e.g., about 60°C), vacuum drying, microwave drying, rotary drum drying, or biconical vacuum drying (e.g., from about room temperature to about 100°C, e.g., about 60°C or lower).

Fluidized spray drying techniques may also be employed herein. The process of fluidized spray drying combines spray drying and fluid bed drying technologies. Agglomerated powders are obtained based on the integrated fluid bed or belt and a multi-stage process where moist powder, produced during the first drying stage, forms agglomerates, which are post-dried and cooled in the following stages. Briefly, a pressure nozzle, a two-fluid electrosonic nozzle, a two-fluid nozzle, or a rotary atomizer sprays the liquid mixture down into the spray dryer towards the fluid bed. Agglomeration incorporating finer, recycled material takes place in the spray dryer, and agglomerated particles fall to the bed. Agglomerated particles may be further dried in the bed. Exhaust air outlets through the roof causing further agglomeration in the zone of spraying.

As an example, in the spray dryer, the liquid mixture is sprayed from the atomization nozzle mounted on top of the drying chamber into the drying air and down the spray chamber. The vigorous fluidization of moist powder in the fluid bed located at the chamber base, plus recycle of fines from the cyclone attachment, result in spray drying taking place in a powder-laden atmosphere. Particles of higher moisture content can be handled in the drying chamber due to the resulting powdering effect. Drying can be completed at lower powder and exhaust air temperatures. The degree of agglomeration and thus the particle size distribution can be influenced by changing the operation conditions and the location where fines are re-introduced into the drying chamber. By adjusting the operation conditions, a solid dispersion with properties favorable for downstream processing, can be obtained.

### 2. Lyophilization

Solid dispersions (e.g., solid molecular complexes) may be prepared through lyophilization of aqueous formulations comprising water (and optionally one or more co-solvents), an active ingredient (e.g., a compound of Formula (I) or a pharmaceutically acceptable salt, a polymorph, stereoisomer, a tautomer, or solvate thereof), a suitable polymer(s) (e.g., gelatin), and any desired optional pharmaceutically acceptable excipient (e.g., mannitol).

Typically, a water-soluble polymer and any desired pharmaceutically acceptable excipient may be dissolved in water or aqueous solvent system comprising water and organic solvent(s), examples of which are set forth herein. Optional heating may be employed if desired, for example from about 40°C, from about 45°C, from about 50°C, from about 55°C, to about 100°C, to about 90°C, to about 80°C, to about 70°C, to about 60°C, to ensure complete dissolution of components. Optional cooling may also be employed prior to addition of the active ingredient. The active ingredient may then be mixed with the aqueous mixture (e.g., via stirring, vortexing, etc.), followed by any desired pH adjustment using a pH modifier (e.g., sodium hydroxide solution). The aqueous formulation may be charged into blister pockets, if desired, for producing unit dosage forms. Flash freezing may then be performed, e.g., using liquid nitrogen, dry ice, or cryogenic equipment, and the frozen mixture may then be subjected to low pressure (vacuum) conditions, preferably while being held at reduced temperature (e.g., 0°C or below, -5°C or below, -10°C or below, -15°C or below).

By means of lyophilization, the solvent (water and optionally one or more co-solvents) is evaporated under vacuum (low vapor pressure), leaving the compound of Formula (I) precipitated in a matrix formed by the polymer(s) and any optional excipients present.

### 3. Solvent Controlled Precipitation

In solvent controlled precipitation processes, an active ingredient (e.g., a compound of Formula (I) or a pharmaceutically acceptable salt, a polymorph, stereoisomer, a tautomer, or solvate thereof), a suitable polymer(s), and any desired optional pharmaceutically acceptable excipient may be dissolved in a solvent, e.g., dimethylacetamide, dimethylformamide, dimethyl sulfoxide (DMSO), N-methyl pyrrolidone (NMP), etc. The resulting solution is added to an aqueous phase comprising water adjusted to an appropriate pH (for example, in some embodiments, a pH of 3 or less). The aqueous phase may be set to any desired temperature, such as from about 0°C to about 7°C, or about 2°C to about 5°C. This causes the compound of Formula (I) to microprecipitate in a matrix formed by the polymer. The microprecipitate may be washed several times with aqueous medium until the residual solvent falls below an acceptable limit for that solvent. An "acceptable limit" for each solvent is determined pursuant to the International Conference on Harmonization (ICH) guidelines.

In some embodiments, a solution comprising the compound of Formula (I), an organic solvent (e.g., dimethylformamide, dimethylacetamide (DMA), dimethyl sulfoxide (DMSO), N-methyl pyrrolidone (NMP), and the like) and the polymer is formed. For example, the organic solvent can be DMA at 20 to 25°C. The solution may be formed by first dissolving the compound of Formula (I) into the organic solvent. Then, while stirring, the polymer is added. The mixture may be optionally heated to, for example to between about 50°C to about 110°C, e.g., about 70°C.

The aqueous phase may be an acidic aqueous solution such as dilute HCl (e.g., 0.01 N HCl) The aqueous phase may be set to any desired temperature, typically between 0°C and about 60°C, or between 5°C and 15°C.

The aqueous phase may be circulated through the mixing chamber of a high shear mixer while the solution comprising the active ingredient, polymer, and any excipients is dosed into the chamber while the chamber is operating. Dosing may be accomplished with, for example, a gear pump, a hose pump, or a syringe pump. In some embodiments, dosing is accomplished using a gear pump with an injector nozzle pointed into the mixing chamber. The mixing chamber can comprise a rotor and a stator. The rotor and the stator may, for example, each have either one or two rows of teeth. In some embodiments, the rotor and the stator each have one row of teeth. The tip speed of the rotor can be set at between about 15 and about 25 m/sec.

During the mixing process, the active ingredient (e.g., the compound of Formula (I)) and the polymer precipitate, producing a suspension of particles of the solid dispersion in aqueous-organic media. The suspension may then be subjected to a number of passes through a dispersing unit in order to adjust the particle size of the particles of the solid dispersion. The suspension may then be centrifuged and washed with the aqueous phase several times in order to remove the organic solvent and then washed once with pure water. The obtained product may then be delumped and dried to isolate the solid dispersion of the present disclosure. During the drying process, the temperature can be kept below 40°C, if needed, to prevent recrystallization of the compound of Formula (I).

Potentially beneficial excipients may fall generally into the following classes: other matrix materials or diluents, surface active agents, drug complexing agents or solubilizing agents, fillers, disintegrants, binders, lubricants, and pH modifiers (e.g., acids, bases, or buffers). Examples of other matrix materials, fillers, or diluents include lactose, mannitol, xylitol, microcrystalline cellulose, calcium diphosphate, and starch. Examples of surface active agents include sodium lauryl sulfate and polysorbate 80. Examples of drug complexing agents or solubilizing agents include the polyethylene glycols, caffeine, xanthene, gentisic acid and cylodextrins. Examples of disintegrants include sodium starch gycolate, sodium alginate, carboxymethyl cellulose sodium, methyl cellulose, and croscarmellose sodium. Examples of binders include methyl cellulose, microcrystalline cellulose, starch, and gums such as guar gum, and tragacanth. Examples of lubricants include magnesium stearate and calcium stearate. Examples of pH modifiers include acids such as citric acid, acetic acid, ascorbic acid, lactic acid, aspartic acid, succinic acid, phosphoric acid, and the like; bases such as sodium acetate, potassium acetate, calcium oxide, magnesium oxide, trisodium phosphate, sodium hydroxide, calcium hydroxide, aluminum hydroxide, and the like, and buffers generally comprising mixtures of acids and the salts of said acids. At least one function of inclusion of such pH modifiers is to control the dissolution rate of the drug, matrix polymer, or both, thereby controlling the local drug concentration during dissolution.

Excipients may be incorporated into the amorphous solid dispersion during or after its formation. In addition to the above excipients, use of any conventional materials and procedures for formulation and preparation of suitable dosage forms (e.g., oral dosage forms) using the pharmaceutical compositions disclosed herein known by those skilled in the art may be used.

### 4. pH-Controlled Precipitation

A pH-controlled precipitation process involves the microprecipitation of an active ingredient (e.g., a compound of Formula (I) or a pharmaceutically acceptable salt, a polymorph, stereoisomer, a tautomer, or solvate thereof), a suitable polymer(s), and any desired optional pharmaceutically acceptable excipient. In this process, the active ingredient (e.g., the compound of Formula (I)), the polymer, and any desired excipients are dissolved at a high pH and precipitated by lowering the pH of the solution, or vice versa.

In some embodiments, the polymer is insoluble at low pH. The compound of Formula (I) and the polymer are dissolved in an organic solvent such as dimethylformamide, dimethylacetamide (DMA), dimethyl sulfoxide (DMSO), N-methyl pyrrolidone (NMP), and the like. The pH of the solution is then lowered through use of an acid. The acid may be added to the solution of the compound of Formula (I) and polymer, the solution of the compound of Formula (I) and polymer may be added to the acid, or the solution and the acid may be combined and mixed simultaneously. At the lowered pH, both the compound of Formula (I) and the polymer simultaneously precipitate out, resulting in the solid dispersion containing the compound of Formula (I) embedded in a matrix formed by the polymer. The resulting solid dispersion may then be washed with water to remove the organic solvent, and dried to pharmaceutically acceptable levels.

### 5. Hot Melt Extrusion Process

Microprecipitation of the active ingredient (e.g., compound of Formula (I) or a pharmaceutically acceptable salt, a polymorph, stereoisomer, a tautomer, or solvate thereof) and optionally a pharmaceutically acceptable excipient in a polymer can be achieved in some embodiments by a hot melt extrusion process. Here, the components are mixed and fed continuously to a temperature-controlled extruder causing the compound of Formula (I) to be molecularly dispersed, together with any excipients present, in the molten polymer.

Hot melt extruders typically contain four primary parts: a motor that controls the rotation of the screws, the screws (primary source of shear and moving the material), the barrels that house the screws and provide temperature control, and the die (the exit port) that controls the shape and size of the extrudates. The desired materials constituting the solid dispersions (usually granular or in powder form) are generally fed into the extruder feeding port at a controlled rate while the extruder screws are rotating. The material is then conveyed forward using the rotation of screw and the friction of the material against the barrel surface. Depending on the type of extruder, a single screw or a twin screw may be used to operate either in counter or co-rotating mode. The screws can be appropriately designed to achieve a desired degree of mixing. In general, the barrels are segmented to enable temperature adjustment in each zone throughout the screw length. The exit port (the die system) controls the shape and size of the extrudates.

The resulting extrudate may then be cooled, e.g., to room temperature to produce the solid dispersion in extrudate form, which may be milled, e.g., into a fine powder.

### 6. Supercritical Fluid Process

In this process, the active ingredient (e.g., compound of Formula (I) or a pharmaceutically acceptable salt, a polymorph, stereoisomer, a tautomer, or solvate thereof), optional pharmaceutically acceptable excipient, and a polymer are dissolved in a supercritical fluid such as liquid nitrogen or liquid carbon dioxide. The supercritical fluid is then removed by evaporation leaving the compound of Formula (I) microprecipitated in the matrix formed by the polymer. In a different method, the compound of Formula (I) and a polymer are dissolved in a suitable solvent. A microprecipitated powder can then be formed by spraying the solution in a supercritical fluid which acts as an antisolvent.

Methods of making solid dispersions (e.g., solid molecular complexes) of the present disclosure are not limited to the above-described methods, and other methods known to those of ordinary skill in the art may also be used, for example, solution casting to make solid dispersions in film form.

In any of the above methods, determination of whether the compound of Formula (I) has been successfully immobilized in amorphous form in the solid dispersion (i.e., whether an amorphous solid dispersion has been formed) can be made by various means, including X-ray powder diffraction. In addition, the glass transition temperature of the solid dispersion can be measured using modulated DSC and this can also provide information whether the dispersion is a multiphase or uniphase. A uniphase is indicative of such immobilization.

Therapeutic applications and methods Any references to methods of treatment by therapy or surgery or *in vivo* diagnosis methods of this description is to be interpreted as a reference to compounds, pharmaceutical compositions and medicaments of the present invention for use in those methods.

Provided herein are methods of treating a subject with a disease or disorder comprising administering to the subject one or more (e.g., 1, 2, 3, 4, 5, or more) pharmaceutical compositions as disclosed herein.

Also disclosed is a method of treating a subject with a disease or disorder associated with a serotonin 5-HT₂ receptor comprising administering to the subject one or more pharmaceutical compositions as disclosed herein.

The dosage and frequency (single or multiple doses) of administration can vary depending upon a variety of factors, including, but not limited to, the active ingredient(s) to be administered; the disease/condition being treated; route of administration; size, age, sex, health, body weight, body mass index, and diet of the recipient; nature and extent of symptoms of the disease being treated; presence of other diseases or other health-related problems; kind of concurrent treatment; and complications from any disease or treatment regimen. Other therapeutic regimens or agents can be used in conjunction with the methods and compounds disclosed herein.

Therapeutically effective amounts for use in humans may be determined from animal models. For example, a dose for humans can be formulated to achieve a concentration that has been found to be effective in animals. The dosage in humans can be adjusted by monitoring response to the treatment and adjusting the dosage upwards or downwards.

Dosages may be varied depending upon the requirements of the subject and the active ingredient (e.g., a compound of Formula (I)) being employed. The dose administered to a subject, in the context of the pharmaceutical compositions presented herein, should be sufficient to affect a beneficial therapeutic response in the subject over time. The size of the dose also will be determined by the existence, nature, and extent of any adverse side effects. Generally, treatment is initiated with smaller dosages, which are less than the optimum dose of the active ingredient. Thereafter, the dosage is increased by small increments until the optimum effect under circumstances is reached.

Dosage amounts and intervals can be adjusted individually to provide levels of the administered compounds effective for the particular clinical indication being treated. This will provide a therapeutic regimen that is commensurate with the severity of the individual's disease state.

Routes of administration may include oral routes (e.g., enteral/gastric delivery, intraoral administration such buccal, lingual, and sublingual routes), parenteral routes (e.g., intravenous, intraarterial, intraperitoneal, intrathecal, intraventricular, intraurethral, intrasternal, intracranial, intramuscular, intrasynovial, and subcutaneous administration), and topical routes (e.g., (intra)dermal, conjuctival, intracorneal, intraocular, ophthalmic, auricular, transdermal, nasal, vaginal, uretheral, respiratory, and rectal administration), or others sufficient to affect a beneficial therapeutic response.

Administration may follow a continuous administration schedule, or an intermittent administration schedule. The administration schedule may be varied depending on the active ingredient employed, the condition being treated, the administration route, etc. For example, administration may be performed once a day (QD), or in divided dosages throughout the day, such as 2-times a day (BID), 3-times a day (TID), 4-times a day (QID), or more. In some embodiments administration may be performed nightly (QHS). In some embodiments, the compounds/pharmaceutical compositions may be administered as needed (PRN). Administration may also be performed on a weekly basis, e.g., once a week, twice a week, three times a week, four times a week, every other week, or other administration schedules deemed appropriate using sound medical judgement.

The dosing can be continuous (7 days of administration in a week) or intermittent, for example, depending on the pharmacokinetics and a particular subject's clearance/accumulation of the active ingredient. If intermittently, the schedule may be, for example, 4 days of administration and 3 days off (rest days) in a week or any other intermittent dosing schedule deemed appropriate using sound medical judgement. For example, intermittent dosing may involve administration of a single dose within a treatment course. The dosing whether continuous or intermittent is continued for a particular treatment course, typically at least a 28-day cycle (1 month), which can be repeated with or without a drug holiday. Longer or shorter courses can also be used such as 14 days, 18 days, 21 days, 24 days, 35 days, 42 days, 48 days, or longer, or any range therebetween. The course may be repeated without a drug holiday or with a drug holiday depending upon the subject. Other schedules are possible depending upon the presence or absence of adverse events, response to the treatment, patient convenience, and the like.

In some embodiments, the pharmaceutical compositions of the disclosure may be used as a standalone therapy. In some embodiments, the pharmaceutical compositions of the disclosure may be used as an adjuvant/combination therapy.

Utilizing the teachings provided herein, an effective prophylactic or therapeutic treatment regimen can be planned that does not cause substantial toxicity or adverse side effects (e.g., caused by sedative or psychotomimetic toxic spikes in plasma concentration of any of the compounds Formula (I)), and yet is entirely effective to treat the clinical symptoms demonstrated by the particular patient. This planning should involve the careful choice of active ingredient (e.g., a compound of Formula (I) or a pharmaceutically acceptable salt, a polymorph, stereoisomer, a tautomer, or solvate thereof) and dosage form by considering factors such as compound potency, release kinetics, relative bioavailability, patient body weight, presence and severity of adverse side effects, preferred mode of administration, and the toxicity profile of the selected agent.

A therapeutically effective dose of the pharmaceutical composition disclosed herein may vary depending on the variety of factors described above, but is typically that which provides the compound of Formula (I) in an amount of about 0.00001 mg to about 10 mg per kilogram body weight of the recipient, or any range in between, e.g., about 0.00001 mg/kg, about 0.00005 mg/kg, about 0.0001 mg/kg, about 0.0005 mg/kg, about 0.001 mg/kg, about 0.005 mg/kg, about 0.01 mg/kg, about 0.05 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1.0 mg/kg, about 2.0 mg/kg, about 3.0 mg/kg, about 4.0 mg/kg, about 5.0 mg/kg, about 6.0 mg/kg, about 7.0 mg/kg, about 8.0 mg/kg, about 9.0 mg/kg, about 10.0 mg/kg of the compound of Formula (I) (active).

The pharmaceutical compositions may be administered to provide the compound of Formula (I), or a pharmaceutically acceptable salt, a polymorph, stereoisomer, a tautomer, or solvate thereof, at a psychedelic dose. Psychedelic dosing, by mouth or otherwise, may range from about 0.083 mg/kg, about 0.09 mg/kg, about 0.1 mg/kg, about 0.15 mg/kg, about 0.2 mg/kg, about 0.25 mg/kg, about 0.3 mg/kg, about 0.35 mg/kg, about 0.4 mg/kg, about 0.45 mg/kg, about 0.5 mg/kg, and up to about 1 mg/kg, about 0.95 mg/kg, about 0.9 mg/kg, about 0.85 mg/kg, about 0.8 mg/kg, about 0.75 mg/kg, about 0.7 mg/kg, about 0.65 mg/kg, about 0.6 mg/kg, about 0.55 mg/kg of the compound of Formula (I) (active). Higher dosing may also be used in some embodiments, as described above. In some embodiments, psychedelic doses are administered once by mouth, with the possibility of repeat doses at least one week apart. In some instances, no more than 5 doses are given in any one course of treatment. Courses can be repeated as necessary, with or without a drug holiday. Such acute treatment regimens may be accompanied by psychotherapy, before, during, and/or after the psychedelic dose. These treatments are appropriate for a variety of mental health disorders disclosed herein, examples of which include, but are not limited to, major depressive disorder (MDD), therapy resistant depression (TRD), anxiety disorders, and substance use disorders (e.g., alcohol use disorder, opioid use disorder, amphetamine use disorder, nicotine use disorder, smoking, and cocaine use disorder).

The pharmaceutical compositions may be administered to provide the compound of Formula (I), or a pharmaceutically acceptable salt, a polymorph, stereoisomer, a tautomer, or solvate thereof, at serotonergic, but sub-psychoactive concentrations to achieve durable therapeutic benefits, with decreased toxicity, and may thus be suitable for low dosing or microdosing. For example, when administered by mouth, the dose range for sub-psychedelic dosing may range from about 0.00001 mg/kg, about 0.00005 mg/kg, about 0.0001 mg/kg, about 0.0005 mg/kg, about 0.001 mg/kg, about 0.005 mg/kg, about 0.006 mg/kg, about 0.008 mg/kg, about 0.009 mg/kg, about 0.01 mg/kg, and less than about 0.083 mg/kg, about 0.08 mg/kg, about 0.075 mg/kg, about 0.07 mg/kg, about 0.06 mg/kg, about 0.05 mg/kg, about 0.04 mg/kg, about 0.03 mg/kg, about 0.02 mg/kg of the compound of Formula (I) (active). In some embodiments, sub-psychedelic doses are administered orally up to every day, for a treatment course (e.g., 1 month). However, there is no limitation on the number of doses at sub-psychedelic doses-dosing can be less frequent or more frequent as deemed appropriate. Courses can be repeated as necessary, with or without a drug holiday.

Sub-psychedelic dosing can also be carried out, for example, by transdermal delivery, subcutaneous administration, modified, controlled, slow, or extended-release dosage forms, including, but not limited to, depot dosage forms, implants, patches, and pumps, which can be optionally remotely controlled, matrix controlled release devices, osmotic controlled release devices, multiparticulate controlled release devices, ion-exchange resins, enteric coatings, multilayered coatings, microspheres, liposomes, and combinations thereof. Here, doses would achieve similar blood levels as low oral dosing, but would nevertheless be sub-psychedelic.

Sub-psychedelic doses can be used, e.g., for the chronic treatment a variety of diseases or disorders disclosed herein, examples of which include, but are not limited to, inflammation, pain and neuroinflammation. In such settings where chronic administration is performed over extended periods of time, the stabilized forms of the compounds provided in the present disclosure become increasingly valuable.

The subjects treated herein may have a disease or disorder associated with a serotonin 5-HT₂ receptor.

In some embodiments, the disease or disorder is a neuropsychiatric disease or disorder or an inflammatory disease or disorder. In some embodiments, the neuropsychiatric disease or disorder is not schizophrenia or cognitive deficits in schizophrenia.

In some embodiments, the disease or disorder is a central nervous system (CNS) disorder, including, but not limited to, major depressive disorder (MDD), treatment-resistant depression (TRD), post-traumatic stress disorder (PTSD), bipolar and related disorders (including, but not limited to, bipolar I disorder, bipolar II disorder, cyclothymic disorder), obsessive-compulsive disorder (OCD), generalized anxiety disorder (GAD), social anxiety disorder, substance use disorders (including, but not limited to, alcohol use disorder, opioid use disorder, amphetamine use disorder, nicotine use disorder, smoking, and cocaine use disorder), eating disorders (including, but not limited to anorexia nervosa, bulimia nervosa, binge-eating disorder, etc.), Alzheimer's disease, cluster headache and migraine, attention deficit hyperactivity disorder (ADHD), pain and neuropathic pain, aphantasia, childhood-onset fluency disorder, major neurocognitive disorder, mild neurocognitive disorder, suicidal ideation, suicidal behavior, major depressive disorder with suicidal ideation or suicidal behavior, melancholic depression, atypical depression, dysthymia, non-suicidal self-injury disorder (NSSID), chronic fatigue syndrome, Lyme's disease, gambling disorder, paraphilic disorders (including, but not limited to, pedophilic disorder, exhibitionistic disorder, voyeuristic disorder, fetishistic disorder, sexual masochism or sadism disorder, and transvestic disorder, etc.), sexual dysfunction (e.g., low libido, hypoactive sexual desire disorder (HSDD), etc.), peripheral neuropathy, and obesity.

In some embodiments, the disease or disorder is major depressive disorder (MDD).

In some embodiments, the disease or disorder is treatment-resistant depression (TRD).

In some embodiments, the disease or disorder is an anxiety-related disorder, such as generalized anxiety disorder (GAD), social anxiety disorder, panic disorder, a phobia-related disorder (e.g., phobias related to flying, heights, specific animals such as spiders/dogs/snakes, receiving injections, blood, etc., agoraphobia), separation anxiety disorder, selective mutism, etc. In some embodiments, the disease or disorder is generalized anxiety disorder (GAD). In some embodiments, the disease or disorder is social anxiety disorder.

In some embodiments, the disease or disorder is a compulsive disorder, such as obsessive-compulsive disorder (OCD), body-focused repetitive behavior, hoarding disorder, gambling disorder, compulsive buying, compulsive internet use, compulsive video gaming, compulsive sexual behavior, compulsive eating, compulsive exercise, etc. In some embodiments, the disease or disorder is obsessive-compulsive disorder (OCD).

In some embodiments, the disease or disorder is headaches (e.g., cluster headache, migraine, etc.).

In some embodiments, the disease or disorder is a substance use disorder. In some embodiments, the disease or disorder is alcohol use disorder. In some embodiments, the disease or disorder is smoking disorder and the therapy is used for smoking cessation.

Pharmaceutical compositions of the present disclosure may provide cognitive benefits to subject's suffering from neurological and neurodegenerative diseases such as Alzheimer's disease and other dementia subtypes, Parkinson's disease, etc. For example, emerging psychedelic research/clinical evidence indicates that psychedelics, such as psilocybin, may be useful as disease-modifying treatments in subjects suffering from neurodegenerative diseases such as Alzheimer's disease and other forms of dementia. *See* Vann Jones, S.A. and O'Kelly, A. "Psychedelics as a Treatment for Alzheimer's Disease Dementia" Front. Synaptic Neurosci., 21, August 2020; Kozlowska, U., Nichols, C., Wiatr, K., and Figiel, M. (2021), "From psychiatry to neurology: Psychedelics as prospective therapeutics for neurodegenerative disorders" Journal of Neurochemistry, 00, 1- 20; Garcia-Romeu, A., Darcy, S., Jackson, H., White, T., Rosenberg, P. (2021), "Psychedelics as Novel Therapeutics in Alzheimer's Disease: Rationale and Potential Mechanisms" In: Current Topics in Behavioral Neurosciences. Springer, Berlin, Heidelberg. For example, psychedelics such as psilocybin are thought to stimulate neurogenesis, provoke neuroplastic changes, and to reduce neuroinflammation. Thus, in some embodiments, the solid dispersions of the present disclosure are used for the treatment of neurological and neurodegenerative disorders. In some embodiments, the solid dispersions of the present disclosure are used for the treatment of Alzheimer's disease. In some embodiments, the solid dispersions of the present disclosure are used for the treatment of dementia. In some embodiments, the solid dispersions of the present disclosure are used for the treatment of Parkinson's disease. As described above, such treatment may stimulate neurogenesis, provoke neuroplastic changes, and/or provide neuroinflammatory benefits (e.g., reduced neuroinflammation compared to prior to the beginning of treatment), and as a result, may slow or prevent disease progression, slow or reverse brain atrophy, and reduce symptoms associated therewith (e.g., memory loss in the case of Alzheimer's and related dementia disorders). While not limited thereto, pharmaceutical compositions adapted for oral and/or extended-release dosing are appropriate for such treatment methods, with sub-psychedelic dosing being preferred.

Further, many of the behavioral issues associated with chronic and/or life-threatening illnesses, including neurodegenerative disorders such as Alzheimer's disease, may benefit from treatment with the solid dispersions disclosed herein. Indeed, depression, anxiety, or stress can be common among patients who have chronic and/or life-threatening illnesses such as Alzheimer's disease, autoimmune diseases (e.g., systemic lupus erythematosus, rheumatoid arthritis, and psoriasis), cancer, coronary heart disease, diabetes, epilepsy, HIV/AIDS, hypothyroidism, multiple sclerosis, Parkinson's disease, and stroke. For example, depression is common in Alzheimer's disease as a consequence of the disease, as well as being a risk factor for the disease itself. Symptoms of depression, anxiety, or stress can occur after diagnosis with the disease or illness. Patients that have depression, anxiety, or stress concurrent with another medical disease or illness can have more severe symptoms of both illnesses and symptoms of depression, anxiety, or stress can continue even as a patient's physical health improves. Pharmaceutical compositions described herein can be used to treat depression, anxiety, and/or stress associated with a chronic or life-threatening disease or illness.

Accordingly, in some embodiments, the methods herein are used to treat symptoms, e.g., depression, anxiety, and/or stress, associated with a chronic and/or life-threatening disease or disorder. In some embodiments, the disease or disorder is Alzheimer's disease. In some embodiments, the methods herein are used for the treatment of depression, anxiety, and/or stress associated with Alzheimer's disease. In some embodiments, the disease or disorder is Parkinson's disease. In some embodiments, the methods herein are used for the treatment of depression, anxiety, and/or stress associated with Parkinson's disease. In some embodiments, the disease or disorder is cancer related depression and anxiety. As discussed above, oral and/or extended-release dosing is appropriate for such applications, particularly when blood concentrations of active ingredient (e.g., a compound of Formula (I)) are kept below the psychedelic threshold.

In some embodiments, the disease or disorder is a neurological and developmental disorder such as autism spectrum disorder, including Asperger's syndrome. For example, Asperger's syndrome is a subtype of autism spectrum disorder that is treatable with anxiety drugs.

In some embodiments, the disease or disorder is a genetic condition that causes learning disabilities and cognitive impairment. An example of such a genetic condition is fragile X syndrome, which can cause mild to moderate intellectual disabilities in most males and about one-third of affected females. Subjects with fragile X syndrome may display anxiety, hyperactive behavior (e.g., fidgeting and impulsive actions), attention deficit disorder, and/or features of autism spectrum disorder, and these signs and symptoms may be treated with the methods herein.

In some embodiments, the disease or disorder is mental distress, e.g., mental distress in frontline healthcare workers.

In some embodiments, the disease or disorder includes conditions of the autonomic nervous system (ANS).

In some embodiments, the disease or disorder includes pulmonary disorders including asthma and chronic obstructive pulmonary disorder (COPD).

In some embodiments, the disease or disorder includes cardiovascular disorders including atherosclerosis.

The administering physician can provide a method of treatment that is prophylactic or therapeutic by adjusting the amount and timing of any of the compounds described herein on the basis of observations of one or more symptoms of the disorder or condition being treated. In some embodiments, the subject is a mammal. In some embodiments, the mammal is a human.

Also disclosed herein is a method for decreasing time of therapeutic onset relative to a crystalline psilocybin-based drug, comprising administering a pharmaceutical composition comprising a solid dispersion as disclosed herein to a patient in need thereof. For example, solid dispersions of the present disclosure formulated with a therapeutically effective amount of a compound of Formula (I) in amorphous form dispersed in a polymer may provide a faster therapeutic onset compared to solid dosage forms formulated with a crystalline psilocybin-based drug administered in substantially the same way (e.g., each administered orally).

Also disclosed herein is a method of reducing psychedelic side effects relative to a crystalline psilocybin-based drug, comprising administering a pharmaceutical composition as disclosed herein to a patient in need thereof. For example, solid dispersions of the present disclosure formulated with a therapeutically effective amount of a compound of Formula (I) in amorphous form dispersed in a polymer may provide fewer psychedelic side effects compared to solid dosage forms formulated with a crystalline psilocybin-based drug administered in substantially the same way (e.g., each administered orally).

The terms "hallucinogenic" side effects and "psychedelic side effects" are used in the present disclosure interchangeably to refer to unwanted and/or unintended secondary effects caused by the administration of a medicament to an individual resulting in subjective experiences being qualitatively different from those of ordinary consciousness. These experiences can include derealization, depersonalization, hallucinations and/or sensory distortions in the visual, auditory, olfactory, tactile, proprioceptive and/or interoceptive spheres and/or any other perceptual modifications, and/or any other substantial subjective changes in cognition, memory, emotion and consciousness.

In some embodiments, the administration of pharmaceutical compositions as disclosed herein cause less hallucinogenic and/or psychedelic side effects and/or no hallucinogenic and/or psychedelic side effects relative to a psilocybin-based drug currently available (e.g., crystalline psilocybin-based drugs) or a psilocybin-based drug administered without alkaline phosphatase (ALP). In some embodiments, the administration of pharmaceutical compositions as disclosed herein alleviates, reduces, removes, and/or eliminates the hallucinogenic and/or psychedelic side effects caused by a psilocybin-based drug currently available (e.g., crystalline psilocybin-based drugs) or a psilocybin-based drug administered without ALP. In some embodiments, the administration of the pharmaceutical composition as disclosed herein alleviates, reduces, removes, and/or eliminates any neurologically toxic spikes relative to a psilocybin-based drug currently available (e.g., crystalline psilocybin-based drug) or a psilocybin-based drug administered without ALP.

Neurologically toxic spikes are spikes in the concentration of an active ingredient as described herein that can produce side-effects of sedation or psychotomimetic effects, e.g., hallucination, dizziness, and nausea; which can not only have immediate repercussions, but also effect treatment compliance. In particular, side effects may become more pronounced at blood concentration levels of about 250, 300, 400, 500 ng/L or more.

Also disclosed herein is a method of decreasing duration of therapeutic effect compared to a crystalline psilocybin-based drug, comprising administering the pharmaceutical composition as disclosed herein to a patient in need thereof.

Generally, a duration of therapeutic effect for a psilocybin-based drug currently available (e.g., a crystalline psilocybin-based drug or a psilocybin-based drug administered without ALP), is about 6-8 hours. In some embodiments, the duration of therapeutic effect of the pharmaceutical composition as disclosed herein is less than the duration of therapeutic effect for a crystalline psilocybin-based drug. In some embodiments, the duration of therapeutic effect of the pharmaceutical composition as disclosed herein is 120, 110, 100, 90, 80, 70, 60, 55, 50, 45, 40, 35, 30, 25, 20, 15, 10, 5 minutes or less. In some embodiments, the duration of therapeutic effect of the novel compositions discussed herein is less than the duration of therapeutic effect of current conventional psilocybin-based drugs or current conventional psilocybin-based drugs administered without ALP by 7, 6.5, 6, 5.5, 5, 4.5, 4, 3.5, 3, 2.5, 2, 1.5, or 1 hour or less, or 120, 110, 100, 90, 80, 70, 60, 55, 50, 45, 40, 35, 30, 25, 20, 15, 10, 5 minutes or less.

### EXAMPLES

### I. Compound Synthesis

### Synthesis of 3-(2-(bis(methyl-d₃)amino)ethyl-1,1,2,2-d₄)-1H-indol-4-yl dihydrogen phosphate (I-3)(psilocybin-d₁₀ or PY-d₁₀)

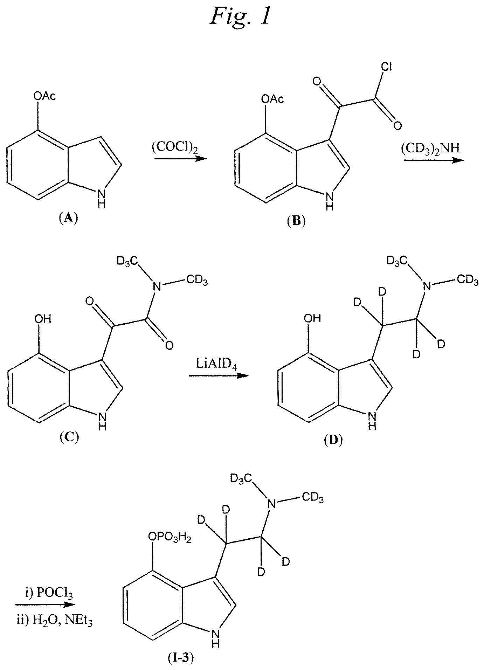

Compound 3-(2-(bis(methyl-*d*₃)amino)ethyl-1,1,2,2-*d*₄)-1H-indol-4-yl dihydrogen phosphate (**I-3**)(psilocybin-*d*₁₀ or PY-*d*₁₀) is synthesized according to Fig. 1. 4-acetoxyindole (**A**) is acylated using oxalyl chloride producing intermediate **B** as a yellow solid. Treatment of intermediate **B** with dimethyl-d*₆*-amine (Cambridge Isotopes Labs, Tewksbury, MA) results in amidation and de-acetylation forming intermediate C, which is then reduced by LiAlD₄ to form psilocin-*d*₁₀ (**D**).

Psilocin-*d*₁₀ (**D**) is converted into psilocybin-*d*₁₀ (**I-3**) following a direct phosphorylation procedure described by Kargbo et al. (Kargbo, Robert B et al. "Direct Phosphorylation of Psilocin Enables Optimized cGMP Kilogram-Scale Manufacture of Psilocybin." ACS omega vol. 5,27 16959-16966, 1 Jul. 2020). To a clean, dry reactor under a nitrogen atmosphere THF (28.0 L, 10 vol) and phosphorus oxychloride (3.15 kg, 20.6 mol, 1.5 equiv) are charged at 20 to 25°C. The vessel is cooled to -5 to -15 °C. Separately, to a second clean, dry reactor under the nitrogen atmosphere is charged psilocin-*d*₁₀ (**D**)(2.80 kg, 13.7 mol, 1 equiv) and celite (2.80 kg, 1 wt) followed by THF (42.0 L, 15 vol). The resultant slurry is held at 18 to 25 °C for at least 2 h. The reactor contents are then cooled to 0 to -15 °C. The intermediate **D**/celite/THF slurry is slowly charged to the POCl₃ solution via pump while maintaining the internal temperature at -15 to 0 °C. The mixture is stirred for no more than 2 h at -15 to 0 °C.

During this time, to a clean reactor is prepared a quench solution of THF/H₂O (70:30) (28.0 L, 10 vol) and Et₃N (8.32 kg, 82.2 mol, 6 equiv). The vessel containing the quench mixture is cooled to -20 to 0 °C, and the crude reaction mixture is slowly added into the THF/ H₂O/Et₃N solution, maintaining the internal temperature at -20 to 0 °C. THF (2 × 5.60 L, 2 × 2 vol) is charged to the reaction mixture reactor, cooled to 0 to -5 °C, and used as a rinse into the quench medium, maintaining the internal temperature of the quenched mixture at -20 to 0°C. Purified water (8.40 L, 3 vol) is charged to the reaction mixture reactor, cooled to 2 to 7 °C, and used as a rinse into the quench medium, maintaining the internal temperature at -20 to 0 °C. The mixture was stirred at -20 to 0 °C for at least 60 min. The mixture was filtered, and the cake was washed with water at 5 to 10 °C (2 × 5.60 L, 2 × 2 vol) to dissolve any product stuck to the celite cake. The biphasic filtrate is transferred back to the reactor. A rinse with water (1.40 L, 0.5 vol) can be used as part of the transfer. The temperature is adjusted to 18 to 25 °C, and the lower aqueous phase is separated. The organic phase is removed. The lower aqueous phase contains product and the upper organic phase will typically contain residual psilocin-*d*₁₀ (**D**). The aqueous phase is transferred back to the reactor. A rinse with water (1.40 L, 0.5 vol) can be used as part of the transfer. Isopropyl alcohol (IPA, 28.0 L, 10 vol) is charged to the aqueous phase. The mixture is concentrated at <45 °C internal temp to ca. 5 vol of the remaining water. Further additions of IPA (14.0 L, 5 vol) or purified water (5.60 L, 2 vol) can be added to aid azeotropic distillation of water. Upon reaching the aqueous distillation, volume purified water (14.0 L, 5 vol) is charged at 18 to 25 °C and the solution is stirred for at least 24 h. Psilocybin-*d*₁₀ (**I-3**) will normally precipitate at this time. The reactor contents are filtered under the nitrogen atmosphere and the cake is washed with cold (2 to 6 °C) purified water (2 × 5.60 L, 2 × 2 vol) and pulled dry for at least 60 min under the nitrogen atmosphere. The solid is dried at 35 to 45 °C under vacuum for at least 24 h. The crude product is charged to a clean, dry reactor under the nitrogen atmosphere at 20 to 25 °C. Methanol (10 vol, based on crude product discharge weight) is charged to the reactor at 20 to 25 °C and the contents are stirred for at least 12 h at 20 to 25 °C. The mixture is filtered under nitrogen and the cake rinsed with methanol (2 × 1.5 vol, based on crude product discharge weight) at 20 to 25 °C. The solid is pulled dry for at least 2 h under nitrogen then charged to a clean, dry reactor under the nitrogen atmosphere. Purified water (10 vol, based on crude product discharge weight) is charged to the reactor at 20 to 25 °C, and the contents are heated to 45 to 55 °C for at least 24 h. The contents are cooled to 20 to 30 °C at a rate of 10 degrees per hour and held for at least 2 h. The mixture is filtered under the nitrogen atmosphere and washed in turn with 20 to 25 °C purified water (1 × 1 vol, 1 × 2 vol) (based on crude product discharge weight) and pulled dry under the nitrogen atmosphere for at least 2 h. The product is initially isolated in trihydrate form A by XRPD. The solid may be dried at 35 to 45 °C under vacuum for at least 24 h and subsequently at 50 to 60 °C (target 55 °C) under vacuum for at least 24 h, to convert the trihydrate form initially isolated to anhydrate form A by XRPD. A white solid is afforded as pure material by ultraperformance liquid chromatography (UPLC). The structure of the final product with deuterium enrichment over 90% will be confirmed by ¹H NMR and LC-MS.

### II. Solid dispersions prepared by freeze-drying (lyophilization)

### Materials.

Gelatin (fish gelatin (super fine), available from Ajinomoto, USA) unless specified as bovine gelatin.
Mannitol (Sigma Aldrich).
KOLLIDON^{®} 12PF (polyvinylpyrrolidone (PVP) with a weight average molecular weight of 2,500 g/mol; bulk density of 400-600 g/L; D(0.5) of 35 µm ± 5 µm, available from BASF).
METHOCEL^{™} E3 LV (low viscosity hydroxypropyl methyl cellulose (HPMC) with a 2910 substitution type: 28-30% methoxy substitution, 7-12% hydroxypropyl substitution; viscosity of 4.0-6.0 mPa·s as 2% solution in water at 20°C, available from DuPont).
METHOCEL^{™} E6 premium LV (low viscosity hydroxypropyl methyl cellulose (HPMC) with a molecular weight of 70,000-80,000 g/mol, 2910 substitution type: 28-30% methoxy substitution, 7-12% hydroxypropyl substitution; viscosity of 4.8-7.2 mPa·s as 2% solution in water at 20°C, available from DuPont).
KOLLIDON^{®} VA 64 (a 60:40 copolymer of VP:VAc, 45,000-75,000 g/mol, available from BASF).
METHOCEL^{™} K100LV (hydroxypropyl methyl cellulose (HPMC) with a molecular weight of 164,000 g/mol, available from Colorcon, Inc.).
METHOCEL^{™} K4M (hydroxypropyl methyl cellulose (HPMC) with a molecular weight of 400,000 g/mol, available from Colorcon, Inc.).
METHOCEL^{™} K15M (hydroxypropyl methyl cellulose (HPMC) with a molecular weight of 575,000 g/mol, available from Colorcon, Inc.).
AQUASOLVE^{™} HPMCAS MF (HPMCAS polymer with a substitution pattern of 7-11% acetyl, 10-14% succinoyl, 21-25% methoxyl, 5-9% hydroxypropoxy; viscosity of 2.4-3.6 mPa·s as 2% solution in water at 20°C; less than 10 µm mean particle size, available from Ashland).
BENECEL^{™} K35M Pharm (hydroxypropyl methyl cellulose (HPMC) with a 2208 substitution type; 675,000 g/mol; available from Ashland).
BENECEL^{™} K100LV PH PRM (hydroxypropyl methyl cellulose (HPMC) with a 2208 substitution type; 164,000 g/mol; available from Ashland).

Psilocybin (3-(2-(dimethylamino)ethyl)-1H-indol-4-yl dihydrogen phosphate; PY; 1-7) starting material used was in crystalline form as crystalline methanol solvate with a small quantity of crystalline Form B as described below, commercially available from Quality Chemical Labs.

### Analytical methods.

### X-ray power diffraction (XRPD):

The samples were prepared in silicon low background holders using light manual pressure to keep the sample surface flat and level with the reference surface of the sample holder. The single crystal Si low background holder has a circular recess (10 mm diameter and about 0.2 mm depth) that holds the sample.

The Rigaku Smart-Lab diffraction system used was configured for Bragg-Brentano reflection geometry using a line source X-ray beam. The Bragg-Brentano geometry was controlled by passive divergence and receiving slits with the sample itself acting as the focusing component for the optics. The figures were created using PlotMon V2.1.1.0. The XRPD parameters that were used are summarized in Table 2.

**Table 2**

| **Parameter** | **Value** |
|---|---|
| Geometry | Bragg-Brentano |
| Tube Anode | Cu K*α* |
| Tube Type | Long Fine Focus |
| Tube Voltage (kV) | 40 |
| Tube Current (mA) | 44 |
| Detector | D/teX Ultra 250 |
| Monochromatization | K*β* Filter |
| Incident Slit (°) | 1/3 |
| Receiving Slit 1 (mm) | 18 |
| Receiving Slit 2 (mm) | open |
| Start Angle (°2*θ*) | 2 |
| End Angle (°2*θ*) | 40 |
| Step Size (°2*θ*) | 0.02 |
| Scan Speed (°2*θ*/min) | 6 |
| Spinning (rpm) | 11 |
| Sample Holder | Large well silicon low background holder |

### High-resolution X-ray power diffraction (XRPD):

High-resolution XRPD analysis was performed using Rigaku Smart-Lab diffraction system configured for Debye-Scherrer transmission geometry. The Debye-Scherrer convergent beam geometry utilizes a curved x-ray mirror to focus the incident beam through the sample and onto the detector plane. The axial divergence of the X-ray beam was controlled by 5.0° Soller slits in both the incident and diffracted beam paths. The high-resolution XRPD parameters that were used are summarized in Table 3.

**Table 3**

| Parameter | Value |
|---|---|
| Geometry | Transmission |
| Tube Anode | Cu Kα |
| Tube Type | Long Fine Focus |
| Tube Voltage (kV) | 40 |
| Tube Current (mA) | 44 |
| Detector | D/teX Ultra 250 |
| Monochromatization | K*β* Filter |
| Incident Slit (mm) | 1.0 |
| Receiving Slit 1 (mm) | 18 |
| Receiving Slit 2 (mm) | 19.125 |
| Start Angle (°2*θ*) | 5 |
| End Angle (°2*θ*) | 30 |
| Step Size (°2*θ*) | 0.02 |
| Scan Speed (°2*θ*/min) | 0.5 |
| Spinning (rpm) | 12 |
| Sample Holder | Etnom^{®} films |

### Modulated differential scanning calorimetry (mDSC):

The mDSC analyses were carried out using a TA Instruments Q2000 instrument. The instrument temperature calibration was performed using indium. For each analysis, approximately 1 to 3 mg of the sample were weighed into a Tzero aluminum pan that was covered with a lid, crimped, and loaded into the DSC instrument. An empty pan of the same configuration was loaded into the reference position. Each sample was heated from 25 °C to 220 °C or 250 °C at a rate of 2 °C per minute with a ±0.42 °C modulation every 40 seconds. The DSC cell was kept under a nitrogen purge of about 50 mL per minute during each analysis. Data collection was performed using Thermal Advantage 5.5.3 software. Data collection and analysis was performed using Trios v5.0.0.44608.

Each mDSC plot shows three heat flows: total heat flow (*), reversing heat flow (**) and non-reversing heat flow (***). In typical mDSC data, reversing heat flow shows glass transition, heat capacity change and melting events whereas the non-reserving heat flow shows enthalpic recovery, evaporation, crystallization, decomposition (including chemical reactions). Some melting events may also appear in the non-reversing heat flow curve.

*Freeze-dried solid dispersions and experimental procedures.* Freeze-dried solid dispersions containing psilocybin (PY; **I-7**) in various polymer matrices were prepared as outlined below.

### Example 1

Freeze-dried tablets (50 mg) were prepared with psilocybin (PY; **I-7**) (26.67 wt.%) according to composition described in Table 4. Gelatin was dissolved in water and incubated at 60°C for 30 min until a clear solution was obtained. Crystalline psilocybin was added to the gelatin solution, vortexed vigorously, and was briefly incubated at 60°C to ensure complete solubility of the drug. The aqueous formulation of gelatin/PY was flash frozen in liquid nitrogen (-196°C), stored at -15°C for 12 hours, and lyophilized at 0°C for 12 hours under vacuum.

Reference Example 1a (placebo, matrix only) and Reference Example 1b (prepared by physical mixing (admixture of) PY + matrix) were also prepared and characterized for reference.

**Table 4.**

| Matrix composition | **Example 1** | **Reference Example 1a** | **Reference Example 1b*** |
|---|---|---|---|
| | wt. (g) | wt. (g) | wt. (g) |
| Gelatin | 0.0275 | 0.0275 | 0.0275 |
| Psilocybin | 0.01 | - | 0.01 |
| Water | 0.465 | 0.465 | 0.465 |
| Total Weight (g) | 0.5025 | 0.4925 | 0.5025 |
| Dry Weight (g) | 0.0375 | 0.0275 | 0.0375 |
| PY (g)/tablet | 0.01 | N/A | 0.01 |
| PY (wt.%) in dry 50 mg tablet | 26.67 | N/A | 26.67 |

| | | | |
|---|---|---|---|
| * Physical mixture (admixture) | | | |

### Example 2

Freeze-dried tablets (50 mg) were prepared with psilocybin (PY; **I-7**) (26.67 wt.%) according to composition described in Table 5. PVP (KOLLIDON^{®} 12PF) was dissolved in water and incubated at 60°C for 30 to 60 min until a clear solution was obtained. Crystalline psilocybin was dissolved in water and was incubated at 60°C and vortexed vigorously. Both solutions were mixed, and the resulting aqueous formulation of PVP/PY was flash frozen in liquid nitrogen (-196°C), stored at -15°C for 12 hours, and lyophilized at 0°C for 12 hours under vacuum.

Reference Example 2a (placebo, matrix only) and Reference Example 2b (prepared by physical mixing (admixture of) PY + matrix) were also prepared and characterized for reference.

**Table 5.**

| Matrix composition | **Example 2** | **Reference Example 2a** | **Reference Example 2b*** |
|---|---|---|---|
| | wt. (g) | wt. (g) | wt. (g) |
| KOLLIDON^{®} 12PF | 0.0275 | 0.0275 | 0.0275 |
| Psilocybin | 0.01 | - | 0.01 |
| Water | 0.465 | 0.465 | 0.465 |
| Total Weight (g) | 0.5025 | 0.492 | 0.5025 |
| Dry Weight (g) | 0.0375 | 0.0275 | 0.0375 |
| PY (g)/tablet | 0.01 | N/A | 0.01 |
| PY (wt.%) in dry 50 mg tablet | 26.67 | N/A | 26.67 |

| | | | |
|---|---|---|---|
| * Physical mixture (admixture) | | | |

### Example 3

Freeze-dried tablets (50 mg) were prepared with psilocybin (PY; **I-7**) (26.67 wt.%) according to composition described in Table 6. HPMC (METHOCEL^{™} E3 LV) was dissolved in water and incubated at 60°C for 30 to 60 min until a clear solution was obtained. Crystalline psilocybin was dissolved in water and was incubated at 60°C and vortexed vigorously. Both solutions were mixed, and the resulting aqueous formulation of HPMC/PY was flash frozen in liquid nitrogen (-196°C), stored at -15°C for 12 hours, and lyophilized at 0°C for 12 hours under vacuum.

Reference Example 3a (placebo, matrix only) and Reference Example 3b (prepared by physical mixing (admixture of) PY + matrix) were also prepared and characterized for reference.

**Table 6.**

| Matrix composition | **Example 3** | **Reference Example 3a** | **Reference Example 3b*** |
|---|---|---|---|
| | wt. (g) | wt. (g) | wt. (g) |
| METHOCEL^{™} E3 LV | 0.0275 | 0.0275 | 0.0275 |
| Psilocybin | 0.01 | - | 0.01 |
| Water | 0.465 | 0.465 | 0.465 |
| Total Weight (g) | 0.5025 | 0.492 | 0.5025 |
| Dry Weight (g) | 0.0375 | 0.0275 | 0.0375 |
| PY (g)/tablet | 0.01 | N/A | 0.01 |
| PY (wt.%) in dry 50 mg tablet | 26.67 | N/A | 26.67 |

| | | | |
|---|---|---|---|
| * Physical mixture (admixture) | | | |

### Example 4

Freeze-dried tablets (50 mg) were prepared with psilocybin (PY; **I-7**) (15.4 wt.%) according to composition described in Table 7. HPMC (METHOCEL^{™} E3 LV) and PVP (KOLLIDON^{®} 12PF) were dissolved in water and incubated at 60°C for 30 to 60 min until a clear solution was obtained. Crystalline psilocybin was dissolved in water and was incubated at 60°C and vortexed vigorously. Both solutions were mixed, and the resulting aqueous formulation of HPMC/PVP/PY was flash frozen in liquid nitrogen (-196°C), stored at -15°C for 12 hours, and lyophilized at 0°C for 12 hours under vacuum.

Reference Example 4a (placebo, matrix only) and Reference Example 4b (prepared by physical mixing (admixture of) PY + matrix) were also prepared and characterized for reference.

**Table 7.**

| Matrix composition | **Example 4** | **Reference Example 4a** | **Reference Example 4b*** |
|---|---|---|---|
| | wt. (g) | wt. (g) | wt. (g) |
| KOLLIDON^{®} 12PF | 0.0275 | 0.0275 | 0.0275 |
| METHOCEL^{™} E3 LV | 0.0275 | 0.0275 | 0.0275 |
| Psilocybin | 0.01 | - | 0.01 |
| Water | 0.435 | 0.435 | 0.435 |
| Total Weight (g) | 0.50 | 0.49 | 0.50 |
| Dry Weight (g) | 0.065 | 0.055 | 0.065 |
| PY (g)/tablet | 0.01 | N/A | 0.01 |
| PY (wt.%) in dry 50 mg tablet | 15.4 | N/A | 15.4 |

| | | | |
|---|---|---|---|
| * Physical mixture (admixture) | | | |

### Example 5

Freeze-dried tablets (50 mg) were prepared with psilocybin (PY; **1-7)** (13.0 wt.%) according to composition described in Table 8. Gelatin and mannitol were dissolved in water and incubated at 60°C for 30 to 60 min until a clear solution was obtained. Crystalline psilocybin was added to the gelatin/mannitol solution, which was vortexed vigorously, followed by pH modification using sodium hydroxide solution (7.5% w/w). The aqueous formulation of gelatin/mannitol/NaOH/PY was flash frozen in liquid nitrogen (-196°C), stored at -15°C for 12 hours, and lyophilized at 0°C for 12 hours under vacuum.

Reference Example 5a (placebo, matrix/excipients only) and Reference Example 5b (prepared by physical mixing (admixture of) PY + matrix/excipients) were also prepared and characterized for reference.

**Table 8.**

| Matrix composition | **Example 5** | **Reference Example 5a** | **Reference Example 5b*** |
|---|---|---|---|
| | wt. (g) | wt. (g) | wt. (g) |
| Gelatin | 0.0275 | 0.0275 | 0.0275 |
| NaOH | 0.0175 | 0.0175 | 0.0175 |
| Mannitol | 0.022 | 0.022 | 0.022 |
| Psilocybin | 0.01 | - | 0.01 |
| Water | 0.423 | 0.433 | 0.423 |
| Total Weight (g) | 0.50 | 0.50 | 0.50 |
| Dry Weight (g) | 0.077 | 0.067 | 0.077 |
| PY (g)/tablet | 0.01 | N/A | 0.01 |
| PY (wt.%) in dry 50 mg tablet | 13.0 | N/A | 13.0 |

| | | | |
|---|---|---|---|
| * Physical mixture (admixture) | | | |

### Example 6

Orally disintegrating tablets (ODT), also known as fast dissolving tablets (FDTs), were prepared according to the formulation of Example 5 in Table 8, but using the following procedure: A mixture of gelatin and mannitol was prepared in water and the solution was heated to 60°C for 10 min. The solution was cooled to 12°C followed by addition of crystalline psilocybin. The pH was modified using sodium hydroxide solution (7.5% w/w). The resulting aqueous formulation was dosed into blister pockets in an amount which provides 5 mg of psilocybin per tablet, and subjected to lyophilization by freezing at -90°C for 4 minutes. The frozen product was placed in a freezer (-14°C) for storage for ≥ 12 hours, and then dried in a freeze dryer under vacuum at a shelf temperature of 0°C for 12 hours.

### Examples 7-8

Freeze-dried tablets (50 mg) were prepared with psilocybin (PY; **I-7**) (26.67 wt.%) according to composition described in Table 9. KOLLIDON^{®} VA 64 (Example 7) or METHOCEL^{™} E6 premium LV (Example 8) were dissolved in water and incubated at 60°C for 30 to 60 min until a clear solution was obtained. Crystalline psilocybin was dissolved in water and was incubated at 60°C and vortexed vigorously. Both solutions were mixed, and the resulting aqueous formulation of polymer/PY was flash frozen in liquid nitrogen (-196°C), stored at -15°C for 12 hours, and lyophilized at 0°C for 12 hours under vacuum.

**Table 9.**

| Matrix composition | **Example 7** | **Example 8** |
|---|---|---|
| | wt. (g) | wt. (g) |
| KOLLIDON^{®} VA 64 | 0.0275 | - |
| METHOCEL^{™} E6 premium LV | - | 0.0275 |
| Psilocybin | 0.01 | 0.01 |
| Water | 0.462 | 0.462 |
| Total Weight (g) | 0.4995 | 0.4995 |
| Dry Weight (g) | 0.0375 | 0.0375 |
| PY (g)/tablet | 0.01 | 0.01 |
| PY (wt.%) in dry 50 mg tablet | 26.67 | 26.67 |

### Examples 9-14

Freeze-dried tablets (50 mg) were prepared with psilocybin (PY; **I-7**) (26.67 wt.%) according to composition described in Table 10. METHOCEL^{™} K100LV (Example 9), METHOCEL^{™} K4M (Example 10), METHOCEL^{™} K15M (Example 11), AQUASOLVE^{™} HPMCAS MF (Example 12), BENECEL^{™} K35M Pharm (Example 13), or BENECEL^{™} K100LV PH PRM (Example 14) were dissolved in water and incubated at 60°C for 30 to 60 min until a clear solution was obtained. Crystalline psilocybin was dissolved in water and was incubated at 60°C and vortexed vigorously. Both solutions were mixed, and the resulting aqueous formulation of polymer/PY was flash frozen in liquid nitrogen (-196°C), stored at -15°C for 12 hours, and lyophilized at 0°C for 12 hours under vacuum.

**Table 10.**

| Matrix composition | **Example 9** | **Example 10** | **Example 11** | **Example 12** | **Example 13** | **Example 14** |
|---|---|---|---|---|---|---|
| | wt. (g) | wt. (g) | wt. (g) | wt. (g) | wt. (g) | wt. (g) |
| METHOCEL^{™} K100LV | 0.0275 | - | - | - | - | - |
| METHOCEL^{™} K4M | - | 0.0275 | - | - | - | - |
| METHOCEL^{™} K15M | - | - | 0.0275 | - | - | - |
| AQUASOLVE^{™} HPMCAS MF | - | - | - | 0.0275 | - | - |
| BENECEL^{™} K35M | - | - | - | - | 0.0275 | - |
| BENECEL^{™} K100LV PH PRM | - | - | - | - | - | 0.0275 |
| Psilocybin | 0.01 | 0.01 | 0.01 | 0.01 | 0.01 | 0.01 |
| Water | 0.465 | 0.465 | 0.465 | 0.465 | 0.465 | 0.465 |
| Total Weight (g) | 0.5025 | 0.5025 | 0.5025 | 0.5025 | 0.5025 | 0.5025 |
| Dry Weight (g) | 0.0375 | 0.0375 | 0.0375 | 0.0375 | 0.0375 | 0.0375 |
| PY (g)/tablet | 0.01 | 0.01 | 0.01 | 0.01 | 0.01 | 0.01 |
| PY (wt.%) in dry 50 mg tablet | 26.67 | 26.67 | 26.67 | 26.67 | 26.67 | 26.67 |

### Example 15

Freeze dried wafers are prepared by dissolving crystalline psilocybin (20 mg) in 1 ml of a solution of 0.1 M phosphate buffer with 5 wt.% bovine gelatin and 4 wt.% mannitol, and adjusting the pH to 7.0 with sodium hydroxide solution (7.5% w/w). The aqueous formulation is transferred into a thin layer into wells, flash frozen in liquid nitrogen (-196°C), stored at -15°C for 12 hours, and lyophilized at 0°C for 12 hours under vacuum. After lyophilization, the occurrence of the amorphous form of PY is confirmed by DSC, TGA and X-ray powder diffraction. The material, in the form of a wafer, will be stored at 4°C and the stability pulls will be conducted at T=1, 3, 6, and 12 months confirming the presence of the amorphous form of PY.

The wafer displays good properties as an orally disintegrating wafer dosage form with a disintegration time of 25 s and very rapid dissolution kinetics, characterized by a dissolution time of 2 min.

### Characterization/results.

Freeze-dried solid dispersions of Examples 1-5, and corresponding Reference Examples, were characterized using X-ray powder diffraction (XRPD). The results are summarized in Table 11.

**Table 11**

| **Example No.** | **Descriptor** | **XRPD Results** |
|---|---|---|
| Crystalline PY starting material | commercially available from Quality Chemical Labs | crystalline PY (methanol solvate)^{a} may contain small amount of Form B^{b} |
| Example 1 | freeze-dried gelatin/PY | amorphous PY |
| Reference Example 1a | placebo | amorphous |
| Reference Example 1b | physical mixture (admix) | crystalline PY (methanol solvate)^{a} present |
| Example 2 | freeze-dried PVP/PY | crystalline PY Form A^{c} present |
| Reference Example 2a | placebo | amorphous |
| Reference Example 2b | physical mixture (admix) | crystalline PY (methanol solvate)^{a} present |
| Example 3 | freeze-dried HPMC/PY | amorphous PY |
| Reference Example 3a | placebo | amorphous |
| Reference Example 3b | physical mixture (admix) | crystalline PY (methanol solvate)^{a} present |
| Example 4 | freeze-dried HPMC/PVP/PY | amorphous PY |
| Reference Example 4a | placebo | amorphous |
| Reference Example 4b | physical mixture (admix) | crystalline PY (methanol solvate)^{a} present |
| Example 5 | freeze-dried gelatin/mannitol/NaOH/PY | amorphous PY + crystalline mannitol (hemihydrate + delta) |
| Reference Example 5a | placebo | crystalline mannitol (hemihydrate + delta) |
| Reference Example 5b | physical mixture (admix) | crystalline PY (methanol solvate)^{a} + mannitol (hemihydrate + delta) |

| | | |
|---|---|---|
| ^{a} CSD Reference Code PSILOC ^{b} CSD Reference Code TAVZID01 ^{c} CSD Reference Code HATCAK & TAVZID | | |

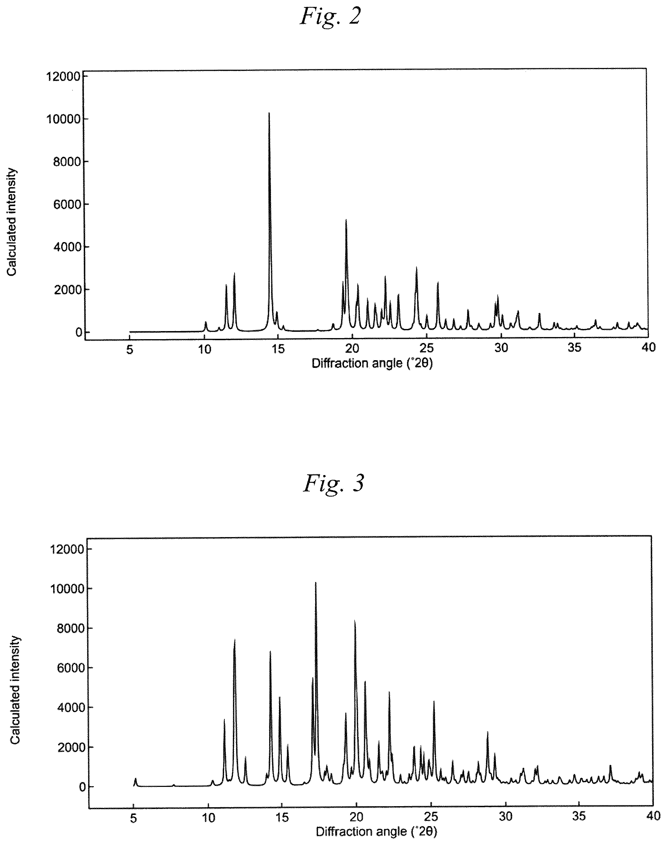

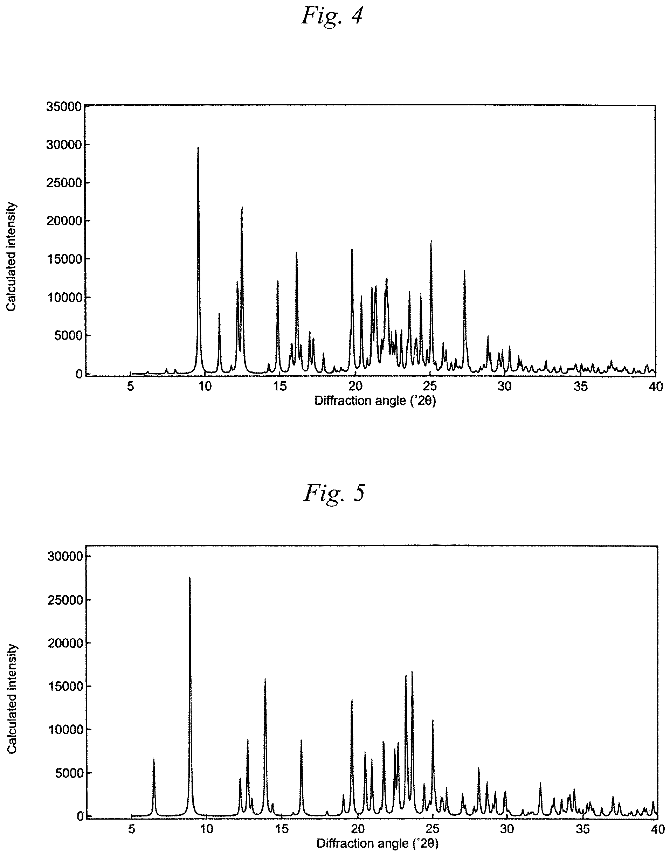

A total of five crystalline forms of psilocybin (PY; **I-7**) have been reported in the literature (with Cambridge structural database (CSD) Reference Codes identified in parentheses): Form A (HATCAK & TAVZID), Form B (TAVZID01), methanol solvate (PSILOC), trihydrate (OKOKAD), and ethanol solvate (KOWHOT), *see* Sherwood et al., "Psilocybin: crystal structure solutions enable phase analysis of prior art and recently patented examples", Acta Cryst. (2022), C78 (1), 36-55. The XRPD calculated from the CSD for Form A (HATCAK & TAVZID), Form B (TAVZID01), methanol solvate (PSILOC), and trihydrate (OKOKAD) are presented in Figs. 2-5, respectively.

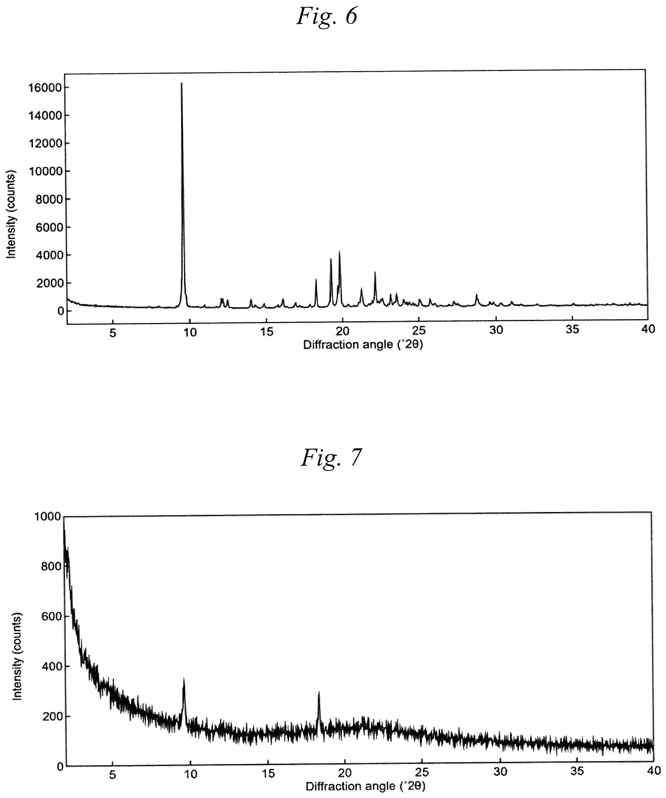

A comparison of XRPD results with the above reference psilocybin data from the Cambridge Structure Database (CSD) show that the crystalline psilocybin starting material used in these studies (commercially available from Quality Chemical Labs) is crystalline psilocybin methanol solvate with a small quantity of Form B (Fig. 6). Some preferred orientation is present and some of the intensities do not match the reference.

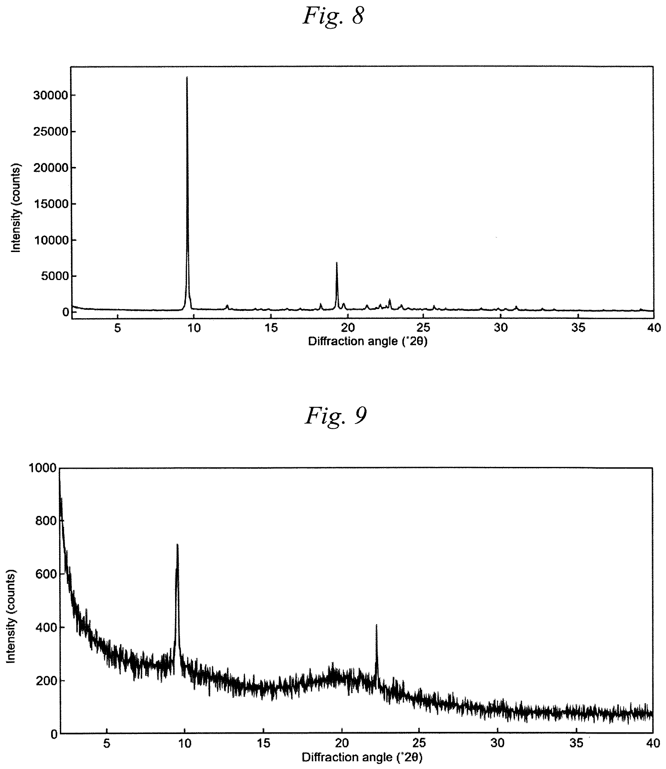

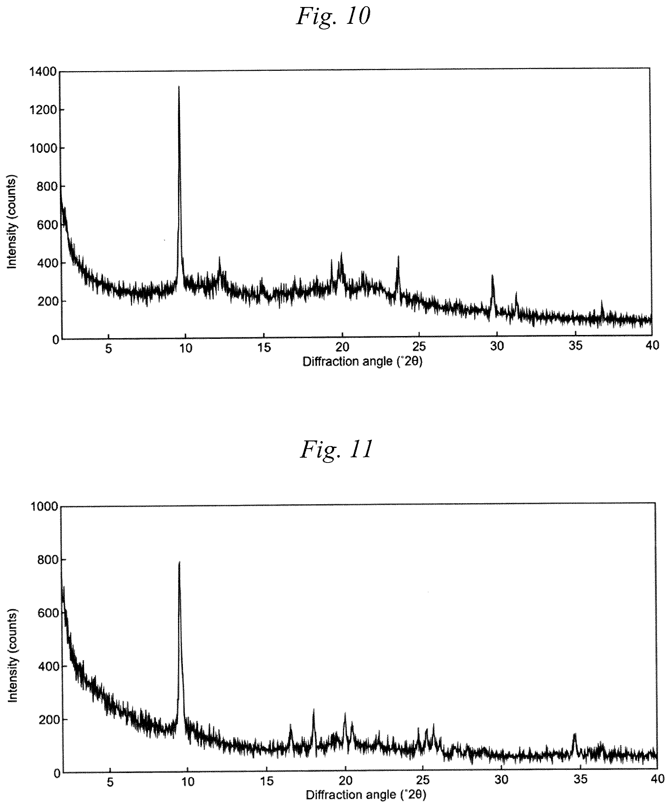

XRPD data showed that Reference Examples 1b, 2b, 3b, 4b, and 5b (physical mixtures (admixtures)) contain small amounts of crystalline psilocybin methanol solvate (Figs. 7-11, respectively). Based on the peak broadening of crystalline psilocybin, psilocybin methanol solvate may be losing crystallinity upon mechanical mixing applied during the mixture preparations. XRPD data for Reference Example 5b also showed crystalline mannitol (mannitol hemihydrate and mannitol delta form) as shown in Fig. 11.

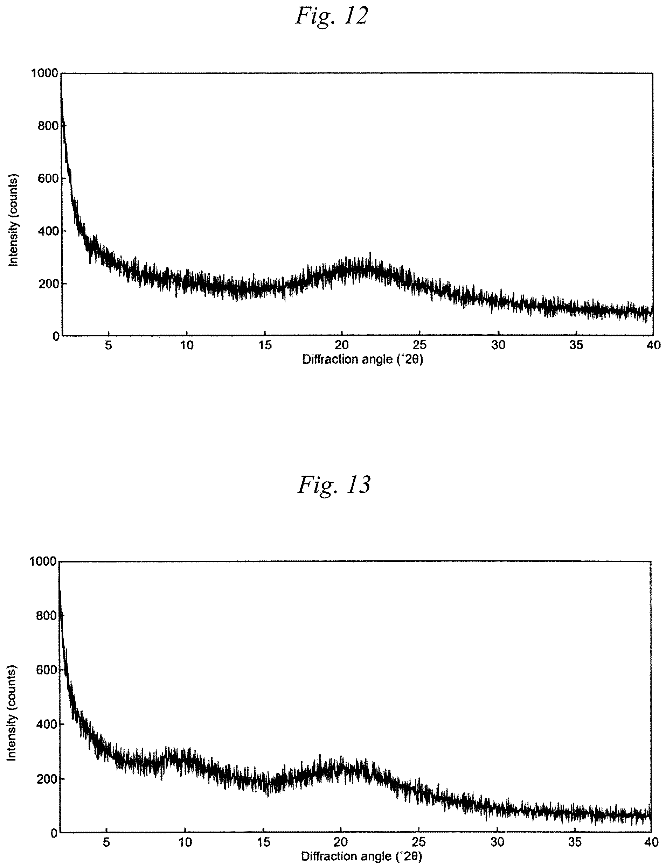

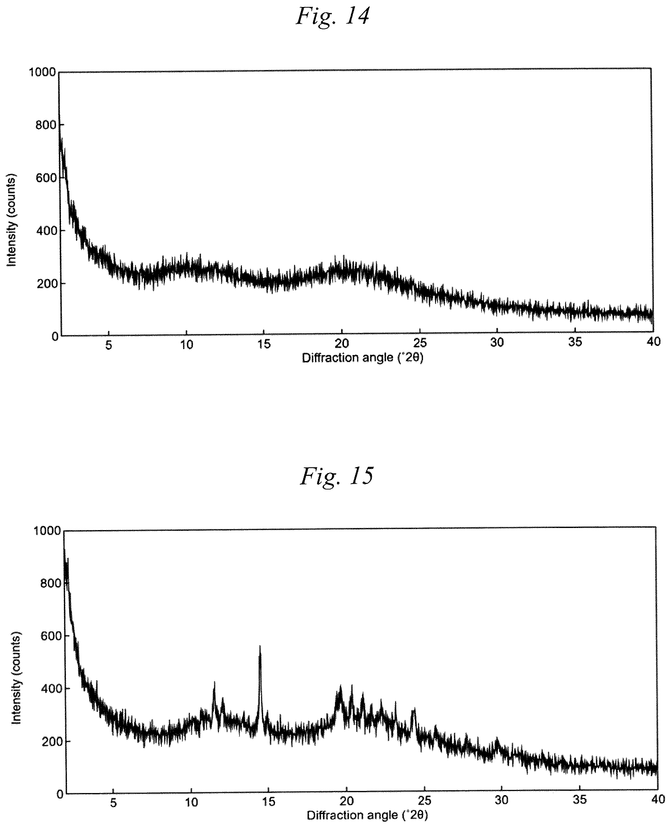

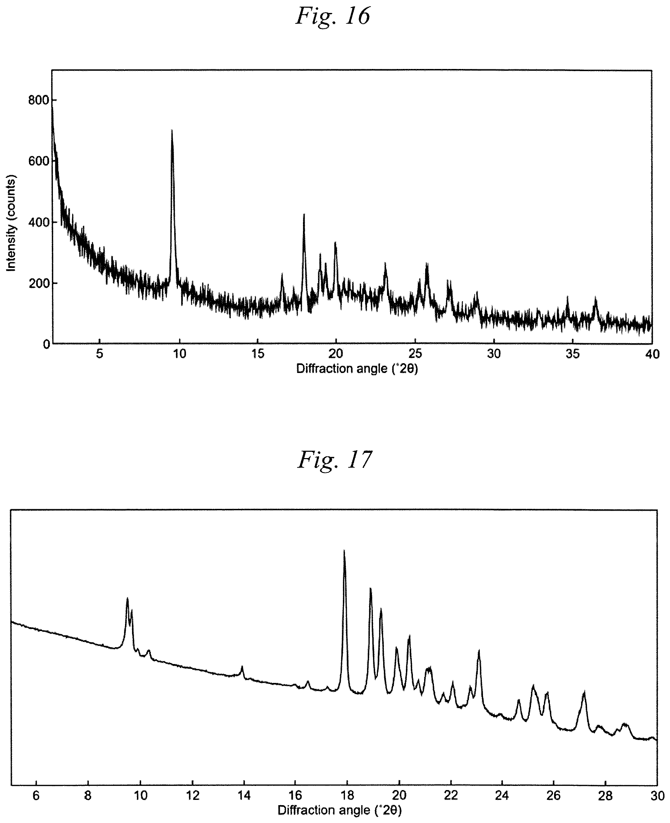

XRPD data for Examples 1, 3, and 4 indicated these solid dispersions were amorphous with no visible crystalline psilocybin peaks (Figs. 12-14, respectively). To the contrary, XRPD of Example 2 showed peaks matching psilocybin Form A (Fig. 15), indicating that PVP alone was not sufficient for stabilizing amorphous psilocybin. XRPD pattern of Example 5 showed only crystalline mannitol (mannitol hemihydrate and mannitol delta form) as shown in Fig. 16.

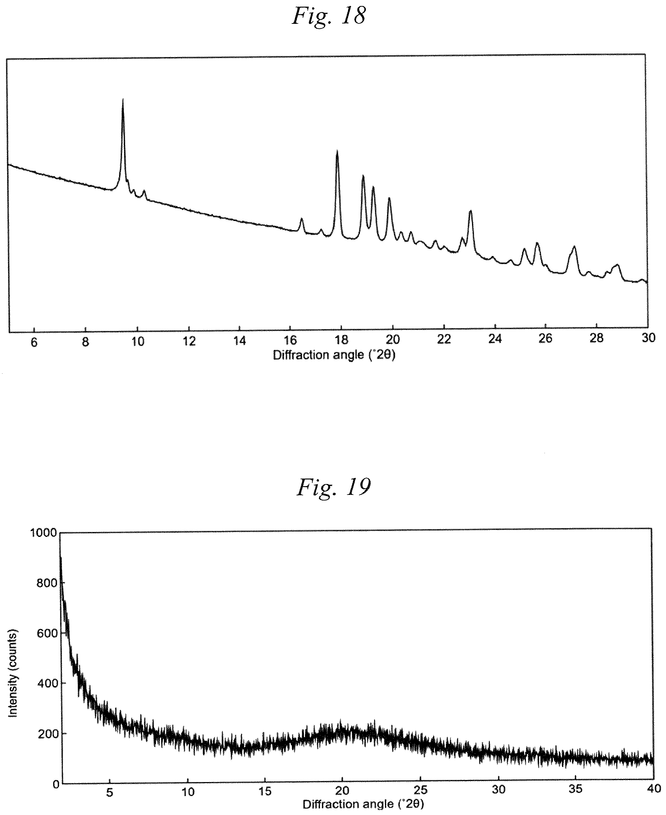

To improve the signal to noise and to attempt to detect even small levels of crystalline psilocybin present in freeze-dried formulations, high-resolution XRPD analysis was performed in transmission configuration on Reference Example 5b and Example 5. The XRPD data showed that the physical mixture (Reference Example 5b) contained crystalline psilocybin methanol solvate (Fig. 17), whereas the freeze-dried product (Example 5) did not show signals corresponding to crystalline psilocybin, only those of amorphous psilocybin (Fig. 18). XRPD patterns of both samples showed crystalline excipients consisting of two crystalline forms of mannitol (hemihydrate and delta forms). It is noted that the crystalline mannitol signals overlap with some of the intense signals of crystalline psilocybin, which somewhat complicated the analysis.

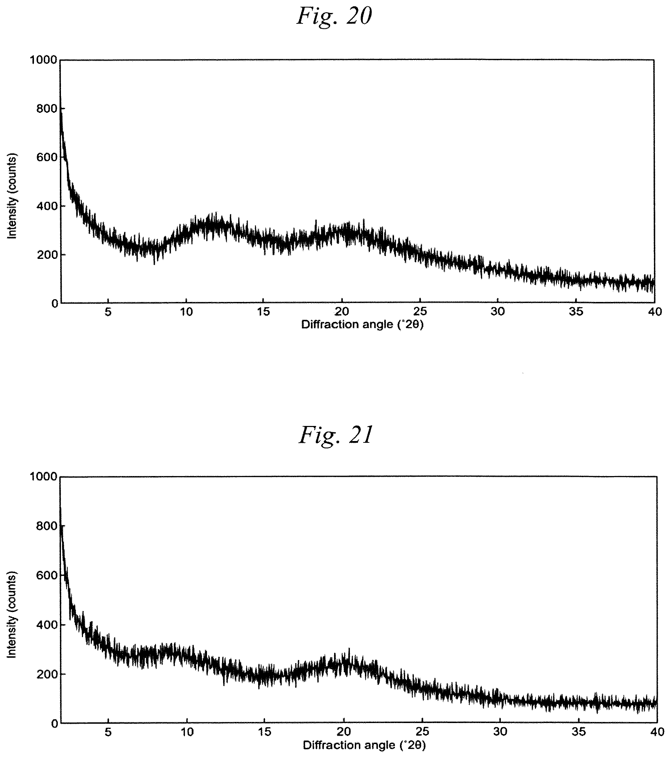

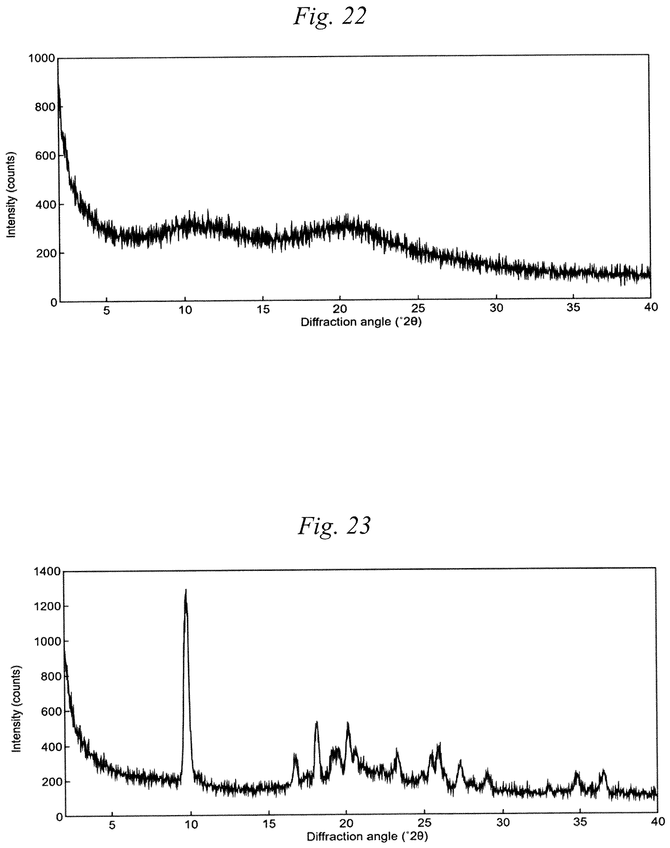

For comparison, XRPD was also performed on Reference Examples 1a-5a (placebo products), which are shown in Figs. 19-23, respectively. All placebo products showed only amorphous material, except for Reference Example 5b, which showed crystalline mannitol, again in both hemihydrate and delta forms (Fig. 23).

In summary, XRPD analysis confirmed that psilocybin starting material used in these studies is a crystalline methanol solvate. XRPD analysis detected psilocybin crystalline Form A in Example 2, however no other crystalline psilocybin products were detected in Examples 1 and 3-5-these examples are characterized as amorphous solid dispersions.

Crystalline psilocybin starting material and freeze-dried solid dispersions of Examples 1 and 3-5 were also analyzed by mDSC, and the results are summarized in Table 12.

**Table 12**

| **Example No.** | **Descriptor** | **mDSC Results** |
|---|---|---|
| Crystalline PY starting material | commercially available from Quality Chemical Labs | Apparent melt onset (methanol solvate) 129°C |
| Example 1 | freeze-dried gelatin/PY | Tg onset 183°C, Tg midpoint 187°C, ΔCp 0.44 J/(g•°C) |
| Example 3 | freeze-dried HPMC/PY | Tg onset 144°C, Tg midpoint 149°C, ΔCp 0.26 J/(g•°C) |
| Example 4 | freeze-dried HPMC/PVP/PY | Tg onset 119°C, Tg midpoint 127°C, ΔCp 0.16 J/(g•°C) |
| Example 5 | freeze-dried gelatin/mannitol/NaOH/PY | Tg onset 126°C, Tg midpoint 129°C, ΔCp 0.73 J/(g•°C) |

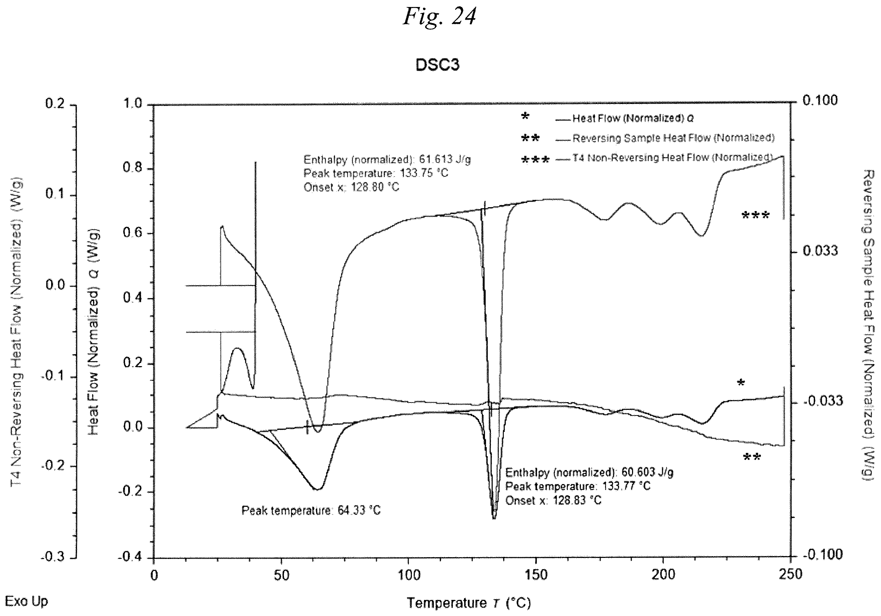

Crystalline psilocybin starting material showed a loss of the solvent near 65°C and an apparent melting at 129°C (onset temperature)(Fig. 24). Melting point of crystalline psilocybin Form A was reported at 212°C, with a small endothermic transition at 149°C. Tg of amorphous psilocybin has been reported at 163°C (midpoint). *See* Greenan et al., Preparation and Characterization of Novel Crystalline solvates and Polymorphs of Psilocybin and Identification of Solid Forms Suitable for Clinical Development, 2020 pre-publication; DOI:10.13140/RG.2.2.32357.14560.

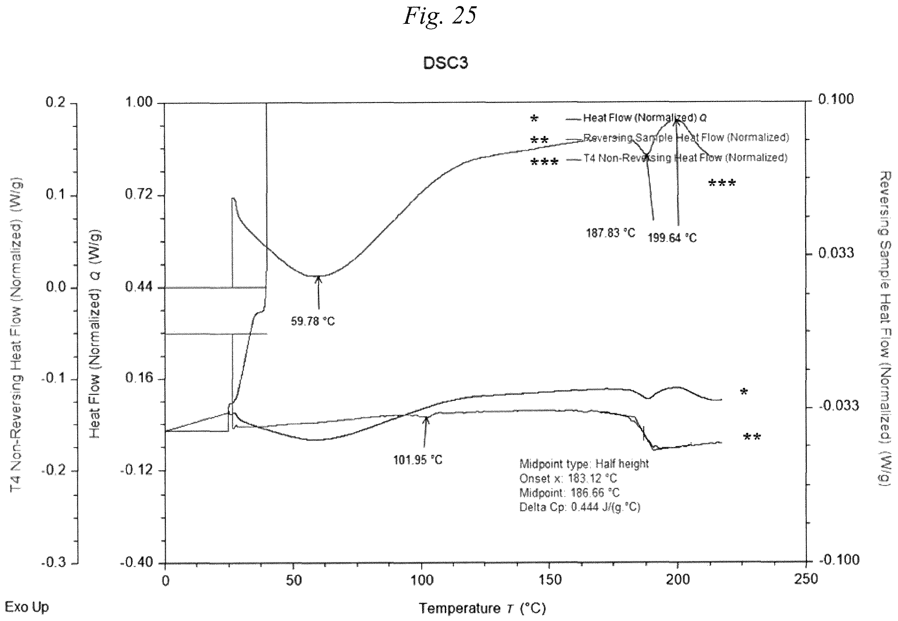

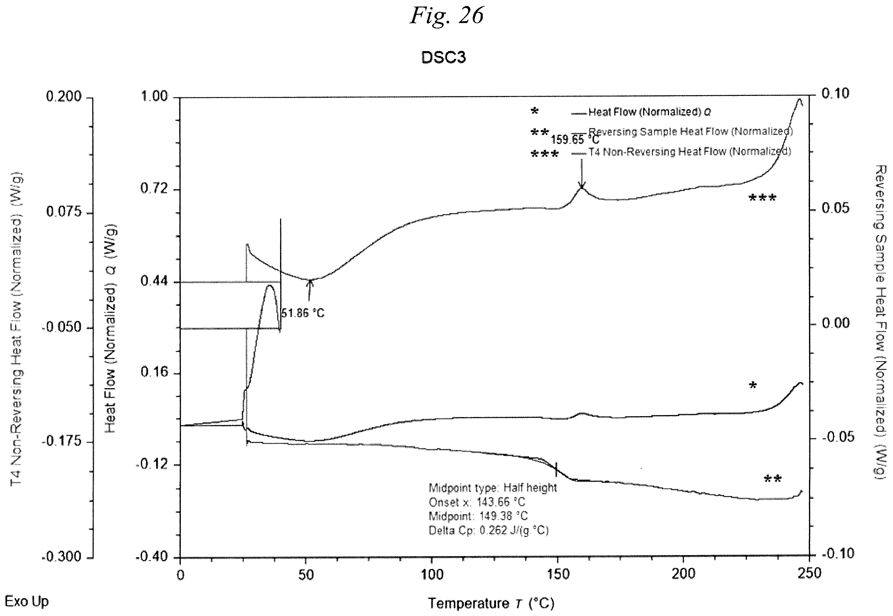

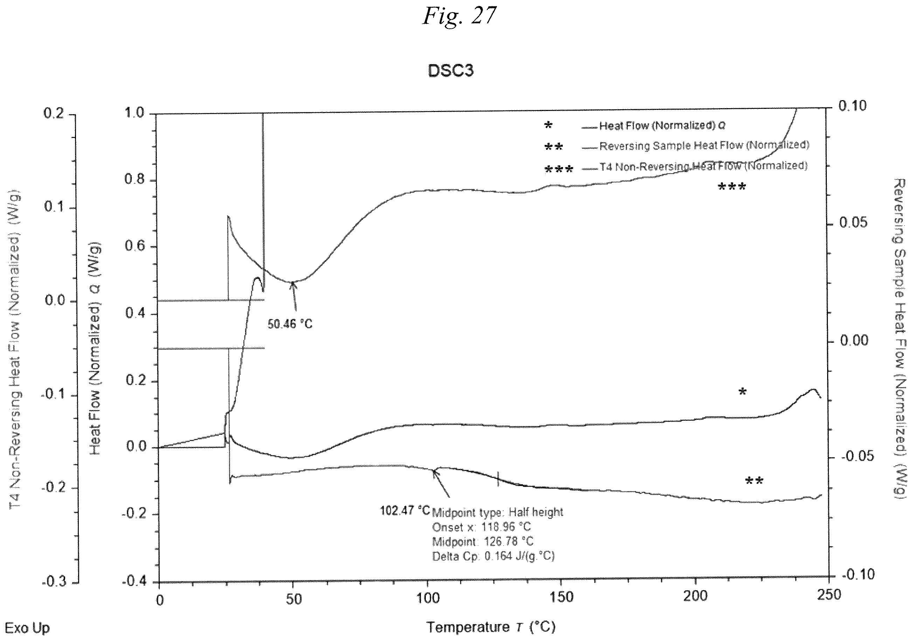

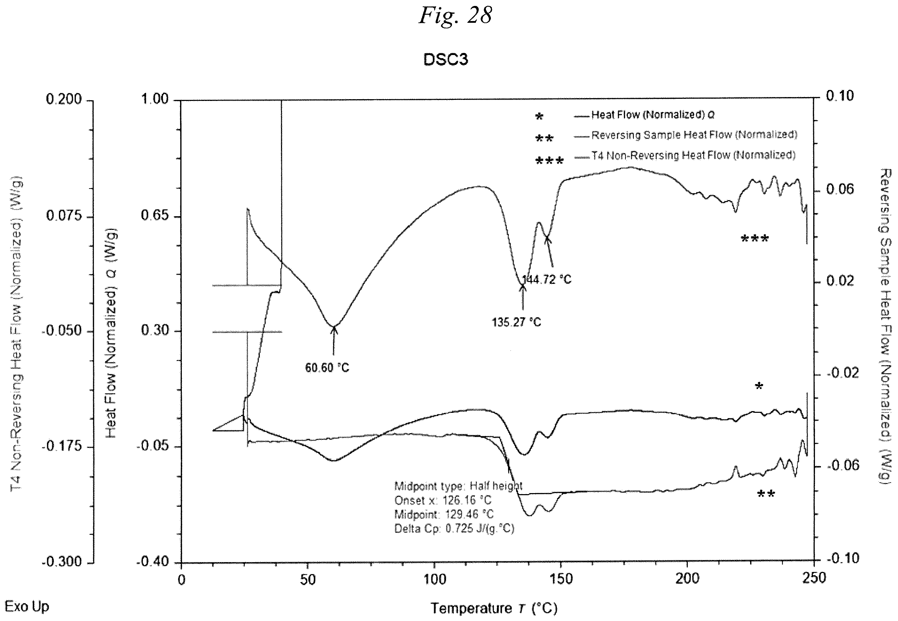

Examples 1 and 3-5 all showed broad endotherms between 50-60°C. These broad endotherms are most likely due to volatile loss. Further, these products also showed glass transition (Tg) events, ranging between 119 and 183°C as shown on the reversing heat flow in the mDSC data (Figs. 25-28, respectively), and also summarized in Table 12).

Example 1 exhibited the highest Tg at 183°C (onset temperature) with ΔCp (heat capacity change) of 0.44 J/(g•°C). Typical ΔCp for amorphous material is 0.5 J/(g•°C). The high Tg and the value of ΔCp for Example 1 suggest the amorphous material would be physically stable. The nature of the small endotherm at 102°C is unknown and a similar endotherm is also observed in Example 4. The non-reversing heat flow in Example 1 shows an endotherm at 188°C which is an enthalpic relaxation corresponding to the glass transition event. The exothermic event at 200°C in the non-reversing heat flow can be recrystallization or chemical reaction(s). A similar exothermic event immediately following the glass transition is also observed in Example 3. On the other hand, Example 4 exhibited the lowest Tg and lowest ΔCp.

The glass transition event of Example 5 overlaps with multiple events (probably due to excipients) and therefore the unusually high ΔCp value (0.73 J/(g•°C)) may not be accurate.

In summary, mDSC analysis of four freeze-dried solid dispersions (Examples 1 and 3-5) showed that while all products exhibited glass transition temperatures, Example 1 exhibited the highest glass transition temperature.

### Dissolution/Release Studies.

The percentage of dissolution/release of PY of Examples 1, 3-5, and 7-12 were measured using a standard curve in (i) 1x phosphate buffered saline (PBS)(pH 7.4) and (ii) 0.1 N citric acid (CA)(pH 1.2).

Diluent solution: 1M citric acid: H₂O: acetonitrile (ACN) = 1:8:1. To prepare 1 liter, 100 mL of ACN was mixed with 800 mL of water and 100 mL of 1.0 M citric acid and mixed well.

Citric acid solution preparation: 1 M citric acid was prepared by dissolving 1.92 g citric acid in 10 mL of water. 0.1 N citric acid was prepared by dissolving 0.96 g citric acid in 50 ml water.

Reference standard solution for linearity curve preparation. Preparation of psilocybin (PY) reference standard solution was performed by diluting stock solution (1000 µg/ml) with diluent solution to 333.3, 200, 50, 10, 1 and 0.1 µg/ml in diluent solution. Table 13 shows the preparation of linearity curve of reference standard solution.

**Table 13**

| **Target Concentration µg/mL** | **Initial concentration µg/ml** | **Initial concentration volume (µl)** | **Diluent (µl)** |
|---|---|---|---|
| 200 | 1000 | 200 | 800 |
| 100 | 200 | 400 | 400 |
| 50 | 200 | 200 | 600 |
| 10 | 50 | 200 | 800 |
| 1 | 10 | 50 | 450 |
| 0.1 | 1 | 50 | 450 |

Freeze-dried samples (10 mg psilocybin) were charged into appropriately sized beakers or flasks, and then poured into 0.1 N citric acid or PBS in scintillation vials equipped with a stir bar, and the contents were stirred at 250 rpm at room temperature. Time course sampling at 1, 5 and 10 min with each solution with 300 µL of diluent was performed, whereby samples were diluted to 50% with 300 µL of diluent (50% 0.1% N citric acid/ACN) in each timepoint, centrifuged to separate solid/liquid, and 200-300 µL of the upper layer was taken up in an HPLC vial for dissolution kinetics testing using the following chromatographic conditions *(see* Table 14). For freeze-dried samples with 10 mg active per sample in the dissolution test, the target concentration was 200 µg/ml when completely dissolved.

### Chromatographic Conditions:

| | | |
|---|---|---|
| Column | Stationary Phase | ZorbaxSB 18 3.5 mm |
| | Material/Dimensions | Stainless steel, 4.6 x 150 mm |
| | Mobile Phase A | 0.1 % TFA in Water |
| | Mobile Phase B | 0.1 % TFA in ACN |

**Table 14**

| **Time (min)** | **%A** | **%B** |
|---|---|---|
| 0 | 95 | 5 |
| 1 | 95 | 5 |
| 14 | 60 | 40 |
| 15 | 5 | 95 |
| 18 | 5 | 95 |
| 18.1 | 95 | 5 |
| 20 | 95 | 5 |

| | |
|---|---|
| Flow rate | 1.0 mL/min |
| Column Temp | 30 °C |
| Injection Vol | 10 µL |
| Needle wash | Diluent |
| Detection wavelength | 267 nm |
| Run Time | 20 min |

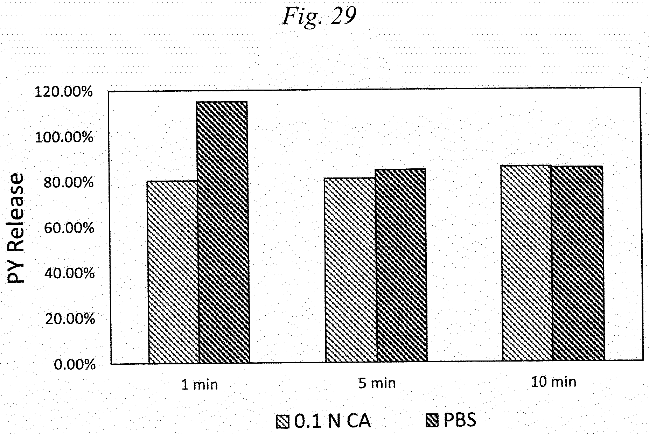

Results. As can be seen in Fig. 29, Example 1 exhibited a similar release rate of PY in both acidic (0.1 N citric acid) and neutral (PBS) pH. A burst release of up to 80% was attained in 1 minute and remained consistent through 10 minutes, characteristic of an immediate release dosage form.

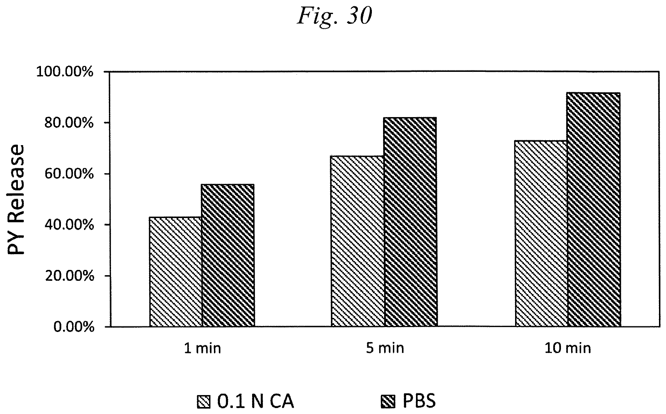

For Example 3, the release of PY was time dependent in both acidic (0.1 N citric acid) and neutral (PBS) conditions. PY release was higher/faster in PBS (100% at 10 min) than in citric acid buffer (70% at 10 min)(Fig. 30). Example 3 can be characterized as a fast release dosage form.

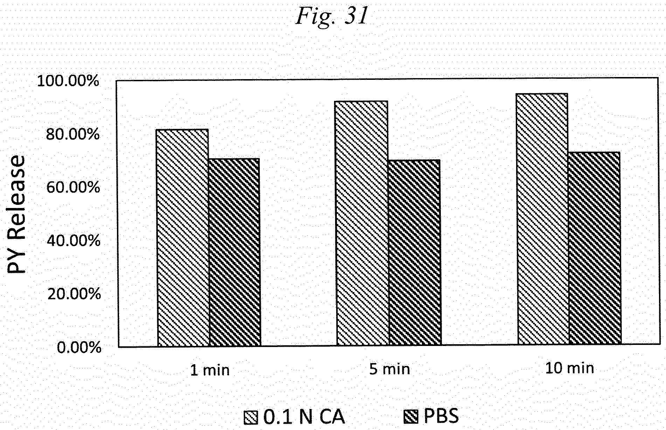

Contrary to Example 3, PY release in Example 4 was higher in acidic pH (95% at 10 min in 0.1 N citric acid) than it was in neutral pH (70% at 10 min in PBS)(Fig. 31). The release kinetics were characteristic of an immediate release dosage form.

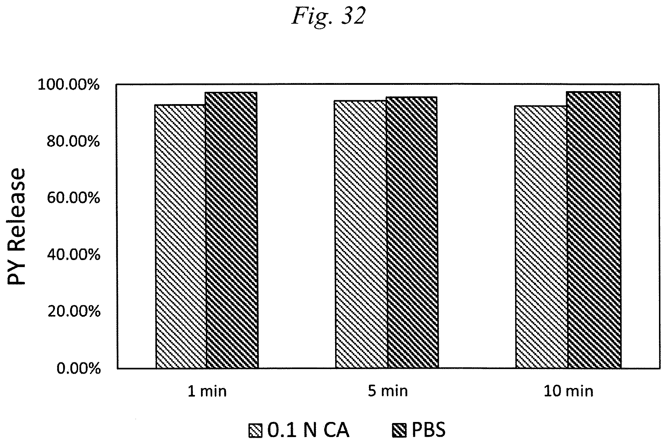

Example 5 exhibited no preference for pH for PY release and demonstrated maximum drug release (95-100%) within 1 minute for both acidic (0.1 N citric acid) and neutral (PBS) conditions (Fig. 32), characteristic of an immediate release dosage form.

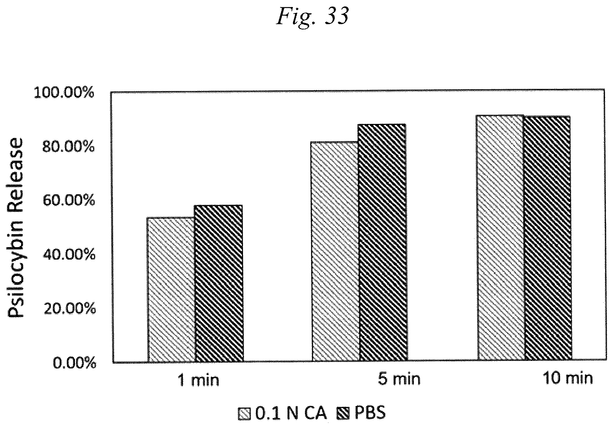

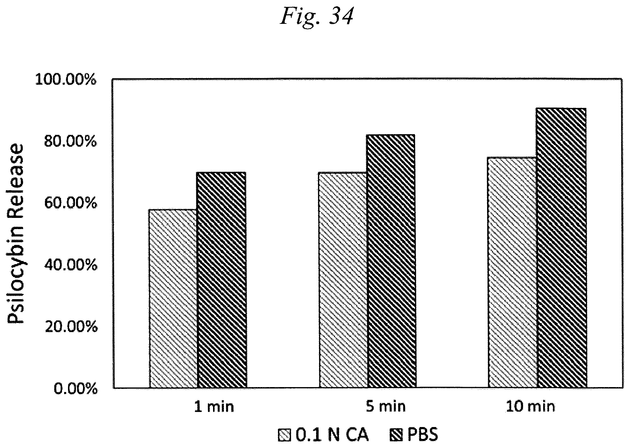

Examples 7 and 8 both exhibited similar release kinetics, despite their different matrix compositions, as can be seen in Figs. 33 and 34, respectively. PY release for both was slightly higher/faster in PBS than in citric acid buffer. Both polymer matrices have a similar molecular weight range and provided similar release kinetics-a fast release profile with about 90% release within 10 minutes.

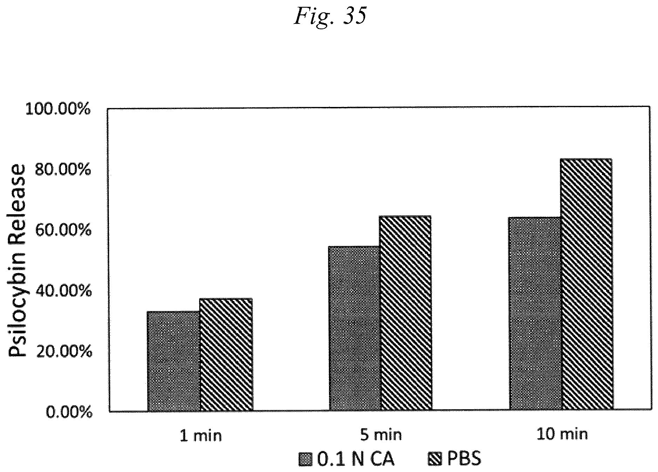

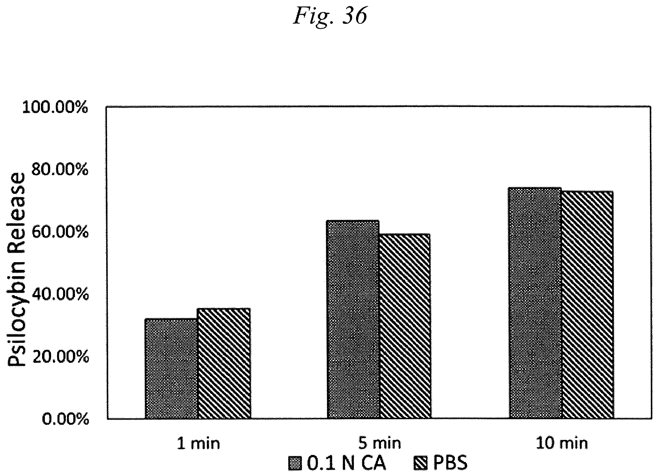

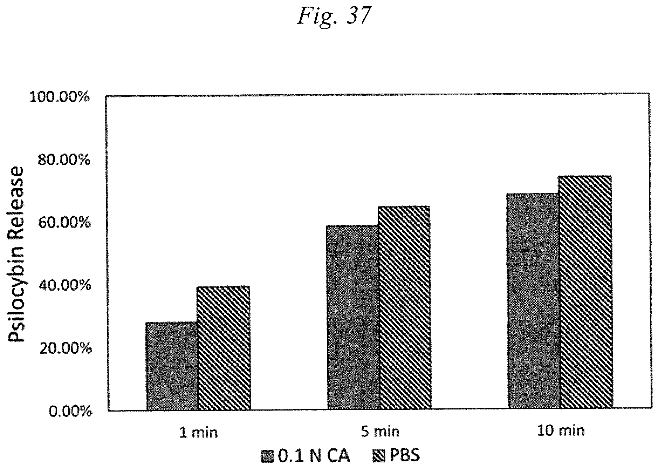

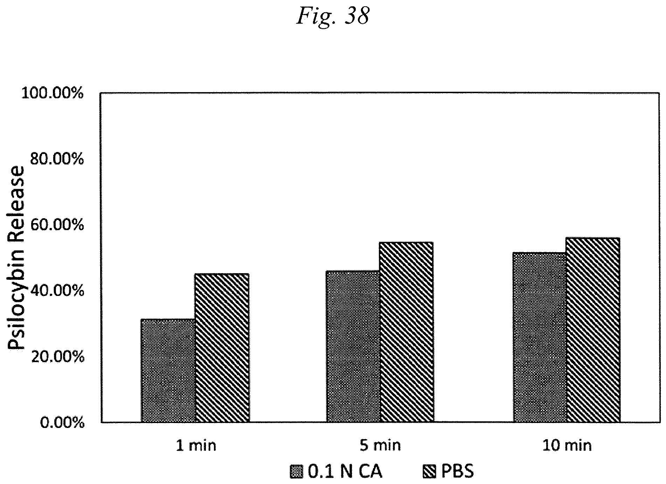

Examples 9-12 all exhibited extended-release kinetics, as can be seen in Figs. 35-38, respectively. PY release in Example 9 was slightly higher/faster in PBS than in citric acid buffer, reaching 80%+ at 10 minutes. PY release was slowed in Examples 10 and 11 (made using HPMC with a molecular weight range of 400-600 kDa), providing less than about 80% release by the 10-minute timepoint. Example 12 prepared using HPMCAS used as enteric coating in extended-release applications exhibited an even slower release profile of about 50% at 5 minutes, which remained nearly the same up to the 10-minute timepoint.

*Stability Studies.* Freeze-dried solid dispersions of Examples 1 and 3-5 (in the form of lyophilized cakes) were stored under the following conditions: (i) 40°C, 75% relative humidity (RH); (ii) 40°C, 15% RH; or (iii) room temperature (r.t., about 20-22°C). After storage for 27 days for Examples 1, 3, and 4 or 24 days for Example 5, the samples were analyzed by high-resolution XRPD to determine whether crystalline psilocybin was present.

The stability results are summarized in Table 15.

**Table 15**

| **Example No.** | **Storage conditions^{a}** | **High-resolution XRPD Results** | |
|---|---|---|---|
| | | Crystalline PY | Excipient(s) |
| 1 | (i) 40°C, 75% RH | Trihydrate^{b} | Not detected |
| | (ii) 40°C, 15% RH | Form A^{c} | Not detected |
| | (iii) r.t. | Form A^{c} (trace) | Not detected |
| 3 | (i) 40°C, 75% RH | Trihydrate^{b} | Not detected |
| | (ii) 40°C, 15% RH | Trihydrate^{b} | Not detected |
| | (iii) r.t. | Trihydrate^{b} | Not detected |
| 4 | (i) 40°C, 75% RH | Trihydrate^{b} | Not detected |
| | (ii) 40°C, 15% RH | Form A^{c} | Not detected |
| | (iii) r.t. | Form A^{c} (trace) | Not detected |
| 5 | (i) 40°C, 75% RH | Not detected | crystalline mannitol (delta + alpha) |
| | (ii) 40°C, 15% RH | Trihydrate | crystalline mannitol (delta + beta + alpha) |
| | (iii) r.t. | Not detected | crystalline mannitol (hemihydrate + delta) |

| | | | |
|---|---|---|---|
| ^{a} Samples were stored for 27 days under the listed conditions, except for those of Example 5, which were stored for 24 days ^{b} CSD Reference Code OKOKAD ^{c} CSD Reference Code HATCAK & TAVZID | | | |

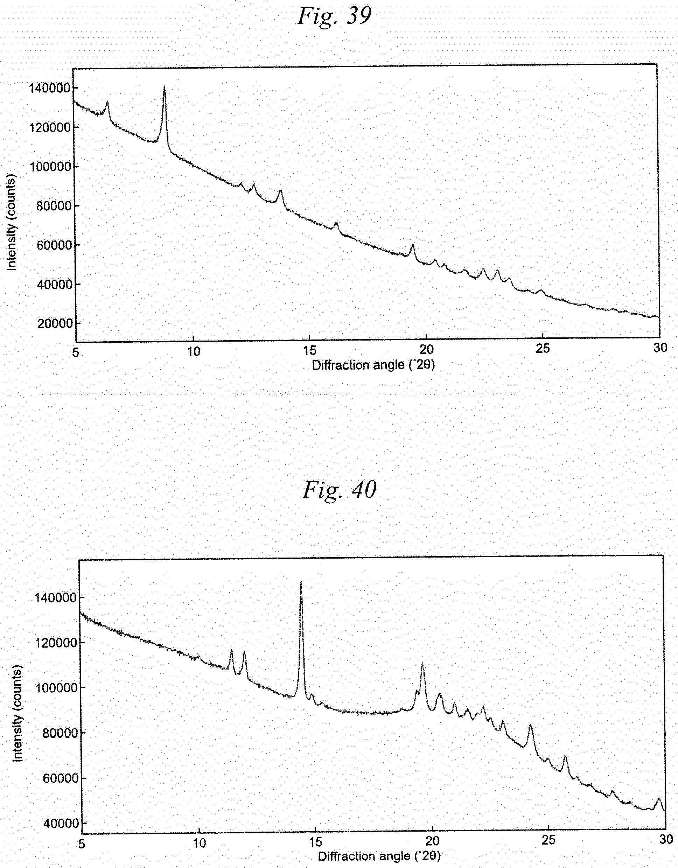

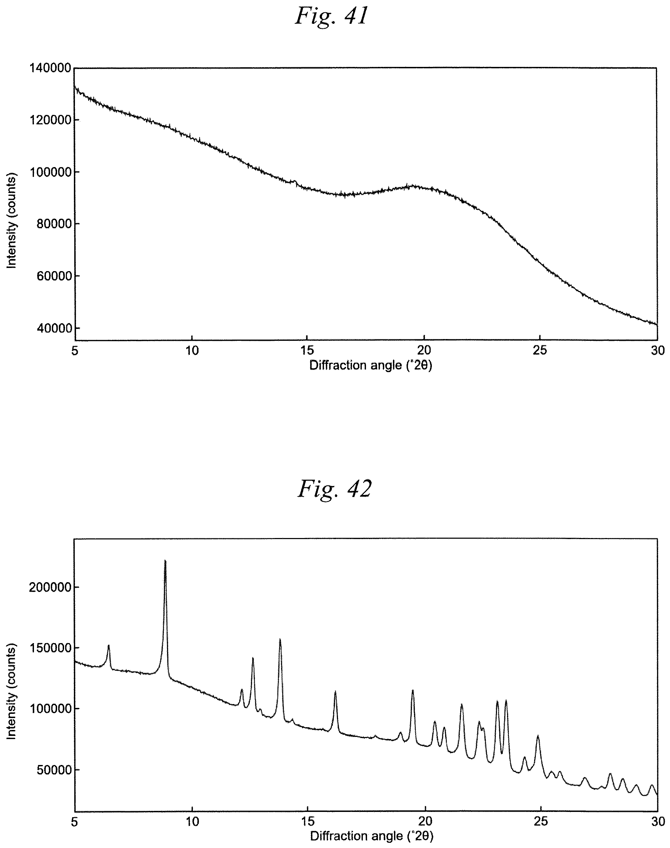

The presence of some crystalline PY was detected in Example 1 after 27 days of storage under stress conditions of (i) 40°C, 75% RH and (ii) 40°C, 15% RH (Figs. 39 and 40, respectively). The room temperature sample on the other hand displayed only a trace of a peak at 14.5 °2θ that was consistent with the strongest peak in the reference pattern of psilocybin Form A, but was otherwise consistent with amorphous material (Fig. 41).

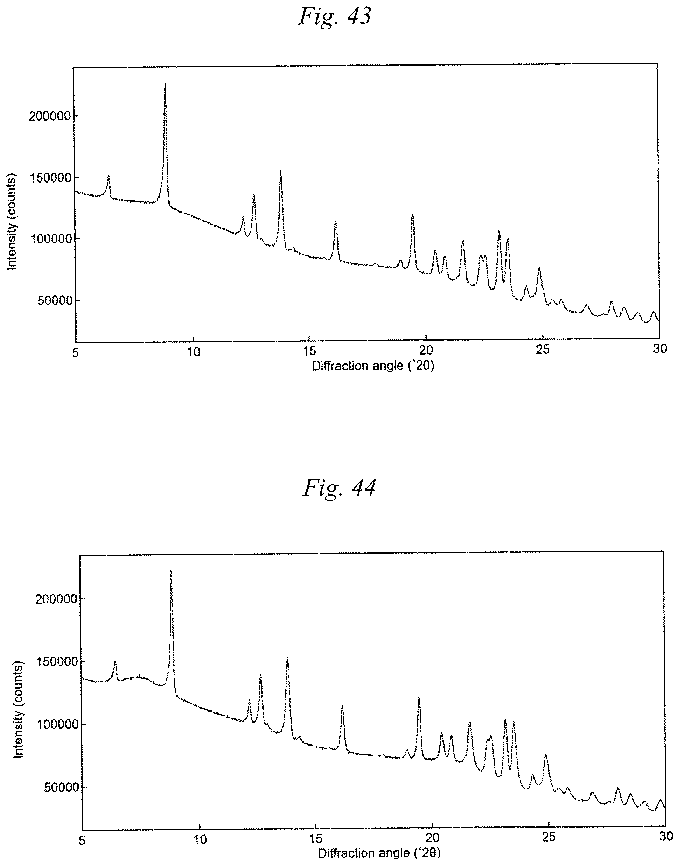

Crystalline PY (trihydrate) was detected in Example 3 after 27 days of storage under the storage conditions of (i) 40°C, 75% RH; (ii) 40°C, 15% RH; and (iii) room temperature (Figs. 42-44, respectively), indicating that Example 3 was less stable to recrystallization under the tested stress conditions in comparison to other samples analyzed.

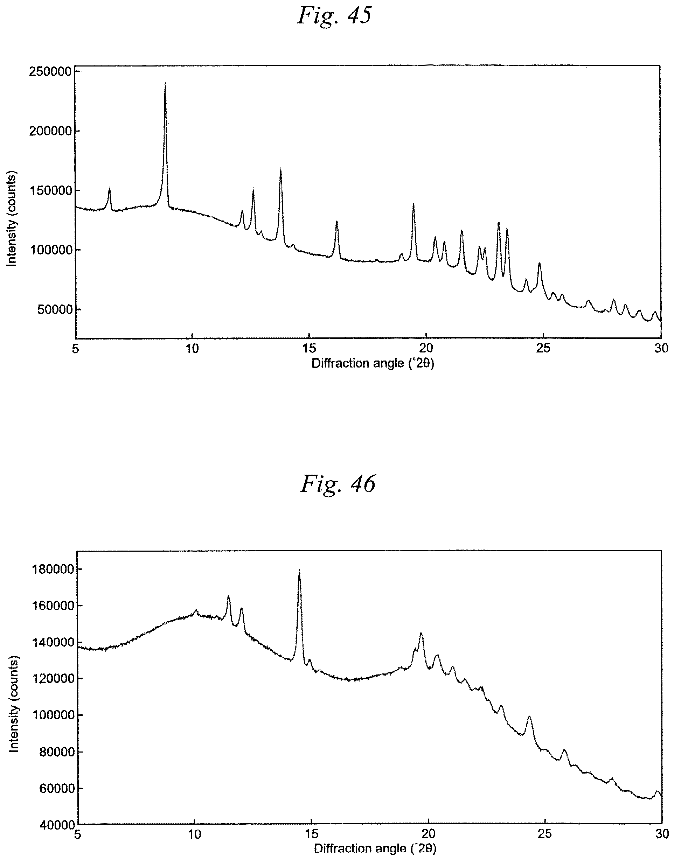

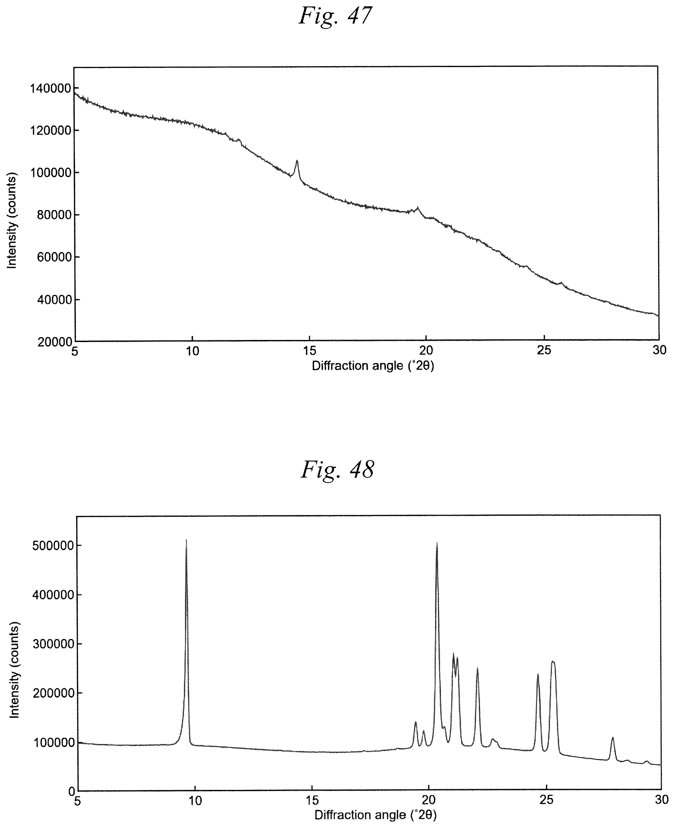

The presence of some crystalline PY was detected in Example 4 after 27 days of storage under stress conditions of (i) 40°C, 75% RH and (ii) 40°C, 15% RH (Figs. 45 and 46, respectively). However, only trace crystalline PY was detected in the room temperature sample, indicating suitable stability under such storage conditions (Fig. 47).

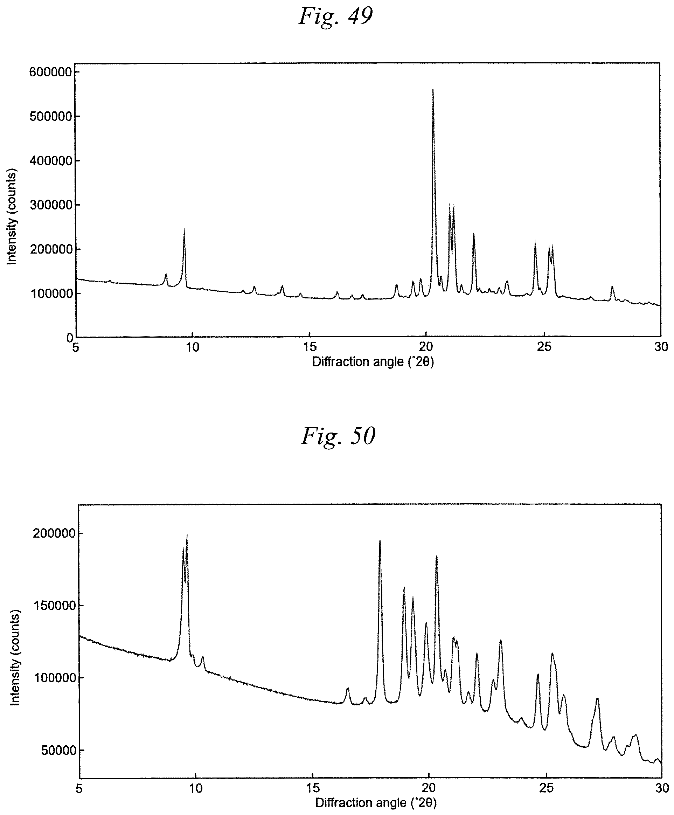

With respect to Example 5, crystalline PY (trihydrate) was detected only in the sample subjected to (ii) 40°C, 15% RH for 24 days (Fig. 49). The samples subjected to (i) 40°C, 75% RH and (iii) room temperature for 24 days showed no signs of crystalline PY, only crystalline mannitol excipient was detected (Figs. 48 and 50, respectively).

Overall, these results indicate that room temperature conditions are more favorable storage conditions to prevent psilocybin recrystallization than higher temperature/high relative humidity conditions, which is not surprising given that freeze-dried pharmaceutical dosage forms are known to be sensitive to water.

### III. Solid dispersions prepared by spray drying

### Materials.

EUDRAGIT^{®} E PO (EPO; a cationic low viscosity terpolymer based on N,N-dimethylaminoethyl methacrylate-methylmethacrylate-butylmethacrylate; 2:1:1; weight average molecular weight of about 47,000 g/mol; immediate release; soluble below and permeable above pH 5.0; available from Evonik).

KOLLIDON^{®} 30 (also called PVP K-30, amorphous, water-soluble polyvinylpyrrolidone with a weight average molecular weight of 44,000 - 54,000 g/mol; available from BASF.

KOLLIDON^{®} VA 64 (a 60:40 copolymer of VP:VAc, 45,000-75,000 g/mol, available from BASF).

PHARMACOAT^{®} 606 (HPMC with a 2910 substitution type: 28-30% methoxy substitution, 7-12% hydroxypropyl substitution; viscosity of 6.0 mPa·s as 2% solution in water at 20°C), available from Shin-Etsu Chemical Co. Ltd.

AQOAT^{®} AS-MG (HPMCAS with 9% acetyl, 11% succinoyl; 1,000 µm mean particle size; dissolution pH≥6.0), available from Shin-Etsu Chemical Co. Ltd.

EUDRAGIT^{®} L 100-55 (an anionic 1:1 copolymer of methacrylic acid-ethyl acrylate; delayed release; dissolution above pH 5.5; available from Evonik).

Psilocybin (3-(2-(dimethylamino)ethyl)-1H-indol-4-yl dihydrogen phosphate; PY; **1-7)** starting material used was in crystalline form as polymorph Form A, commercially available from Biosynth Carbosynth.

### Analytical methods.

X-ray power diffraction (XRPD): XRPD patterns were collected on a Bruker AXS D2 diffractometer using Cu Kα radiation (30 kV, 10 mA), θ-θ geometry, using a LynxEye detector from 5-42 °2θ.

The software used for data collection was DIFFRAC.SUITE and the data were analyzed and presented using Diffrac Plus EVA v 16.0.0.0.

The details of the data collection are:
- Angular range: 5 to 42 °2θ
- Step size: 0.024 °2θ
- Collection time: 0.1 seconds per step.

Samples were run under ambient conditions and prepared as flat plate specimens using powder without grinding. Approximately 1-2 mg of the sample was lightly pressed on a silicon wafer to obtain a flat surface.

Modulated differential scanning calorimetry (mDSC): TOPEM^{®} is a temperature modulated DSC method which differs from conventional DSC in allowing the total heat flow to be separated into reversing and non-reversing heat flow components. Such techniques help distinguish between processes or transitions that overlap or lie very close to one another. The glass transition temperature (Tg) was determined from the reversible heatflow component.

TOPEM^{®} DSC data were collected on a Mettler DSC 3+ equipped with a 34 position auto-sampler. The instrument was calibrated for energy and temperature using certified indium. Typically, 3-5 mg of each sample, in a pin-holed aluminium pan, was heated at 2 K·min⁻¹ from 30°C to 220°C with a pulse height of 0.64 K and a pulse width 15-30 sec. A nitrogen purge at 50 mL·min⁻¹ was maintained over the sample. STARe v15.00 was used for instrument control and data processing.

Predicted Tg: A single glass transition (Tg) by DSC is often an indicator of miscibility. Tg predictions were made using the Fox equation (1) which assumes densities are equal. $1 / {T}_{gmix} = {w}_{1} / {T}_{g 1} + {w}_{2} / {T}_{g 2} \left(w=weight\right)$

Determining the Tg is valuable for stability indication as above the Tg the material is prone to crystallization due to molecular motion. Amorphous solid dispersions (ASDs) can take up water, which acts as a plasticiser, and which will reduce the Tg. If the Tg is less than 90°C there is a significant chance for water uptake, which could cause the Tg to drop to below 25°C where recrystallization is likely to occur.

Thermogravimetric Analysis (TGA): TGA data were collected on a Mettler TGA 2 equipped with a 34 position auto-sampler. The instrument was temperature calibrated using certified isatherm and nickel. Typically, 5-30 mg of each sample was loaded into a pin-holed aluminium pan and heated at 10 °C·min⁻¹ from 30°C to 400°C. A nitrogen purge at 50 mL·min⁻¹ was maintained over the sample. STARe v15.00 was used for instrument control and data processing.

*Miscibility assessment.* The miscibility of psilocybin with a range of polymers was first assessed to determine which polymers were suitable for the preparation of amorphous solid dispersions through spray drying preparation methods. Physical mixtures (admixtures) of psilocybin with 6 different polymers (EUDRAGIT^{®} E PO, KOLLIDON^{®} 30, KOLLIDON^{®} VA 64, PHARMACOAT^{®} 606, AQOAT^{®} AS-MG, and EUDRAGIT^{®} L 100-55) were prepared at five different drug loadings (nominally 10, 25, 50, 75 and 90% w/w). Miscibility was assessed based on a change in melting point of the physical mixture when heated using DSC when compared to the psilocybin alone (melting point onset: 217°C). For EUDRAGIT^{®} L 100-55, miscibility was assessed by glass transition (Tg) temperature.

The outcomes from the different physical mixtures on melting point are summarized in Table 16.

**Table 16**

| **Polymer** | **Melting temperature onset of physical mixture (°C)*** | | | | |
|---|---|---|---|---|---|
| | 10 wt.% psilocybin | 25 wt.% psilocybin | 50 wt.% psilocybin | 75 wt.% psilocybin | 90 wt.% psilocybin |
| EUDRAGIT^{®} E PO | 205 | N/A | 201 | 200 | 200 |
| KOLLIDON^{®} 30 | 198 | N/A | 198 | 200 | 200 |
| KOLLIDON^{®} VA 64 | 205 | 192 | 200 | 198 | 199 |
| PHARMACOAT^{®} 606 | 201 | 200 | 199 | 200 | 198 |
| AQOAT^{®} AS-MG | 202 | 201 | 202 | 201 | 200 |

| | **Predicted Tg (°C) of physical mixture** | | | | |
|---|---|---|---|---|---|
| EUDRAGIT^{®} L 100-55 | 113 | 123 | 131 | 144 | 153 |

| | | | | | |
|---|---|---|---|---|---|
| * For reference, psilocybin alone has a melting point onset of 217°C | | | | | |

All of the tested physical mixtures showed a decrease in melting point, compared to psilocybin alone, and no significant difference in the drop in melting points across the different psilocybin:polymer loading was observed. Thus, it is likely that psilocybin has at least some miscibility with each of the polymers. Miscibility experiments on EUDRAGIT^{®} L 100-55 were not possible due to thermal degradation of polymer. Based on predicted Tg and historical use, it was considered for progression.

Based on the above and the miscibility results, KOLLIDON^{®} VA 64, PHARMACOAT^{®} 606, and AQOAT^{®} AS-MG, and EUDRAGIT^{®} L 100-55 were progressed to the next stage and prepared as solid dispersions *via* spray drying, using a 25 wt.% psilocybin loading.

### Spray dried solid dispersions and experimental procedures.

### Examples 16-19

The polymers used to prepare solid dispersions were as follows: Example 16 (KOLLIDON^{®} VA 64), Example 17 (PHARMACOAT^{®} 606), Example 18 (AQOAT^{®} AS-MG), and Example 19 (EUDRAGIT^{®} L 100-55). Samples were prepared with a psilocybin/polymer ratio of 25/75 (% wt./wt.) on a 400 mg scale, using a 2 wt.% solid loading in solvent for the spray drying procedure. The solvent used was a 25/75 mixture by volume of dichloromethane (DCM)/methanol. Spray dried psilocybin (no polymer) was also prepared as a control.

Spray drying was performed using spray dyer-spray chiller/congealer PROCEPT 4M8-Trix, available from Procept, using parameters outlined in Table 17. After spray drying, the samples were dried under vacuum overnight at 6 mBar at 40°C.

**Table 17**

| **Parameter** | **Set point** |
|---|---|
| Equipment | PROCEPT 4M8-Trix |
| Solvent | DCM/MeOH (1:3) |
| Solid content | 2 wt.% (PY:polymer 1:3 w/w) |
| Cyclone | Large |
| Nozzle size | 0.4 mm |
| Inlet gas flow | 0.4 m³/min |
| Inlet temperature | 120°C |
| Cyclone gas flow | 400 L/min |
| Nozzle gas flow | 3 L/min |
| Pump speed | 200 rpm |
| Post processing condition | Dried under vacuum overnight (6 mBar; 40°C) |

### Characterization/results.

Spray dried solid dispersions of Examples 16-19 were characterized initially (t =0) using X-ray powder diffraction (XRPD) and modulated differential scanning calorimetry (mDSC), and the results are summarized in Table 18.

**Table 18**

| **Example No.** | **Formulation** | **Characterization (t = 0)** | | | |
|---|---|---|---|---|---|
| | | Yield (%) | XRPD | Tg (°C) | Tg predicted |
| (control) | Psilocybin (PY) | N/A | Amorphous | 161 | N/A |
| 16 | PY / KOLLIDON^{®} VA 64 | 47.5 | Amorphous | 117 | 119 |
| 17 | PY / PHARMACOAT^{®} 606 | 57.7 | Amorphous | 153 | 161 |
| 18 | PY / AQOAT^{®} AS-MG | 60.0 | Amorphous | 127 | 130 |
| 19 | PY / EUDRAGIT^{®} L 100-55 | 57.5 | Amorphous | 131 | 125 |

The solid dispersions of Examples 16-19 provided amorphous material by XRPD. All four polymers tested produced solid dispersions with a single Tg event, indicating each was made as a single phase, therefore psilocybin and each polymer were miscible. The measured Tg values were close to predicted glass transition temperatures. Further, all Tg values measured were >90°C, and thus the amorphous solid dispersions are within typical stability limits.

*Stability studies.* To further probe the stability, the amorphous solid dispersions of Examples 16-19 were stored at 40°C in closed vials and at set timepoints at 6 hours (t = 6 hr), 24 hours (t = 24 hr), 1 week (t = 1 w), and/or 4 weeks (t = 4 w) were analyzed by XRPD and/or mDSC to evaluate stability against crystallization, as well as by TGA for water uptake.

The stability results for Example 16 are summarized in Table 19.

**Table 19**

| **Example 16** | **Timepoint** | | | | |
|---|---|---|---|---|---|
| | t = 0 | t = 6 hr | t = 24 hr | t = 1 w | t = 4 w |
| XRPD | Amorphous | Amorphous | Amorphous | Amorphous | Amorphous |
| Tg (°C) | 117 | NT | NT | 115 | 111 |
| TGA (% mass loss) | No mass loss observed | NT | No mass loss observed | ca. 2.2% 32-87°C | ca. 2.2% 30-95°C |

| | | | | | |
|---|---|---|---|---|---|
| NT = not tested | | | | | |

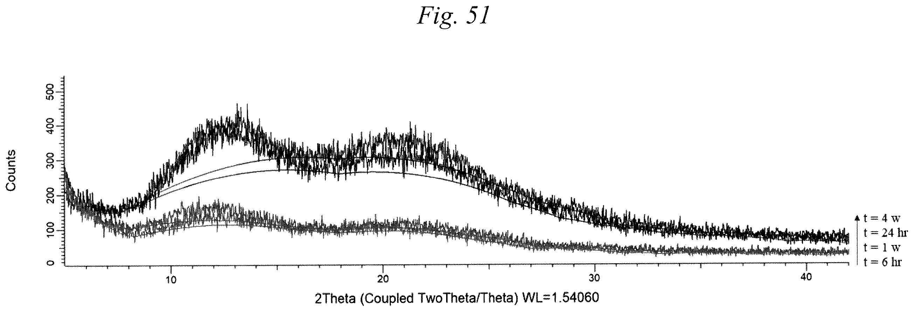

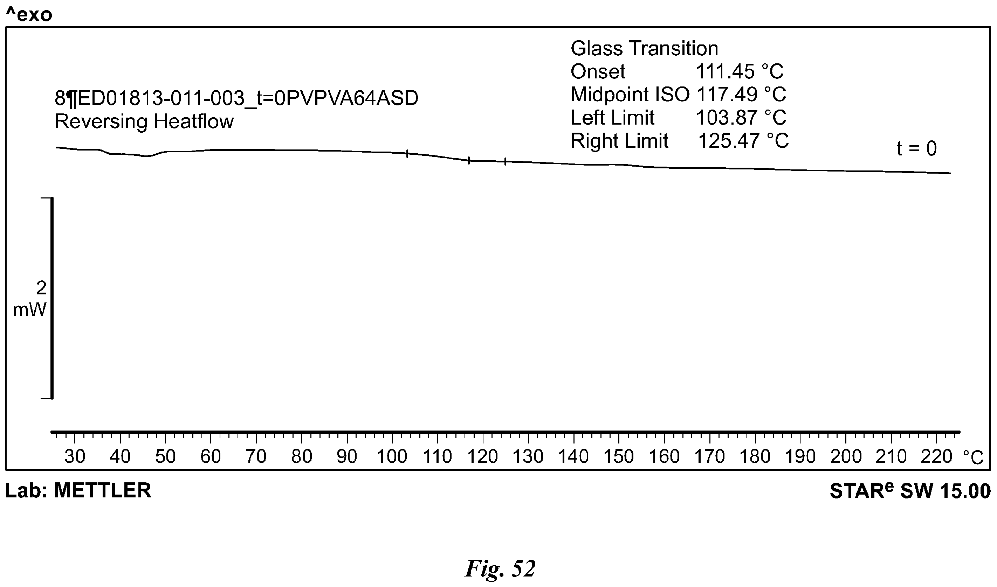

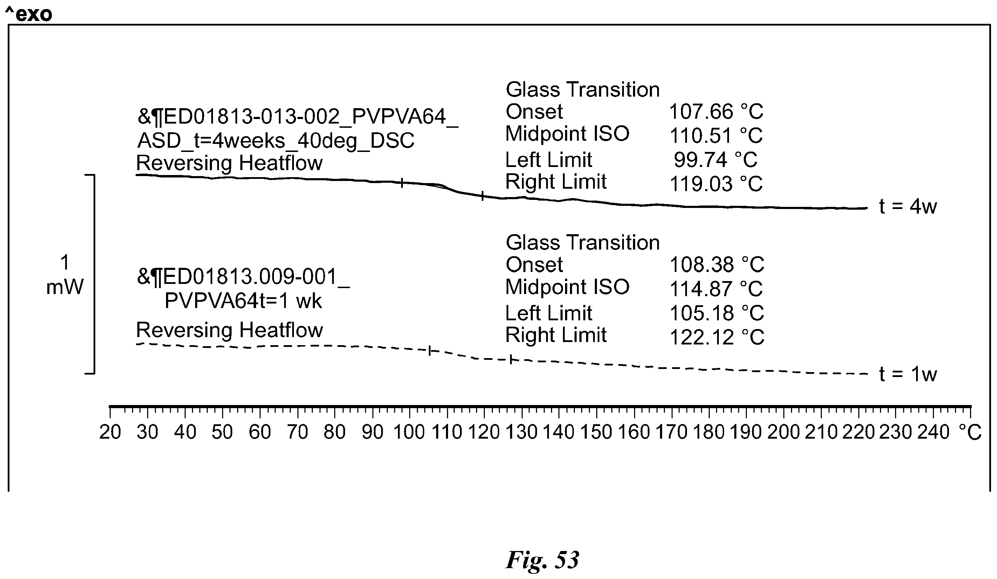

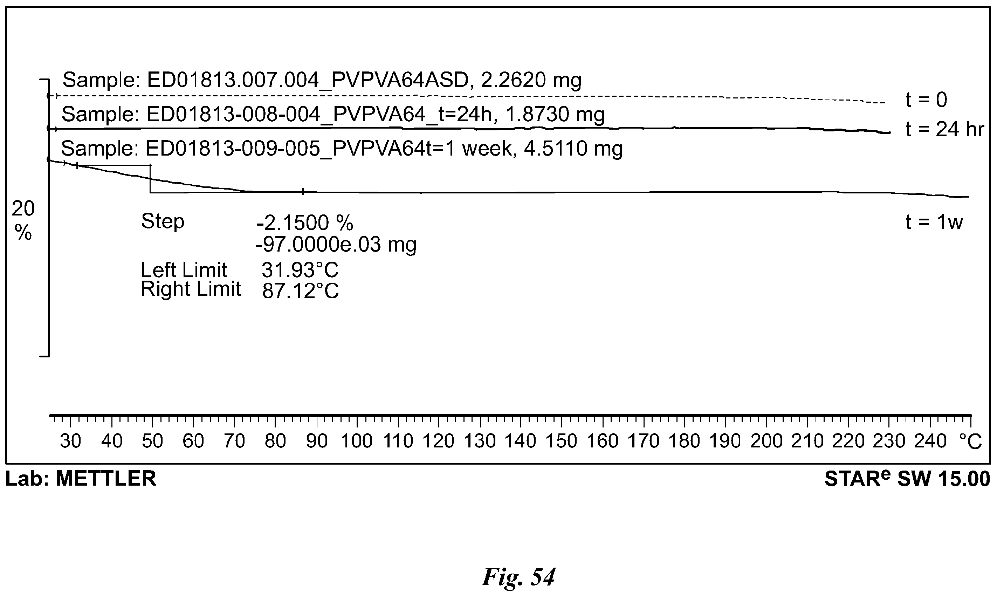

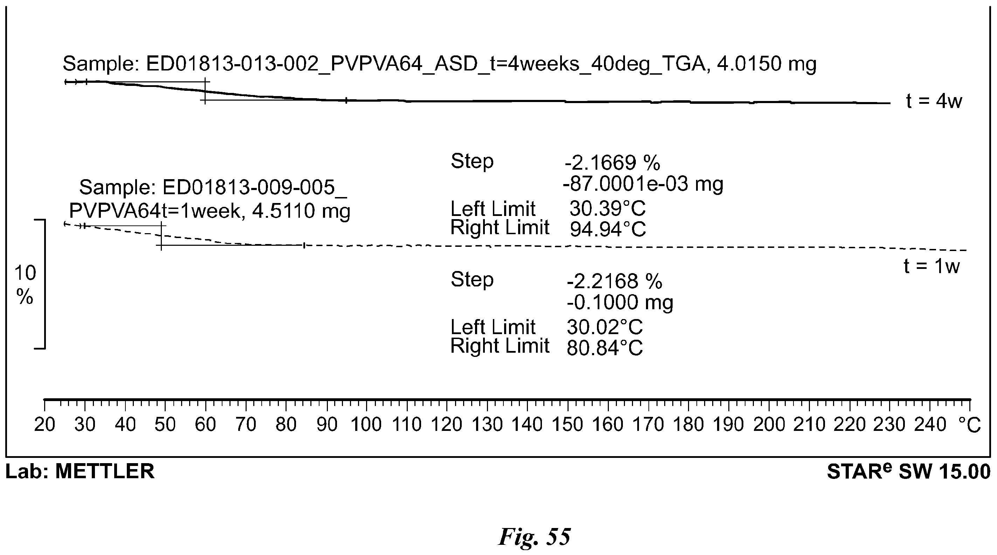

After 4 weeks of storage at 40°C in a closed vial, the solid dispersion of Example 16 remained amorphous by XRPD with no evidence of psilocybin recrystallization (Fig. 51). This material had a single Tg event at 117°C at t = 0 (Fig. 52), and still had a single Tg event at 1 week and 4 weeks of storage at 40°C (Fig. 53). The single Tg on storage indicates no changes in miscibility occurred. There was some evidence of water uptake after 1 week of storage at 40°C (Fig. 54), however this was consistent at the 4 week timepoint (Fig. 55), suggesting water uptake was not increasing over time. Overall, Example 16 is considered stable under the tested storage conditions.

The stability results for Example 17 are summarized in Table 20.

**Table 20**

| **Example 17** | **Timepoint** | | | | |
|---|---|---|---|---|---|
| | t = 0 | t = 6 hr | t = 24 hr | t = 1 w | t = 4 w |
| XRPD | Amorphous | Amorphous | Amorphous | Amorphous | Amorphous |
| Tg (°C) | 153 | NT | NT | 153 | 153 |
| TGA (% mass loss) | No mass loss observed | NT | No mass loss observed | No mass loss observed | ca. 1.4% 34-92°C |

| | | | | | |
|---|---|---|---|---|---|
| NT = not tested | | | | | |

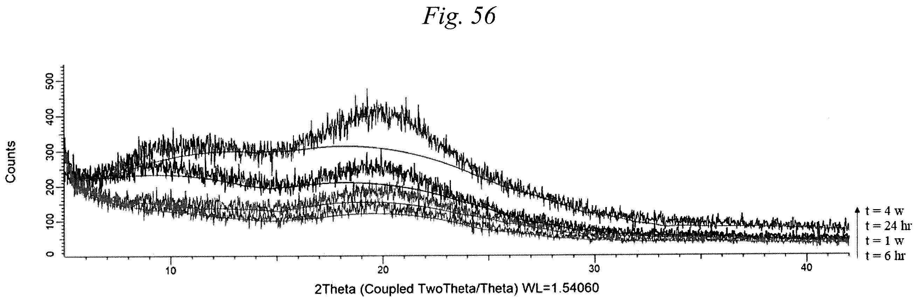

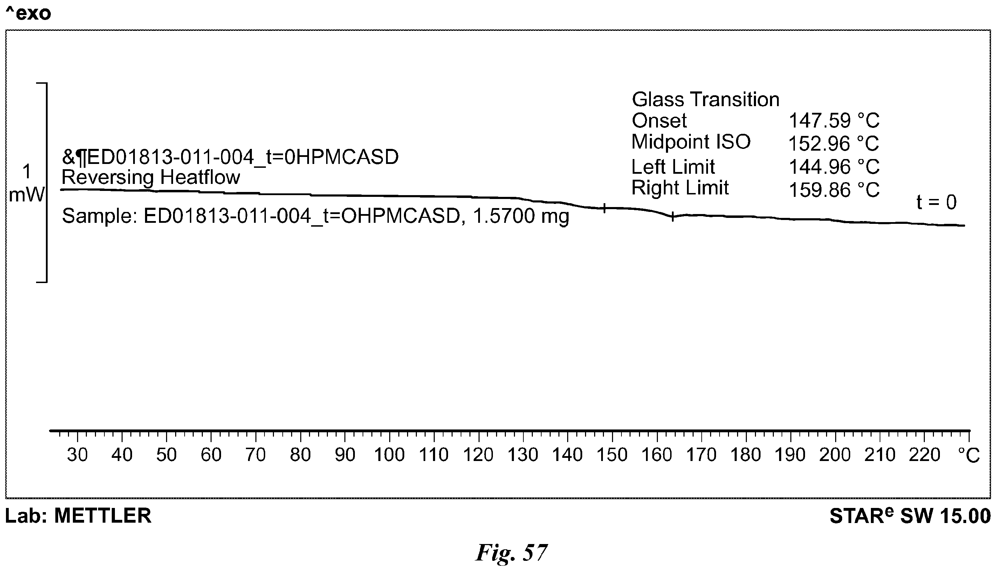

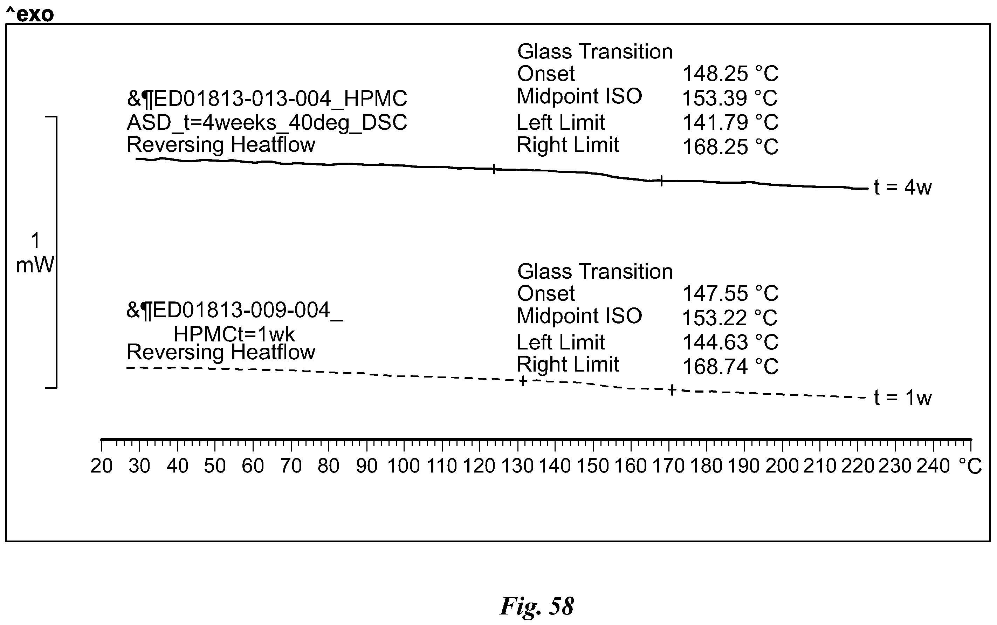

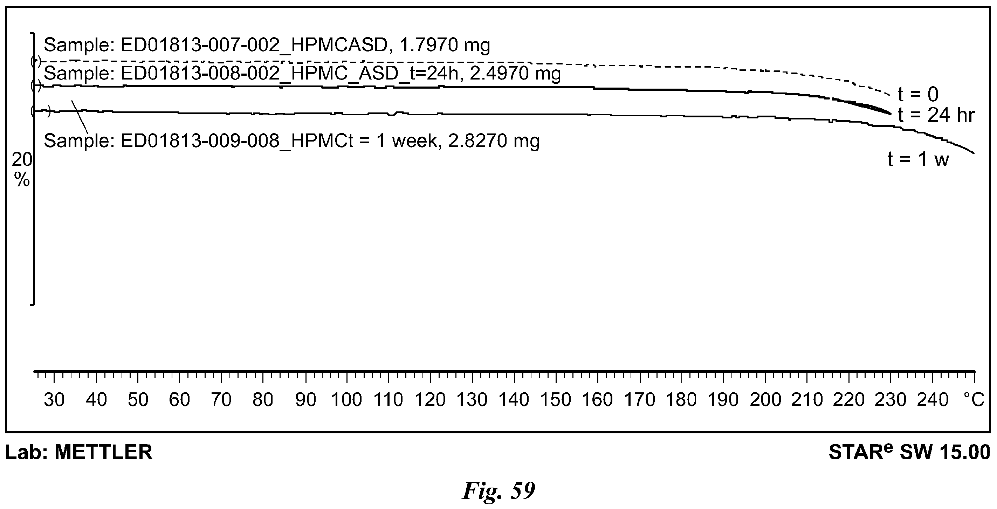

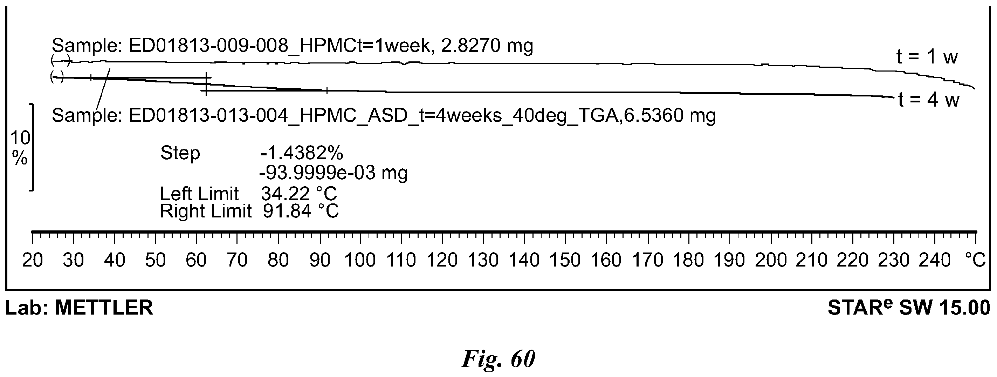

After 4 weeks of storage at 40°C in a closed vial, the solid dispersion of Example 17 remained amorphous by XRPD with no evidence of psilocybin recrystallization (Fig. 56). This material had a single Tg event at 153°C at t = 0 (Fig. 57), and still had a single Tg event at 1 week and 4 weeks of storage at 40°C (Fig. 58). The single Tg on storage indicates no changes in miscibility occurred. There was no evidence of water uptake after 1 week of storage at 40°C (Fig. 59), however there was a slight mass loss of about 1.4% at the 4 week timepoint (Fig. 60), suggesting there may be some water uptake occurring over time. Overall, Example 17 is considered stable under the tested storage conditions.

The stability results for Example 18 are summarized in Table 21.

**Table 21**

| **Example 18** | **Timepoint** | | | | |
|---|---|---|---|---|---|
| | t = 0 | t = 6 hr | t = 24 hr | t = 1 w | t = 4 w |
| XRPD | Amorphous | Amorphous | Amorphous | Amorphous | Amorphous |
| Tg (°C) | 127 | NT | NT | 125 | 128, 152 |
| TGA (% mass loss) | No mass loss observed | NT | No mass loss observed | No mass loss observed | ca. 0.8% 32-97°C |

| | | | | | |
|---|---|---|---|---|---|
| NT = not tested | | | | | |

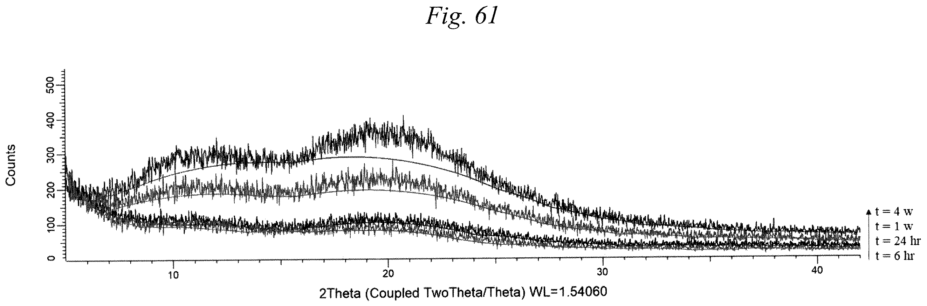

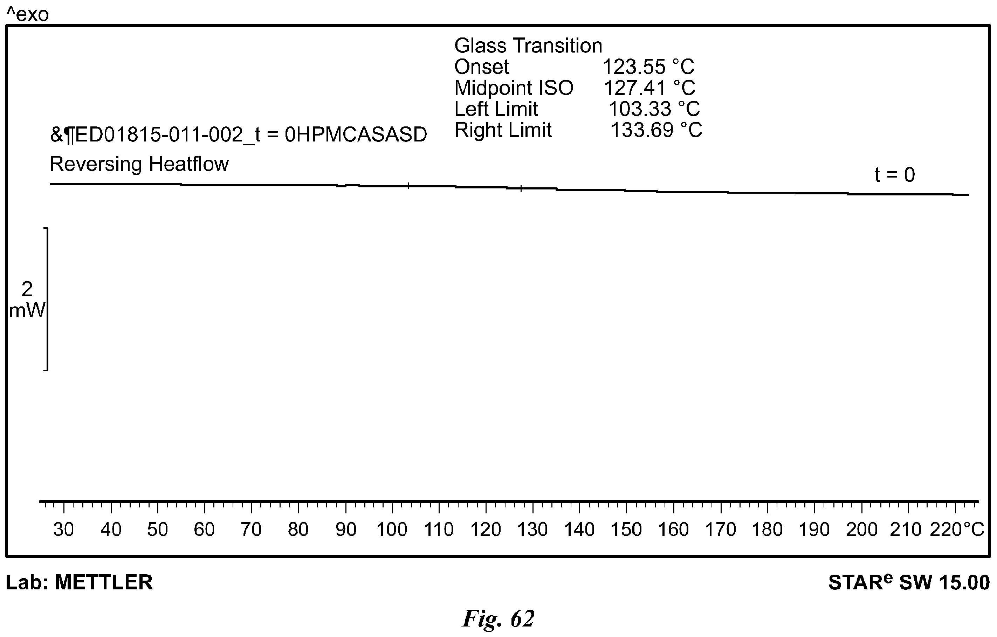

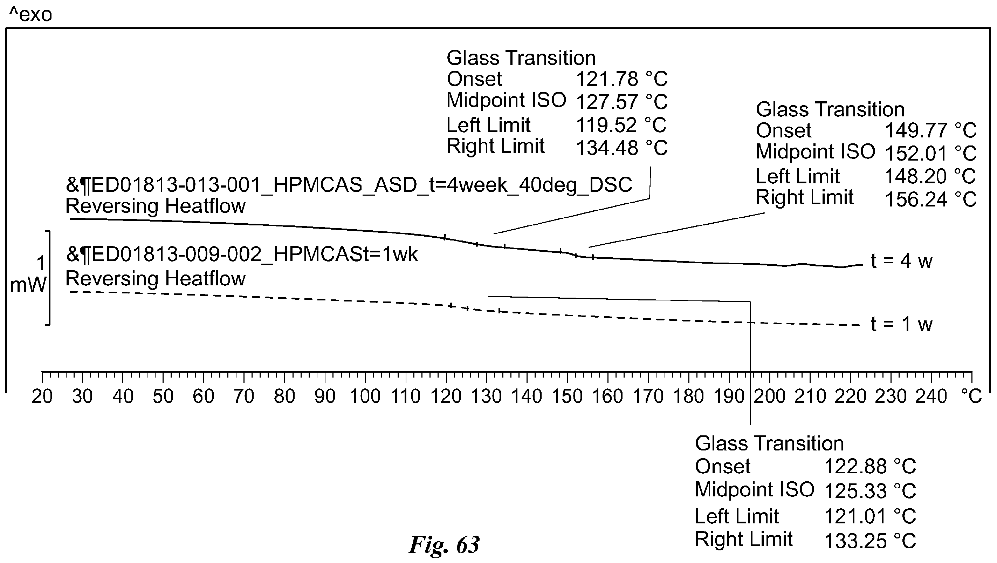

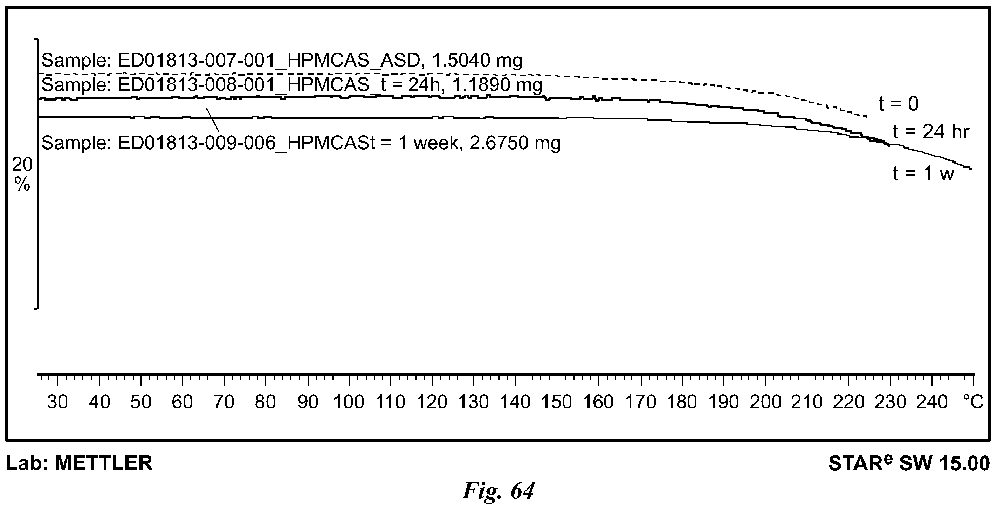

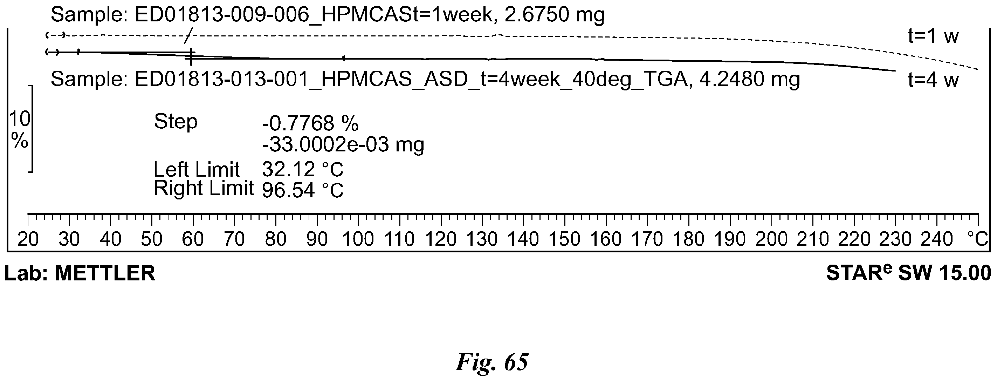

After 4 weeks of storage at 40°C in a closed vial, the solid dispersion of Example 18 remained amorphous by XRPD with no evidence of psilocybin recrystallization (Fig. 61). This material had a single Tg event at 127°C at t = 0 (Fig. 62), but showed an additional Tg event at 152°C at 4 weeks of storage at 40°C (Fig. 63), indicating a potential second phase, which could potentially be free psilocybin. There was no evidence of water uptake after 1 week of storage at 40°C (Fig. 64), however there was a slight mass loss of about 0.8% at the 4 week timepoint (Fig. 65), suggesting there may be some slight water uptake occurring over time. Overall, Example 18 is considered stable under the tested storage conditions, but perhaps less so than Examples 16 and 17 based on the appearance of a second phase overtime.

The stability results for Example 19 are summarized in Table 22.

**Table 22**

| **Example 19** | **Timepoint** | | | | |
|---|---|---|---|---|---|
| | t = 0 | t = 6 hr | t = 24 hr | t = 1 w | t = 4 w |
| XRPD | Amorphous | Amorphous | Amorphous | Amorphous | Amorphous |
| Tg (°C) | 131 | NT | 126 | 136, 147 | 131, 152 |
| TGA (% mass loss) | No mass loss observed | NT | No mass loss observed | NT | ca. 0.7% 46-90°C |

| | | | | | |
|---|---|---|---|---|---|
| NT = not tested | | | | | |

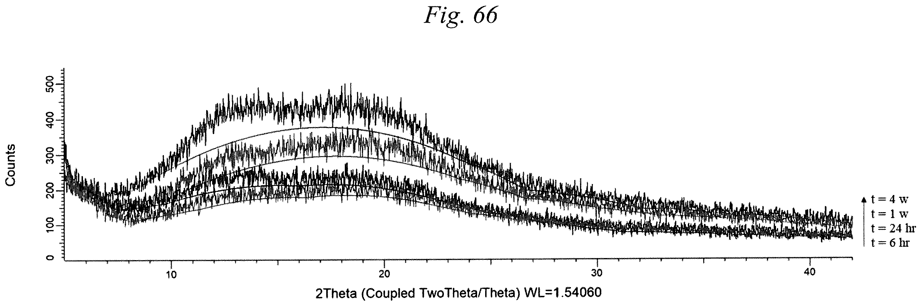

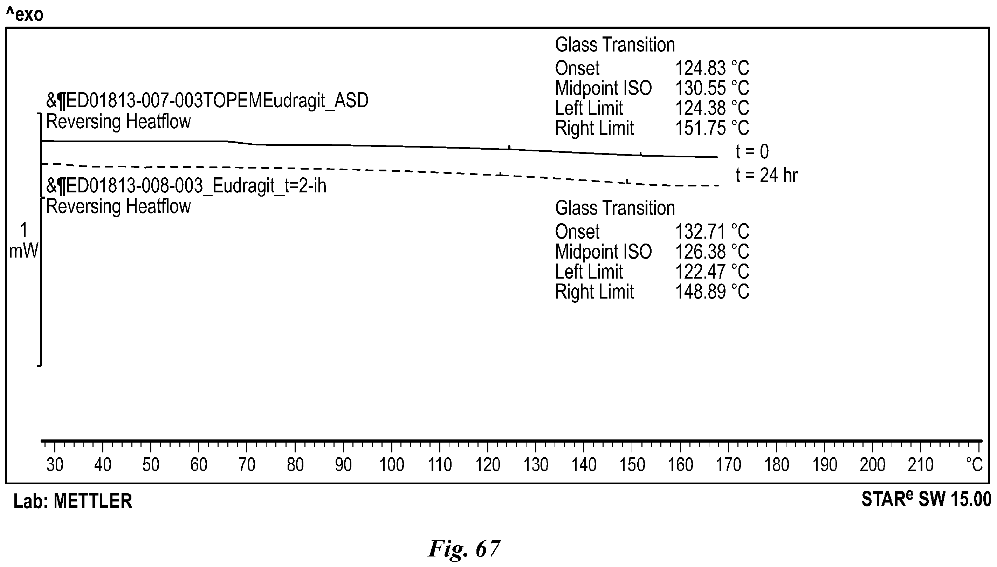

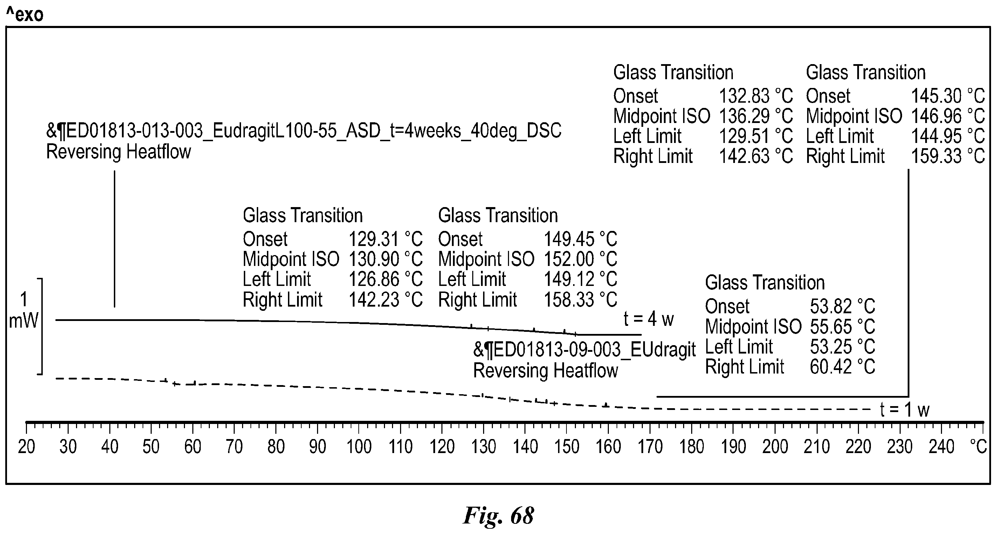

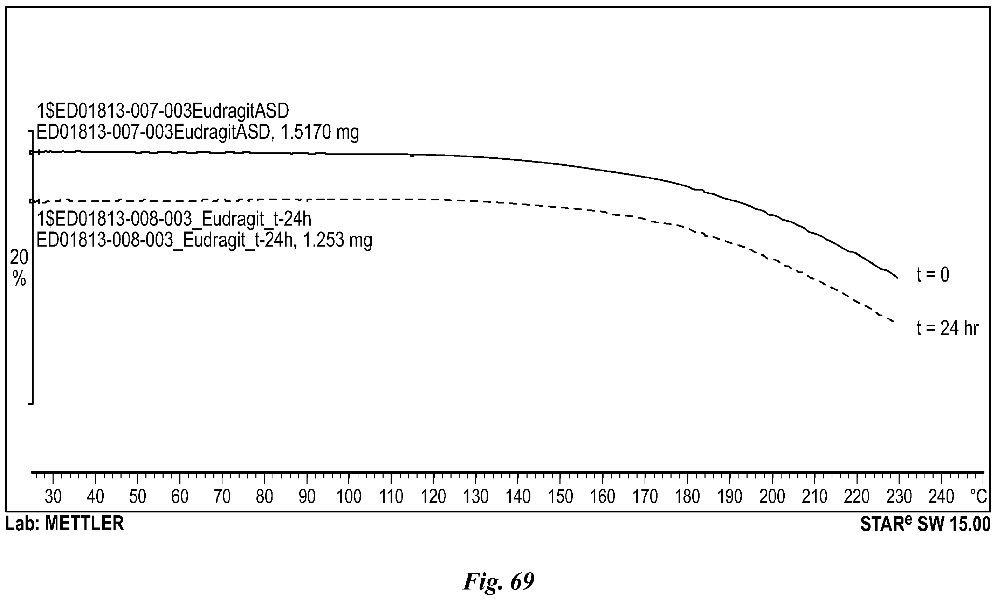

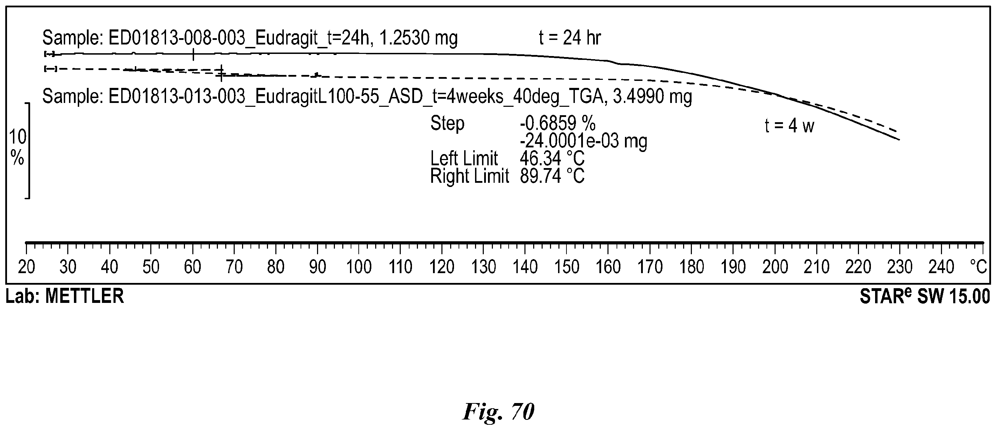

After 4 weeks of storage at 40°C in a closed vial, the solid dispersion of Example 19 remained amorphous by XRPD with no evidence of psilocybin recrystallization (Fig. 66). This material had a single Tg event at 131°C at t = 0 (Fig. 67), but showed an additional Tg event at 147°C at 1 week and at 152°C after 4 weeks of storage at 40°C (Fig. 68), indicating a potential second phase, which could potentially be free psilocybin. There was no evidence of water uptake after 24 hours of storage at 40°C (Fig. 69), however there was a slight mass loss of about 0.7% at the 4 week timepoint (Fig. 70), suggesting there may be some slight water uptake occurring over time. Overall, Example 19 is considered stable under the tested storage conditions, but perhaps less so than Examples 16 and 17 based on the appearance of a second phase overtime.

A summary of the 4 week stability data is presented in Table 23. After 4 weeks of storage at 40°C in a closed vial, all solid dispersions are still amorphous with no evidence of psilocybin recrystallisation. By comparison, amorphous psilocybin (without polymer) is known to crystallize under a variety of stress conditions *(See* Greenan et al., Preparation and Characterization of Novel Crystalline solvates and Polymorphs of Psilocybin and Identification of Solid Forms Suitable for Clinical Development, 2020 pre-publication; DOI:10.13140/RG.2.2.32357.14560). The amorphous solid dispersions of Examples 16 and 17 have a single Tg after 4 weeks of storage indicating they have remained as a single phase system. Examples 18 and 19 developed a second Tg, which could indicate changes in miscibility and a new phase developing. All of the tested dispersions showed evidence of a small amount of mass loss related to water uptake, but not to a significant degree.

**Table 23**

| **Example No.** | **Formulation** | **Characterization (t = 4 weeks at 40°C)** | | |
|---|---|---|---|---|
| | | XRPD | Tg events | Mass loss observed |
| 16 | PY / KOLLIDON^{®} VA 64 | Amorphous | 1 | Yes (~2.2 wt.%) |
| 17 | PY / PHARMACOAT^{®} 606 | Amorphous | 1 | Yes (~1.4 wt.%) |
| 18 | PY / AQOAT^{®} AS-MG | Amorphous | 2 | Yes (~0.8 wt.%) |
| 19 | PY / EUDRAGIT^{®} L 100-55 | Amorphous | 2 | Yes (~0.7 wt.%) |

### IV. Solid dispersion prepared by solution casting

### Example 20

Psilocybin (3-(2-(dimethylamino)ethyl)-1H-indol-4-yl dihydrogen phosphate; PY; **I-7**)(200 mg) in crystalline form as crystalline methanol solvate with a small quantity of crystalline Form B as described above and commercially available from Quality Chemical Labs, is dissolved in 10 ml of a solution of 0.1 M phosphate buffer with 5% hydroxypropyl methyl cellulose (HPMC) and 10% polyvinylpyrrolidone (PVP), and the pH is adjusted to 7.0. The solution is then cast on a polymer liner and dried at ambient temperature for 8 h to form a film, which is subjected to additional drying at 50°C for 2 h in a vacuum oven. The occurrence of the amorphous form of PY in the film will be confirmed by DSC, TGA and X-ray powder diffraction methods. The film is stored at 4°C and the stability pulls conducted at T=1, 3, 6, and 12 months confirming the presence of the amorphous form of PY.

The film displays good properties as a prototype buccal dose form with disintegration time of 3 min and dissolution kinetics characterized by a dissolution time of 5 min.

### V. Animal studies

### Pharmacokinetics of Psilocybin in the Male Beagle Dog Following Oral Administration by Powder in Capsule (PIC) or Orally Disintegrating Tablet (ODT)

The pharmacokinetic profile of psilocin from psilocybin after oral administration in oral disintegrating tablets (ODTs) or powder in capsule (PIC) dosage forms to male beagle dogs was compared.

Animals. Six, non-naïve, male Beagle dogs aged ca 2-5 years and weighing ca 10-15 kg at dosing were used. These animals were supplied by a recognized supplier of laboratory animals and are currently held as part of a colony at the test facility. Following study completion, animals were returned to the colony for further use, following an appropriate washout period.

Housing. Animals were housed and maintained according to established procedures as detailed in the appropriate Standard Operating Procedures (SOPs). Animals were uniquely identified by tattoo or by microchip. During the pre-trial holding periods, the animals were group housed in caging appropriate to the species. The dogs were housed singly for up to 4 h per day and in this period, had access to their daily ration of diet. The dogs were exercised during the study. Animals were checked regularly throughout the duration of the study. Any clinical signs were closely monitored and recorded. Animals had access to 300-350 g/day of Special Diet Services (SDS) D3 (E) SQC diet throughout the study, except during designated procedures. Mains quality tap water was available ad libitum.

Test items. Orally disintegrating tablet (ODT) dosage forms of Example 6 were used containing 5 mg of amorphous psilocybin. Powder in capsule (PIC) dosage forms were prepared using 5 mg of crystalline psilocybin (polymorph Form A, commercially available from Biosynth Carbosynth) as powder inside a capsule.

### Dose levels.

### Psilocybin (amorphous) as ODT contains 5 mg of active; nominal 0.5 mg/kg active

### Psilocybin (crystalline) as PIC contains 5 mg of active; nominal 0.5 mg/kg active

Experimental design. Animals received 5 mg of test item *via* ODT or by PIC. Each animal received a dose level of ca 0.5 mg/kg, but may vary according to the most recent bodyweight of each animal. Bodyweights were recorded for each animal prior to dosing. Oral administration was performed with either an ODT or PIC containing psilocybin. Capsules were placed at the back of the throat and the animals were encouraged to swallow. A flush of 5 mL of water was given if required. Orally disintegrating tablets were placed under the tongue (sublingual). Animal's mouth were held closed for 10 seconds to ensure the tablet was fully dissolved.

Sampling collection. PK samples (ca 1 mL) were collected from the jugular vein by venepuncture into tubes containing K₂EDTA anticoagulant at the following sampling times: Pre-dose, 5, 10, 15, 30, 60, 120, 240 min, 8 and 24 hrs post-dose. Immediately following collection, samples were inverted to ensure mixing with anti-coagulant and placed on wet ice. As soon as practically possible, plasma samples were generated by centrifugation (2500 g, 10 min, 4 °C). All plasma samples generated were transferred from K₂EDTA tube to aliquot A (per animal/timepoint). Then, 300 µL of plasma and 300 µL (1:1 (v/v)) of 200 mM ascorbic acid were decanted into Aliquot B and stored in a freezer set to maintain a temperature of -65°C, until analysis.

Bioanalysis. Plasma samples were analyzed using an established LC-MS/MS assay (BQL were set at zero prior to Cmax; BQL undefined after Cmax). Plasma samples from the psilocybin ODT and capsule groups were analyzed for psilocybin and psilocin.

Pharmacokinetic parameters. Noncompartmental pharmacokinetic parameters were determined from the plasma concentration-time profiles using commercially available software (Phoenix^{®} WinNonlin^{®}).

Results. The data relating to the individual PK parameters are presented in Table 24.

**Table 24**

| **Formulation** | **Compound Dosed** | **Analyte** | **Stats** | **T1/2 (hr)** | **Tmax (hr)** | **Cmax (ng/mL)** | **AUClast (hr*ng/mL)** | **AUCINF_obs (hr*ng/mL)** | **Vz_F_obs (mL/kg)** | **Cl_F_obs (mL/kg/hr)** |
|---|---|---|---|---|---|---|---|---|---|---|
| ODT | Psilocybin | Psilocin | Mean | 2.06 | 0.25 | 28.9 | 71.8 | 77.0 | 16600 | 5570 |
| | | | SD | 0.475 | 0.25-0.5 | 5.75 | 13.3 | 13.1 | 4420 | 4420 |
| PIC | Psilocybin | Psilocin | Mean | 1.80 | 0.5 | 41 | 89.0 | 93.3 | 12000 | 4580 |
| | | | SD | 0.215 | 0.25-2 | 11.6 | 16.5 | 16.9 | 3400 | 872 |

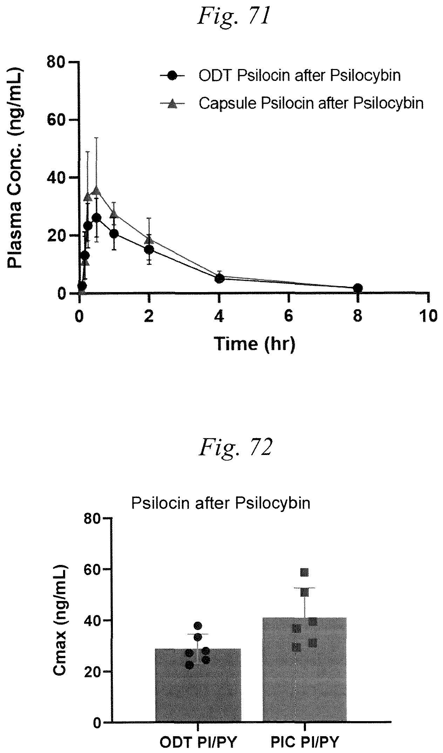

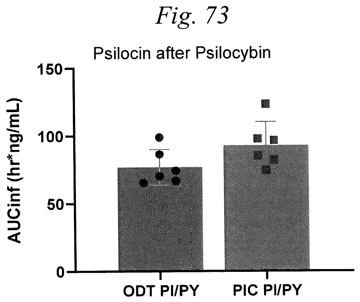

The results are also graphically represented in Fig. 71, which shows the plasma concentration-time profiles for psilocin after psilocybin dosing (ODT and PIC dosage forms), Fig. 72 showing the exposure comparison between psilocybin ODT and PIC dosage forms as assessed by Cmax, and Fig. 73 showing the exposure comparison between psilocybin ODT and PIC dosage forms as assessed by AUCinf.

As can be seen from these graphs, psilocin after psilocybin ODT formulation exposure is not significantly (p>0.5) different than PIC exposure. The ODTs produced a faster onset of action compared to PIC dosage forms as measured by time to maximum plasma concentrations-the time to maximum plasma concentration was twice as fast after ODT compared to PIC.

The terms and expressions which have been employed are used as terms of description and not of limitation, and there is no intention that in the use of such terms and expressions of excluding any equivalents of the features shown and described or portions thereof, but it is recognized that various modifications are possible within the scope of the claims. Thus, it should be understood that although the present methods and compositions have been specifically disclosed by embodiments and optional features, modifications and variations of the concepts herein disclosed can be resorted to by those skilled in the art, and that such modifications and variations are considered to be within the scope of the compositions and methods as defined by the description and the appended claims.

Any single term, single element, single phrase, group of terms, group of phrases, or group of elements described herein can each be specifically excluded from the claims.

Whenever a range is given in the specification, for example, a temperature range, a time range, a composition, or concentration range, all intermediate ranges and subranges, as well as all individual values included in the ranges given are intended to be included in the disclosure. It will be understood that any subranges or individual values in a range or subrange that are included in the description herein can be excluded from the aspects herein. It will be understood that any elements or steps that are included in the description herein can be excluded from the claimed compositions or methods.

In addition, where features or aspects of the compositions and methods are described in terms of Markush groups or other grouping of alternatives, those skilled in the art will recognize that the compositions and methods are also thereby described in terms of any individual member or subgroup of members of the Markush group or other group.

Accordingly, the preceding merely illustrates the principles of the methods and compositions. It will be appreciated that those skilled in the art will be able to devise various arrangements which, although not explicitly described or shown herein, embody the principles of the disclosure.

Furthermore, all examples and conditional language recited herein are principally intended to aid the reader in understanding the principles of the disclosure and the concepts contributed by the inventors to furthering the art, and are to be construed as being without limitation to such specifically recited examples and conditions. The scope of the present disclosure, therefore, is not intended to be limited to the exemplary embodiments shown and described herein.

## Claims

1. A pharmaceutical composition, comprising:
a solid dispersion comprising a therapeutically effective amount of a compound of Formula
(I) in amorphous form dispersed in a polymer, 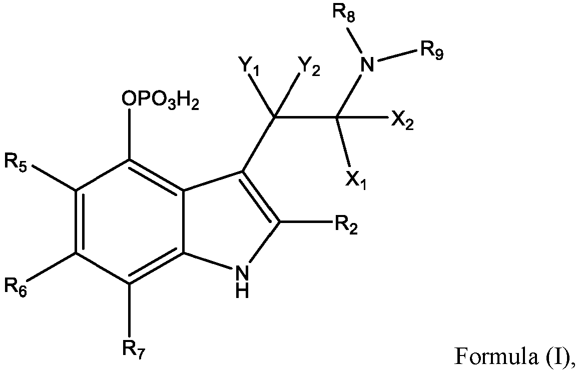 or a pharmaceutically acceptable salt, stereoisomer, a tautomer, or solvate thereof,
wherein:
R₂, R₅, R₆, and R₇ are independently selected from the group consisting of hydrogen and deuterium,
R₈ and R₉ are independently selected from the group consisting of -CH₃ and -CD₃, and
X₁, X₂, Y₁, and Y₂ are independently selected from the group consisting of hydrogen and deuterium.

2. The pharmaceutical composition of claim 1, wherein R₈ and R₉ are -CH₃.

3. The pharmaceutical composition of claim 1, wherein the compound of Formula (I) is at least one selected from the group consisting of: 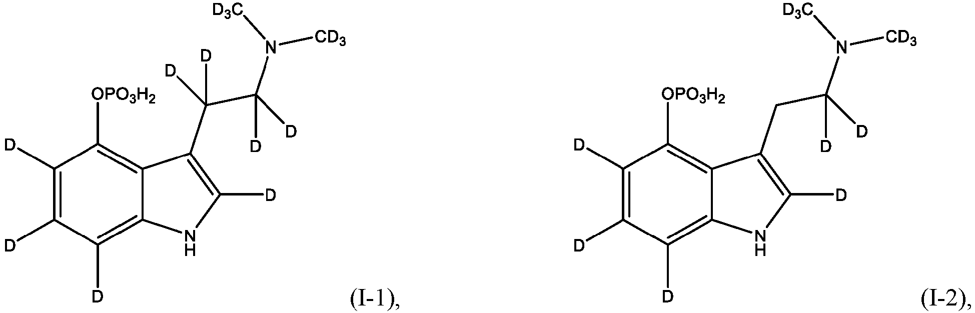 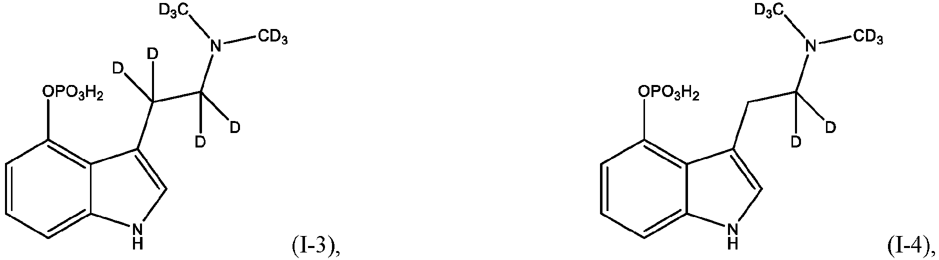 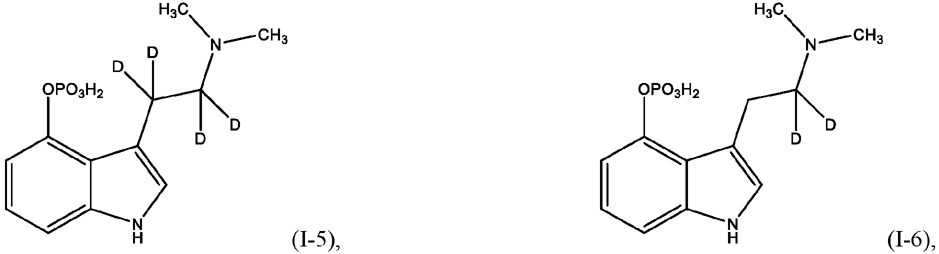 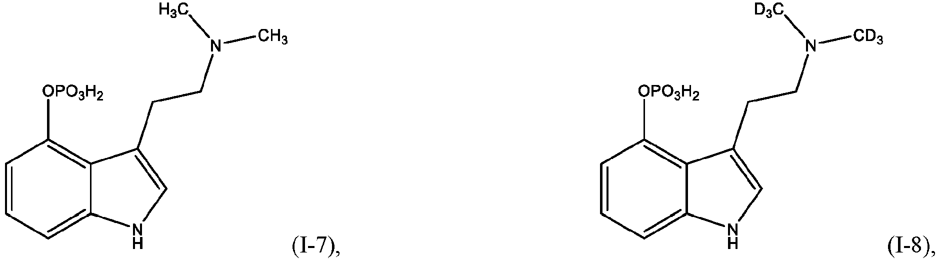 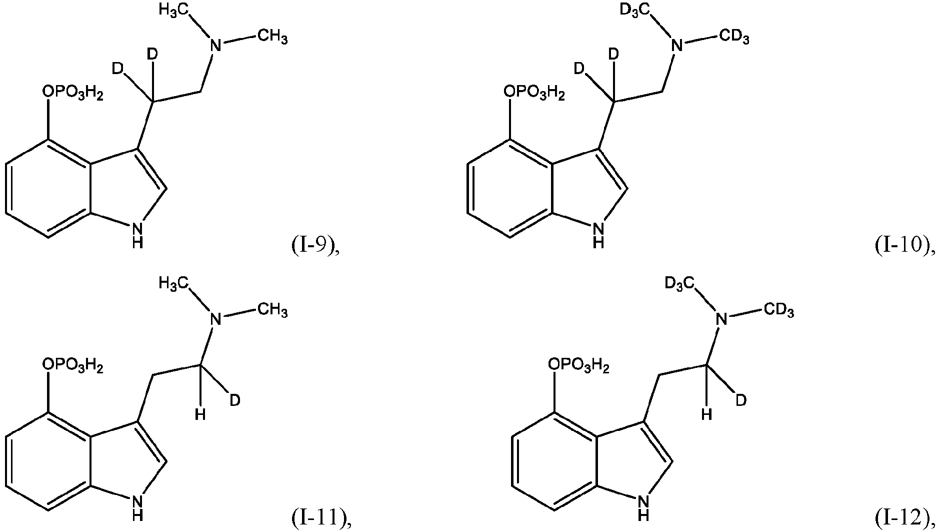 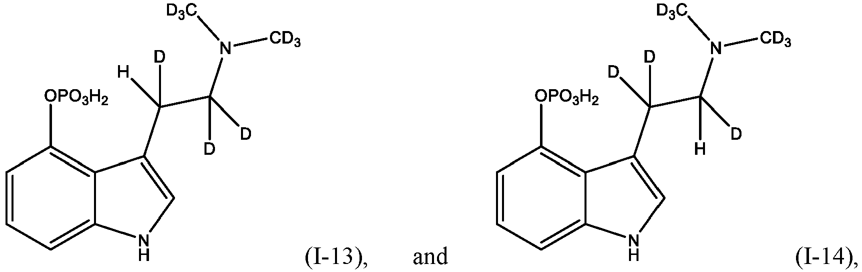 or a pharmaceutically acceptable salt, stereoisomer, a tautomer, or solvate thereof.

4. The pharmaceutical composition of claim 1, wherein the compound of Formula (I) is 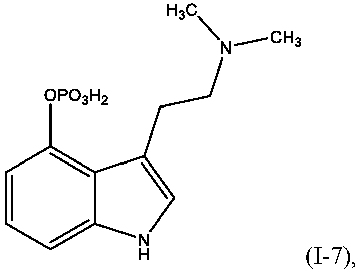 or a pharmaceutically acceptable salt, tautomer, or solvate thereof.

5. The pharmaceutical composition of any one of claims 1 to 4, wherein the solid dispersion is a solid molecular complex.

6. The pharmaceutical composition of any one of claims 1 to 5, wherein a weight ratio of the compound of Formula (I) to the polymer in the solid dispersion is from 1:9 to 9:1.

7. The pharmaceutical composition of any one of claims 1 to 6, wherein the polymer is at least one selected from the group consisting of gelatin, polyvinyl acetate, polyvinyl alcohol, polyvinylpyrrolidone, pullulan, hydroxypropyl methyl cellulose, hydroxypropyl methyl cellulose acetate succinate, and a methacrylate copolymer, or a blend or a copolymer thereof.

8. The pharmaceutical composition of any one of claims 1 to 7, wherein the polymer comprises gelatin, or a copolymer of vinyl pyrrolidone and vinyl acetate (PVP-VAc), or a methacrylate copolymer, or a cellulose polymer.

9. The pharmaceutical composition of claim 8, wherein the polymer comprises a cellulose polymer, and wherein the cellulose polymer has a weight average molecular weight of from 150,000 g/mol to 5,000,000 g/mol or has a weight average molecular weight of from 1,000 g/mol to 100,000 g/mol.

10. The pharmaceutical composition of claim 8, wherein the polymer comprises a cellulose polymer, and wherein the cellulose polymer is hydroxypropyl methyl cellulose acetate succinate or hydroxypropyl methyl cellulose.

11. The pharmaceutical composition of any one of claims 1 to 7, wherein the polymer is a blend of hydroxypropyl methyl cellulose and polyvinylpyrrolidone.

12. The pharmaceutical composition of any one of claims 1 to 11, wherein the solid dispersion has (i) a glass transition (Tg) onset of from 110°C to 200°C, as determined by modulated differential scanning calorimetry (mDSC), (ii) a heat capacity change (ΔCp), in J/(g·°C), of from 0.1 to 0.75, as determined by modulated differential scanning calorimetry (mDSC), or both (i) and (ii).

13. A pharmaceutical composition of any one of claims 1 to 12 for use in treating a subject with a disease or disorder.

14. A pharmaceutical composition of any one of claims 1 to 13 for use in treating a subject with a disease or disorder selected from:
(i) a disease or disorder associated with a serotonin 5-HT₂ receptor;
(ii) a neuropsychiatric disease or disorder or an inflammatory disease or disorder;
(iii) a central nervous system (CNS) disorder, optionally wherein the central nervous system (CNS) disorder is at least one selected from the group consisting of major depressive disorder (MDD), treatment-resistant depression (TRD), post-traumatic stress disorder (PTSD), bipolar and related disorders, obsessive-compulsive disorder (OCD), generalized anxiety disorder (GAD), social anxiety disorder, a substance use disorder, an eating disorder, Alzheimer's disease, cluster headache and migraine, attention deficit hyperactivity disorder (ADHD), pain and neuropathic pain, aphantasia, childhood-onset fluency disorder, major neurocognitive disorder, mild neurocognitive disorder, suicidal ideation, suicidal behavior, major depressive disorder with suicidal ideation or suicidal behavior, melancholic depression, atypical depression, dysthymia, non-suicidal self-injury disorder (NSSID), chronic fatigue syndrome, Lyme's disease, gambling disorder, a paraphilic disorder, sexual dysfunction, peripheral neuropathy, and obesity;
(iv) major depressive disorder (MDD);
(v) treatment-resistant depression (TRD);
(vi) generalized anxiety disorder (GAD);
(vii) social anxiety disorder;
(viii) obsessive-compulsive disorder (OCD);
(ix) cluster headaches or migraine;
(x) a substance use disorder, optionally wherein the substance use disorder is alcohol use disorder;
(xi) mental distress in frontline healthcare workers;
(xii) an autonomic nervous system (ANS) condition;
(xiii) a pulmonary disorder; or
(xiv) a cardiovascular disorder.

15. The pharmaceutical composition for use of claim 14, wherein the pharmaceutical composition is administered orally to the subject.

## Patentansprüche

1. Pharmazeutische Zusammensetzung, umfassend:
eine feste Dispersion, umfassend eine therapeutisch wirksame Menge einer Verbindung der Formel (I) in amorpher Form, dispergiert in einem Polymer, 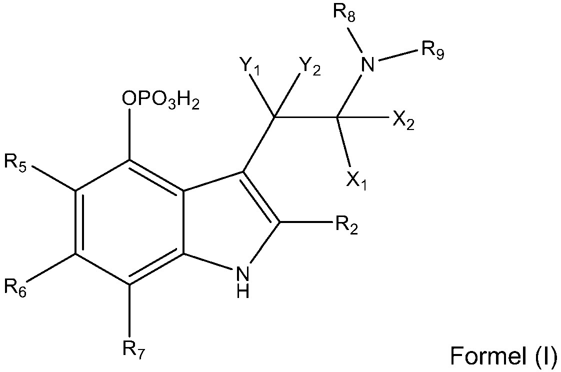
oder ein pharmazeutisch verträgliches Salz, Stereoisomer, ein Tautomer oder Solvat davon,
wobei:
R₂, R₅, R₆ und R₇ unabhängig voneinander aus der Gruppe, bestehend aus Wasserstoff und Deuterium, ausgewählt sind,
R₈ und R₉ unabhängig voneinander aus der Gruppe, bestehend aus -CH₃ und -CD₃, ausgewählt sind,
X₁, X₂, Y₁ und Y₂ unabhängig voneinander aus der Gruppe, bestehend aus Wasserstoff und Deuterium, ausgewählt sind.

2. Pharmazeutische Zusammensetzung nach Anspruch 1, wobei R₈ und R₉ -CH₃ sind.

3. Pharmazeutische Zusammensetzung nach Anspruch 1, wobei die Verbindung der Formel (I) mindestens eine Verbindung ist, ausgewählt aus der Gruppe, bestehend aus: 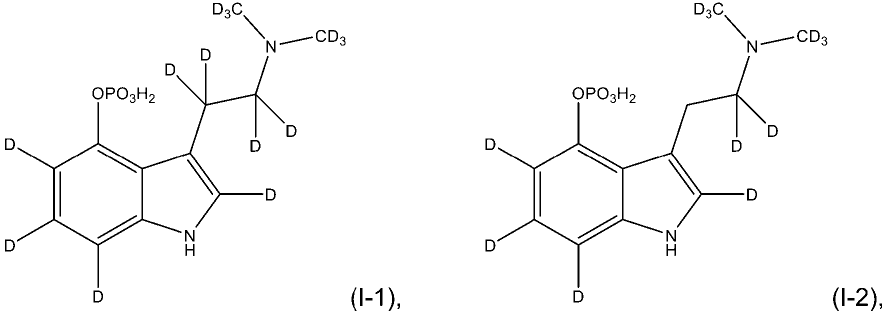 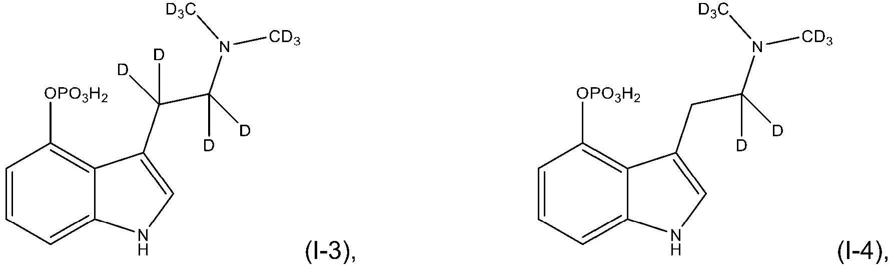 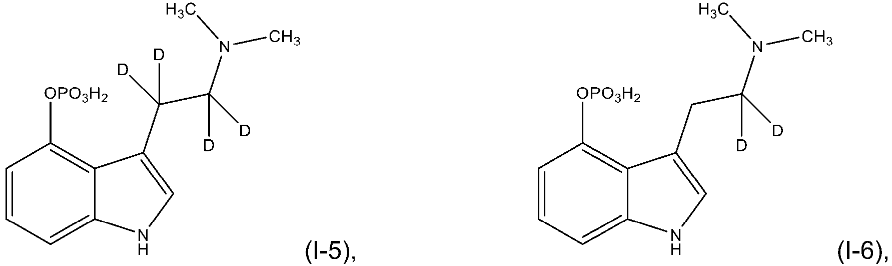 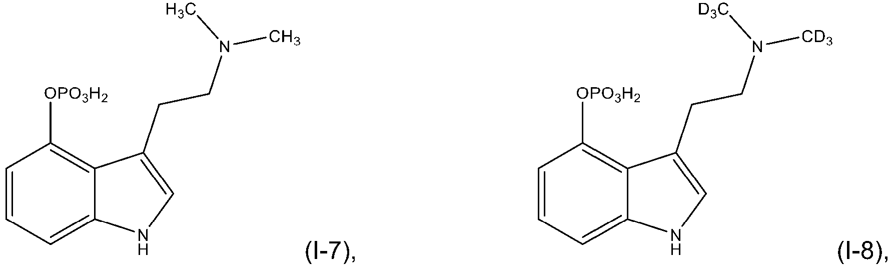 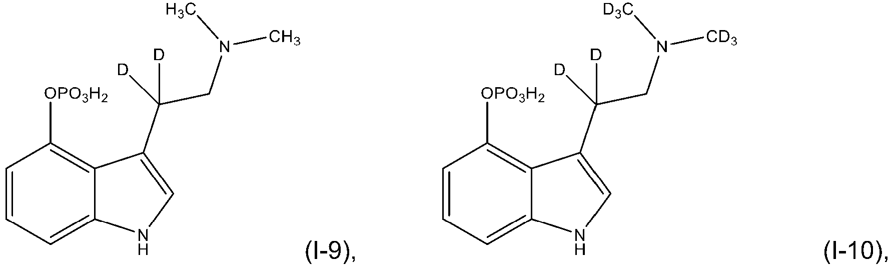 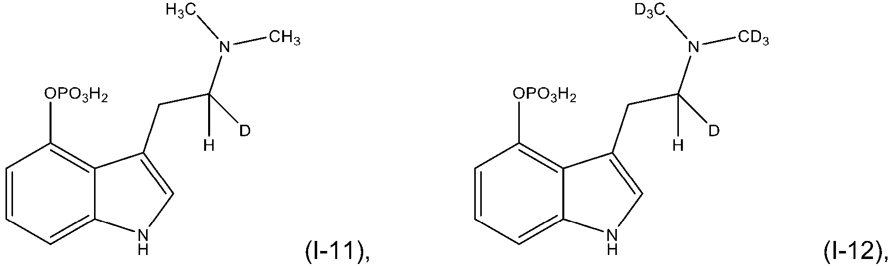 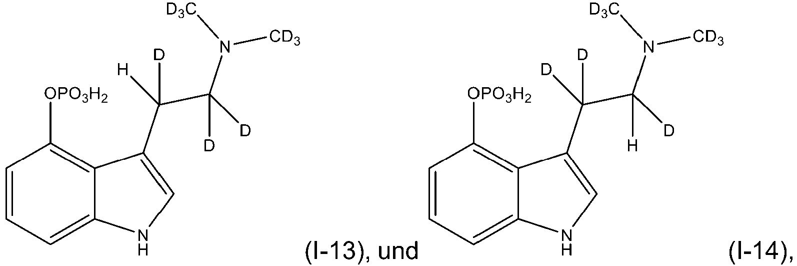 oder ein pharmazeutisch verträgliches Salz, Stereoisomer, ein Tautomer oder Solvat davon.

4. Pharmazeutische Zusammensetzung nach Anspruch 1, wobei die Verbindung der Formel (I) 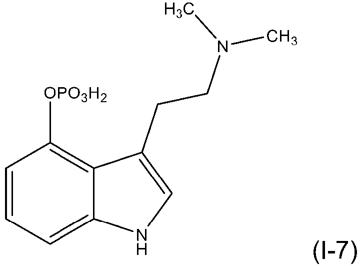 oder ein pharmazeutisch verträgliches Salz, Tautomer oder Solvat davon ist.

5. Pharmazeutische Zusammensetzung nach einem der Ansprüche 1 bis 4, wobei die feste Dispersion ein fester molekularer Komplex ist.

6. Pharmazeutische Zusammensetzung nach einem der Ansprüche 1 bis 5, wobei ein Gewichtsverhältnis der Verbindung der Formel (I) zu dem Polymer in der festen Dispersion von 1:9 bis 9:1 beträgt.

7. Pharmazeutische Zusammensetzung nach einem der Ansprüche 1 bis 6, wobei das Polymer mindestens eines ist, ausgewählt aus der Gruppe, bestehend aus Gelatine, Polyvinylacetat, Polyvinylalkohol, Polyvinylpyrrolidon, Pullulan, Hydroxypropylmethylcellulose, Hydroxypropylmethylcelluloseacetatsuccinat und einem Methacrylat-Copolymer, oder einer Mischung oder einem Copolymer davon.

8. Pharmazeutische Zusammensetzung nach einem der Ansprüche 1 bis 7, wobei das Polymer Gelatine oder ein Copolymer aus Vinylpyrrolidon und Vinylacetat (PVP-VAc) oder ein Methacrylat-Copolymer oder ein Cellulosepolymer umfasst.

9. Pharmazeutische Zusammensetzung nach Anspruch 8, wobei das Polymer ein Cellulosepolymer umfasst und wobei das Cellulosepolymer ein gewichtsgemitteltes Molekulargewicht von 150.000 g/mol bis 5.000.000 g/mol oder ein gewichtsgemitteltes Molekulargewicht von 1.000 g/mol bis 100.000 g/mol aufweist.

10. Pharmazeutische Zusammensetzung nach Anspruch 8, wobei das Polymer ein Cellulosepolymer umfasst und wobei das Cellulosepolymer Hydroxypropylmethylcelluloseacetatsuccinat oder Hydroxypropylmethylcellulose ist.

11. Pharmazeutische Zusammensetzung nach einem der Ansprüche 1 bis 7, wobei das Polymer eine Mischung aus Hydroxypropylmethylcellulose und Polyvinylpyrrolidon ist.

12. Pharmazeutische Zusammensetzung nach einem der Ansprüche 1 bis 11, wobei die feste Dispersion (i) einen Glasübergangsbeginn (Tg) von 110°C bis 200°C, bestimmt durch modulierte Differenzialrasterkalorimetrie (mDSC), (ii) eine Wärmekapazitätsänderung (ΔCp) in J/(g·°C) von 0,1 bis 0,75, bestimmt durch modulierte Differenzialrasterkalorimetrie (mDSC), oder sowohl (i) als auch (ii) aufweist.

13. Pharmazeutische Zusammensetzung nach einem der Ansprüche 1 bis 12 zur Verwendung bei der Behandlung eines Subjekts mit einer Krankheit oder Störung.

14. Pharmazeutische Zusammensetzung nach einem der Ansprüche 1 bis 13 zur Verwendung bei der Behandlung eines Subjekts mit einer Krankheit oder Störung, ausgewählt aus:
(i) einer Krankheit oder Störung, die mit einem Serotonin-5-HT₂-Rezeptor assoziiert ist;
(ii) einer neuropsychiatrischen Krankheit oder Störung oder einer entzündlichen Krankheit oder Störung;
(iii) einer Störung des Zentralnervensystems (ZNS), wobei gegebenenfalls die Störung des Zentralnervensystems (ZNS) mindestens eine ist, ausgewählt aus der Gruppe, bestehend aus schwere depressive Störung (MDD), behandlungsresistente Depression (TRD), posttraumatische Belastungsstörung (PTBS), bipolare und ähnliche Störungen, Zwangsstörung (OCD), generalisierte Angststörung (GAD), soziale Angststörung, einer Substanzkonsumstörung, einer Essstörung, Alzheimer-Krankheit, Clusterkopfschmerzen und Migräne, Aufmerksamkeitsdefizit-Hyperaktivitätsstörung (ADHS), Schmerzen und neuropathische Schmerzen, Aphantasie, im Kindesalter beginnende Sprechflussstörung, schwere neurokognitive Störung, milde neurokognitive Störung, Suizidgedanken, suizidales Verhalten, schwere depressive Störung mit Suizidgedanken oder suizidalem Verhalten, melancholische Depression, atypische Depression, Dysthymie, nicht-suizidale Selbstverletzungsstörung (NSSID), chronisches Erschöpfungssyndrom, Lyme-Borreliose, Glücksspielsucht, paraphile Störung, sexuelle Dysfunktion, periphere Neuropathie und Adipositas;
(iv) schwere depressive Störung (MDD);
(v) behandlungsresistente Depression (TRD);
(vi) generalisierte Angststörung (GAD);
(vii) soziale Angststörung;
(viii) Zwangsstörung (OCD);
(ix) Clusterkopfschmerzen oder Migräne;
(x) eine Substanzkonsumstörung, wobei die Substanzkonsumstörung gegebenenfalls Alkoholkonsumstörung ist;
(xi) psychische Belastungen bei Beschäftigten im Gesundheitswesen an vorderster Linie;
(xii) eine Verfassung des autonomen Nervensystems (ANS);
(xiii) eine Lungenstörung; oder
(xiv) eine Herz-Kreislauf-Störung.

15. Pharmazeutische Zusammensetzung zur Verwendung nach Anspruch 14, wobei die pharmazeutische Zusammensetzung dem Subjekt oral verabreicht wird.

## Revendications

1. Composition pharmaceutique, comprenant :
une dispersion solide comprenant une quantité thérapeutiquement efficace d'un composé de Formule (I) sous forme amorphe dispersé dans un polymère, 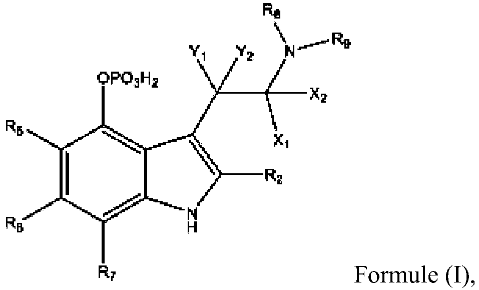
ou d'un sel, stéréoisomère, tautomère ou solvate pharmaceutiquement acceptable de celui-ci,
dans laquelle :
R₂, R₅, R₆ et R₇ sont indépendamment sélectionnés parmi le groupe constitué d'hydrogène et de deutérium,
R₈ et R₉ sont indépendamment sélectionnés parmi le groupe constitué de -CH₃ et -CD₃, et
X₁, X₂, Y₁ et Y₂ sont indépendamment sélectionnés parmi le groupe constitué d'hydrogène et de deutérium.

2. Composition pharmaceutique selon la revendication 1, dans laquelle R₈ et R₉ sont -CH₃.

3. Composition pharmaceutique selon la revendication 1, dans laquelle le composé de Formule (I) est au moins un sélectionné parmi le groupe constitué de : 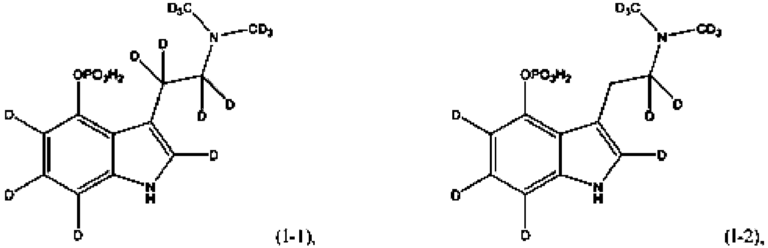 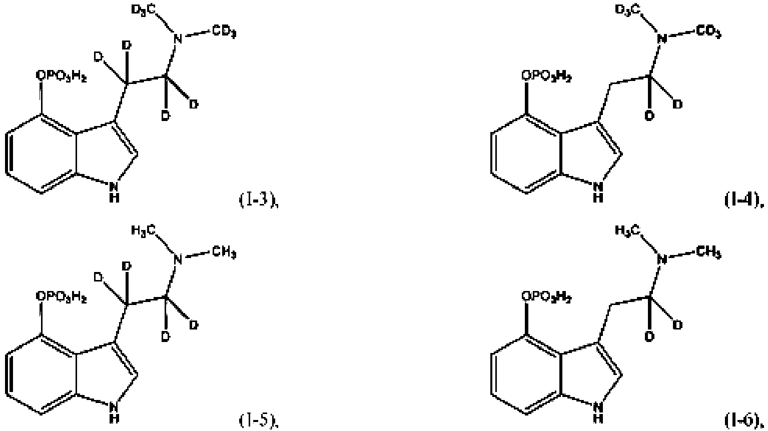 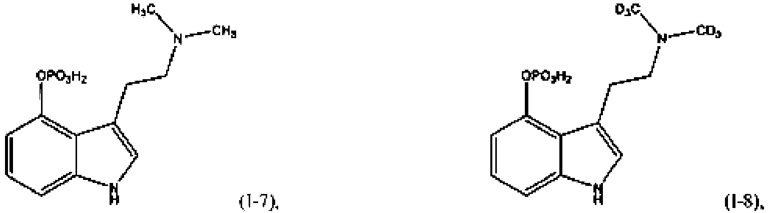 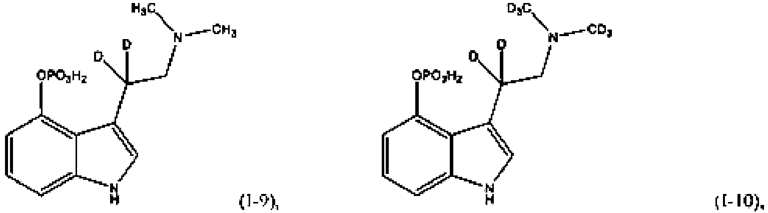 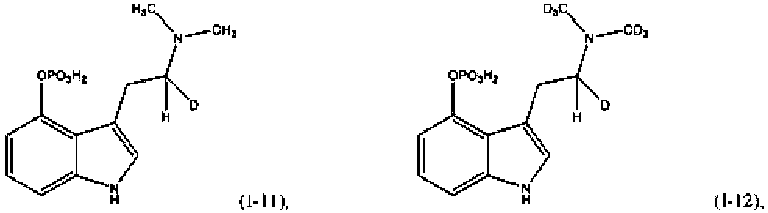 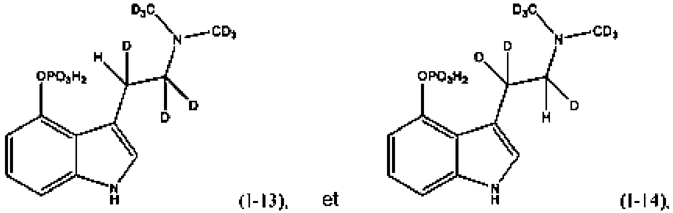 ou un sel, stéréoisomère, tautomère ou solvate pharmaceutiquement acceptable de celui-ci.

4. Composition pharmaceutique selon la revendication 1, dans laquelle le composé de Formule (I) est 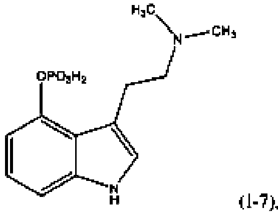 ou un sel, tautomère ou solvate pharmaceutiquement acceptable de celui-ci.

5. Composition pharmaceutique selon l'une quelconque des revendications 1 à 4, dans laquelle la dispersion solide est un complexe moléculaire solide.

6. Composition pharmaceutique selon l'une quelconque des revendications 1 à 5, dans laquelle un rapport pondéral du composé de Formule (I) sur le polymère dans la dispersion solide va de 1/9 à 9/1.

7. Composition pharmaceutique selon l'une quelconque des revendications 1 à 6, dans laquelle le polymère est au moins un sélectionné parmi le groupe constitué de la gélatine, de l'acétate de polyvinyle, de l'alcool polyvinylique, de la polyvinylpyrrolidone, du pullulan, de l'hydroxypropylméthylcellulose, du succinate d'acétate d'hydroxypropylméthylcellulose et d'un copolymère de méthacrylate, ou d'un mélange ou d'un copolymère de ceux-ci.

8. Composition pharmaceutique selon l'une quelconque des revendications 1 à 7, dans laquelle le polymère comprend de la gélatine, ou un copolymère de vinylpyrrolidone et d'acétate de vinyle (PVP-VAc), ou un copolymère de méthacrylate, ou un polymère de cellulose.

9. Composition pharmaceutique selon la revendication 8, dans laquelle le polymère comprend un polymère de cellulose, et dans laquelle le polymère de cellulose a un poids moléculaire moyen en poids allant de 150 000 g/mol à 5 000 000 g/mol ou a un poids moléculaire moyen en poids allant de 1000 g/mol à 100 000 g/mol.

10. Composition pharmaceutique selon la revendication 8, dans laquelle le polymère comprend un polymère de cellulose, et dans laquelle le polymère de cellulose est le succinate d'acétate d'hydroxypropylméthylcellulose ou l'hydroxypropylméthylcellulose.

11. Composition pharmaceutique selon l'une quelconque des revendications 1 à 7, dans laquelle le polymère est un mélange d'hydroxypropylméthylcelllose et de polyvinylpyrrolidone.

12. Composition pharmaceutique selon l'une quelconque des revendications 1 à 11, dans laquelle la dispersion solide a (i) un début de transition vitreuse (Tg) allant de 110 °C à 200 °C, tel que déterminé par calorimétrie différentielle modulée (mDSC), (ii) un changement de capacité thermique (ΔCp), en J/(g·°C), allant de 0,1 à 0,75, tel que déterminé par calorimétrie différentielle modulée (mDSC), ou à la fois (i) et (ii).

13. Composition pharmaceutique selon l'une quelconque des revendications 1 à 12 destinée à être utilisée dans le traitement d'un sujet avec une maladie ou un trouble.

14. Composition pharmaceutique selon l'une quelconque des revendications 1 à 13 destinée à être utilisée dans le traitement d'un sujet avec une maladie ou un trouble sélectionné(e) parmi :
(i) une maladie ou un trouble associé(e) à un récepteur 5-HT₂ de sérotonine ;
(ii) une maladie ou un trouble neuropsychiatrique ou une maladie ou un trouble inflammatoire ;
(iii) un trouble du système nerveux central (CNS), en option dans laquelle le trouble du système nerveux central (CNS) est au moins un sélectionné parmi le groupe constitué d'un trouble dépressif majeur (MDD), d'une dépression résistante aux traitements (TRD), d'un trouble de stress post-traumatique (PTSD), de troubles bipolaires et apparentés, d'un trouble obsessif-compulsif (OCD), d'un trouble anxieux généralisé (GAD), d'un trouble anxieux social, d'un trouble lié à l'usage de substances psychoactives, d'un trouble alimentaire, de la maladie d'Alzheimer, de l'algie vasculaire de la face et de la migraine, d'un trouble déficit de l'attention avec hyperactivité (ADHD), de la douleur et de douleur neuropathique, de l'aphantasie, d'un trouble de la fluence débutant dans l'enfance, d'un trouble neurocognitif majeur, d'un trouble neurocognitif modéré, de l'idéation suicidaire, d'un comportement suicidaire, d'un trouble dépressif majeur avec idéation suicidaire ou comportement suicidaire, de la dépression mélancolique, de la dépression atypique, de la dysthymie, d'un trouble d'automutilation non suicidaire (NSSID), du syndrome de fatigue chronique, de la maladie de Lyme, d'un trouble des jeux d'argent et de hasard, d'un trouble paraphilique, d'un dysfonctionnement sexuel, d'une neuropathie périphérique et de l'obésité ;
(iv) un trouble dépressif majeur (MDD) ;
(v) une dépression résistante aux traitements (TRD) ;
(vi) un trouble anxieux généralisé (GAD) ;
(vii) un trouble anxieux social ;
(viii) un trouble obsessif-compulsif (OCD) ;
(ix) des algies vasculaires de la face ou la migraine ;
(x) un trouble lié à l'usage de substances psychoactives, en option dans laquelle le trouble lié à l'usage de substances psychoactives est un trouble lié à l'usage de l'alcool ;
(xi) une détresse mentale chez les soignants de première ligne ;
(xii) une affection du système nerveux autonome (ANS) ;
(xiii) un trouble pulmonaire ; ou
(xiv) un trouble cardiovasculaire.

15. Composition pharmaceutique destinée à être utilisée selon la revendication 14, dans laquelle la composition pharmaceutique est administrée par voie orale au sujet.
